# EUROPÄISCHE PATENTSCHRIFT

(11) **EP 2 475 646 B1**
(45) Veröffentlichungstag und Bekanntmachung des Hinweises auf die Patenterteilung: **21.06.2017**
(21) Anmeldenummer: 10739516.2
(22) Anmeldetag: 21.07.2010
(51) Int. Cl.: C07D 231/20, C07D 409/12, A01N 43/56

(54) **4- (3-ALKYLTHIOBENZOYL) PYRAZOLE UND IHRE VERWENDUNG ALS HERBIZIDE**
4-(3-ALKYLTHIOBENZOYL)PYRAZOLES AND THEIR USE AS HERBICIDES
4-(3-ALKYLTHIOBENZOYL)PYRAZOLES ET LEUR UTILISATION EN TANT QU'HERBICIDES

(30) Priorität: 29.07.2009 US 229353 P; 29.07.2009 EP 09009778
(43) Veröffentlichungstag der Anmeldung: 18.07.2012
(73) Patentinhaber: Bayer Intellectual Property GmbH, 40789 Monheim am Rhein (DE)
(72) Erfinder: AHRENS, Hartmut, 63329 Egelsbach (DE); VAN ALMSICK, Andreas, 61184 Karben (DE); DITTGEN, Jan, 60316 Frankfurt (DE); ROSINGER, Christopher, Hugh, 65719 Hofheim (DE); HÄUSER-HAHN, Isolde, 51375 Leverkusen (DE); LEHR, Stefan, 65835 Liederbach (DE); FEUCHT, Dieter, 65760 Eschborn (DE)
(74) Vertreter: BIP Patents
(86) Internationale Anmeldenummer: PCT/EP2010/004444
(87) Internationale Veröffentlichungsnummer: WO 2011/012246

(56) Entgegenhaltungen:
- EP-A1- 2 103 215
- WO-A-2005/097754
- WO-A-2008/078811
- WO-A-2008/125214
- US-A1- 2008 305 956

## Beschreibung

Die Erfindung betrifft das technische Gebiet der Herbizide, insbesondere das der Herbizide zur selektiven Bekämpfung von Unkräutern und Ungräsern in Nutzpflanzenkulturen.

Aus verschiedenen Schriften ist bereits bekannt, daß bestimmte Benzoylpyrazole herbizide Eigenschaften besitzen. So werden in US 4,063,925, WO 2008/078811 und US 2008/305956 Benzoylpyrazole beschrieben, die am Phenylring durch verschiedene Reste substituiert sind.

Die aus diesen Schriften bekannten Verbindungen zeigen jedoch häufig eine nicht ausreichende herbizide Wirksamkeit. Aufgabe der vorliegenden Erfindung ist daher die Bereitstellung von weiteren herbizid wirksamen Verbindungen mit - gegenüber den aus dem Stand der Technik bekannten Verbindungen - verbesserten Eigenschaften.

Es wurde nun gefunden, daß 4-Benzoylpyrazole, deren Phenylring in 2,- 3- und 4-Position durch ausgewählte Reste substituiert sind, als Herbizide besonders gut geeignet sind.

Ein Gegenstand der vorliegenden Erfindung sind 4-(3-Alkylthiobenzoyl)pyrazole der Formel (I) oder deren Salze 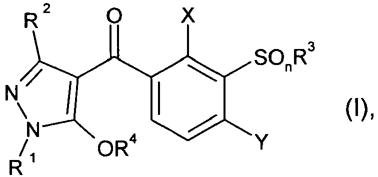 worin
- R¹: bedeutet (C₁-C₄)-Alkyl,
- R²: bedeutet Wasserstoff oder (C₁-C₄)-Alkyl,
- R³: bedeutet (C₁-C₆)-Alkyl,
- R⁴: bedeutet Wasserstoff, (C₁-C₆)-Alkylsulfonyl, (C₁-C₄)-Alkoxy-(C₁-C₆)-alkylsulfonyl, Phenylsulfonyl, Thiophenyl-2-sulfonyl, Benzoyl, Benzoyl-(C₁-C₆)-alkyl, Benzyl, wobei die fünf letztgenannten Reste durch s Reste aus der Gruppe bestehend aus Halogen, (C₁-C₄)-Alkyl und (C₁-C₄)-Alkoxy substituiert sind;
- X: bedeutet OR⁵, OCOR⁵ oder OSO₂R⁶,
- R⁵: bedeutet Wasserstoff, (C₁-C₆)-Alkyl, (C₂-C₆)-Alkenyl, (C₂-C₆)-Alkinyl, (C₃-C₆)-Cycloalkyl, (C₃-C₆)-Cycloalkyl-(C₁-C₆)-alkyl oder Phenyl-(C₁-C₆)-alkyl, wobei die sechs letztgenannten Reste durch s Reste aus der Gruppe bestehend aus Halogen, OR⁷ und S(O)ₘR⁸ substituiert sind,
- R⁶: bedeutet jeweils durch s Reste aus der Gruppe bestehend aus Halogen, OR⁷ und S(O)ₘR⁸ substituiertes (C₁-C₆)-Alkyl, (C₂-C₆)-Alkenyl, (C₂-C₆)-Alkinyl, (C₃-C₆)-Cycloalkyl, (C₃-C₆)-Cycloalkyl-(C₁-C₆)-alkyl oder Phenyl-(C₁-C₆)-alkyl,
- R⁷: bedeutet Wasserstoff, (C₁-C₆)-Alkyl, (C₂-C₆)-Alkenyl oder (C₂-C₆)-Alkinyl,
- R⁸: bedeutet (C₁-C₆)-Alkyl, (C₂-C₆)-Alkenyl oder (C₂-C₆)-Alkinyl,
- Y: bedeutet (C₁-C₆)-Halogenalkyl,
- m: bedeutet 0, 1 oder 2,
- n: bedeutet 0, 1 oder 2,
- s: bedeutet 0, 1, 2 oder 3.

In der Formel (I) und allen nachfolgenden Formeln können Alkylreste mit mehr als zwei Kohlenstoffatomen geradkettig oder verzweigt sein. Alkylreste bedeuten z.B. Methyl, Ethyl, n- oder i-Propyl, n-, i-, t- oder 2-Butyl, Pentyle, Hexyle, wie n-Hexyl, i-Hexyl und 1,3-Dimethylbutyl. Halogen steht für Fluor, Chlor, Brom oder Iod.

Ist eine Gruppe mehrfach durch Reste substituiert, so ist darunter zu verstehen, daß diese Gruppe durch ein oder mehrere gleiche oder verschiedene der genannten Reste substituiert ist.

Die Verbindungen der allgemeinen Formel (I) können je nach Art und Verknüpfung der Substituenten als Stereoisomere vorliegen. Sind beispielsweise ein oder mehrere asymmetrisch substituierte Kohlenstoffatome vorhanden, so können Enantiomere und Diastereomere auftreten. Ebenso treten Stereoisomere auf, wenn n für 1 steht (Sulfoxide). Stereoisomere lassen sich aus den bei der Herstellung anfallenden Gemischen nach üblichen Trennmethoden, beispielsweise durch chromatographische Trennverfahren, erhalten. Ebenso können Stereoisomere durch Einsatz stereoselektiver Reaktionen unter Verwendung optisch aktiver Ausgangs- und/oder Hilfsstoffe selektiv hergestellt werden. Die Erfindung betrifft auch alle Stereoisomeren und deren Gemische, die von der allgemeinen Formel (I) umfasst, jedoch nicht spezifisch definiert sind.

Bevorzugt sind Verbindungen der allgemeinen Formel (I), worin
- R¹: bedeutet (C₁-C₄)-Alkyl,
- R²: bedeutet Wasserstoff oder (C₁-C₄)-Alkyl,
- R³: bedeutet (C₁-C₆)-Alkyl,
- R⁴: bedeutet Wasserstoff, (C₁-C₆)-Alkylsulfonyl, (C₁-C₄)-Alkoxy-(C₁-C₆)-alkylsulfonyl, Phenylsulfonyl, Thiophenyl-2-sulfonyl, Benzoyl, Benzoyl-(C₁-C₆)-alkyl, Benzyl, wobei die fünf letztgenannten Reste durch s Reste aus der Gruppe bestehend aus Halogen, (C₁-C₄)-Alkyl und (C₁-C₄)-Alkoxy substituiert sind;
- X: bedeutet OR⁵,
- R⁵: bedeutet durch s Methoxy- oder Ethoxygruppen substituiertes (C₁-C₆)-Alkyl,
- Y: bedeutet (C₁-C₆)-Halogenalkyl,
- s: bedeutet 0, 1, 2 oder 3.

Besonders bevorzugt sind Verbindungen der allgemeinen Formel (I), worin
- R¹: bedeutet Methyl, Ethyl, n-Propyl oder i-Propyl,
- R²: bedeutet Wasserstoff, Methyl, Ethyl, n-Propyl oder i-Propyl,
- R³: bedeutet Methyl, Ethyl, n-Propyl oder i-Propyl,
- R⁴: bedeutet Wasserstoff, (C₁-C₆)-Alkylsulfonyl, (C₁-C₄)-Alkoxy-(C₁-C₆)-alkylsulfonyl, Phenylsulfonyl, Thiophenyl-2-sulfonyl, Benzoyl, Benzoyl-(C₁-C₆)-alkyl, Benzyl, wobei die fünf letztgenannten Reste durch s Reste aus der Gruppe bestehend aus Halogen, (C₁-C₄)-Alkyl und (C₁-C₄)-Alkoxy substituiert sind;
- X: bedeutet OR⁵,
- R⁵: bedeutet (C₁-C₆)-Alkyl,
- Y: bedeutet (C₁-C₆)-Halogenalkyl,
- s: bedeutet 0, 1, 2 oder 3.

Ganz besonders bevorzugt sind Verbindungen der allgemeinen Formel (I), worin
- R¹: bedeutet Methyl, Ethyl, n-Propyl oder i-Propyl,
- R²: bedeutet Wasserstoff, Methyl, Ethyl, n-Propyl oder i-Propyl,
- R³: bedeutet Methyl, Ethyl, n-Propyl oder i-Propyl,
- R⁴: bedeutet Wasserstoff, (C₁-C₆)-Alkylsulfonyl, (C₁-C₄)-Alkoxy-(C₁-C₆)-alkylsulfonyl, Phenylsulfonyl, Thiophenyl-2-sulfonyl, Benzoyl, Benzoyl-(C₁-C₆)-alkyl, Benzyl, wobei die fünf letztgenannten Reste durch s Reste aus der Gruppe bestehend aus Halogen, (C₁-C₄)-Alkyl und (C₁-C₄)-Alkoxy substituiert sind;
- X: bedeutet OR⁵,
- R⁵: bedeutet (C₁-C₆)-Alkyl,
- Y: bedeutet (C₁-C₆)-Halogenalkyl, vorzugsweise Trifluormethyl,
- s: bedeutet 0, 1, 2 oder 3.

In allen nachfolgend genannten Formeln haben die Substituenten und Symbole, sofern nicht anders definiert, dieselbe Bedeutung wie unter Formel (I) beschrieben.

Erfindungsgemäße Verbindungen, worin R⁴ für Wasserstoff steht, können beispielsweise nach der in Schema 1 angegebenen Methode durch Umsetzung einer Benzoesäure (II) zu einem Säurechlorid oder einem Ester (III), anschließender basenkatalysierter Reaktion mit einem Pyrazol (IV) und nachfolgender Umlagerung in Anwesenheit einer Cyanid-Quelle hergestellt werden. Solche Methoden sind dem Fachmann bekannt und beispielsweise in EP-A 0 369 803 und EP-B 0 283 261 beschrieben. In Formel (III) steht L¹ für Chlor, Brom oder Alkoxy.

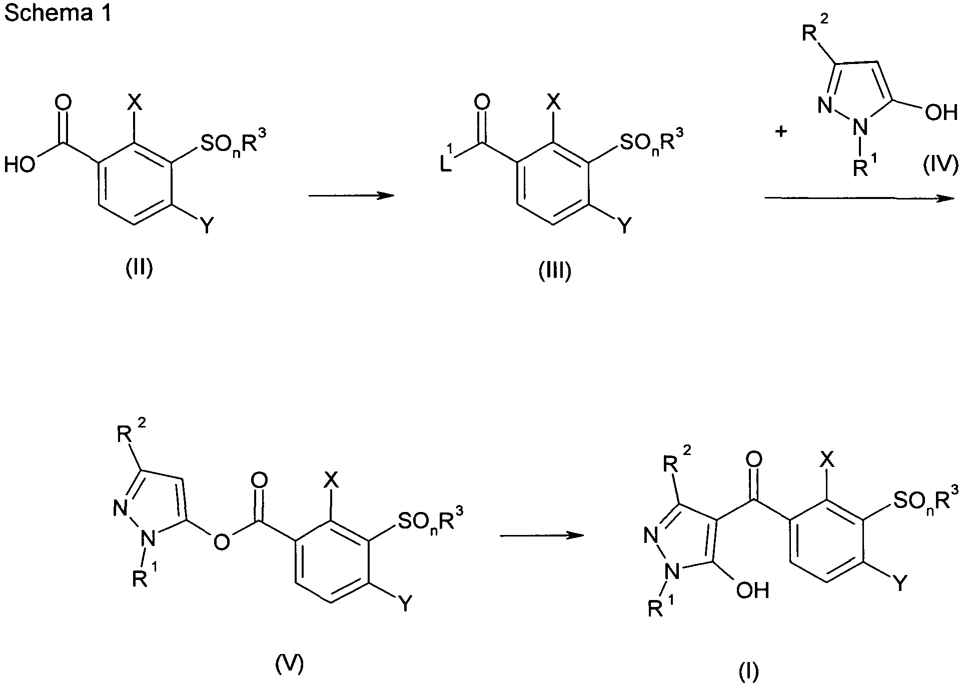

Die 5-Hydroxypyrazole der Formel (IV) sind bekannt und können beispielsweise gemäß den in EP 240 001 A beschriebenen Methoden hergestellt werden.

Erfindungsgemäße Verbindungen, worin R⁴ für einen anderen Rest als Wasserstoff steht, können gemäß Schema 2 aus den erfindungsgemäßen Verbindungen, worin R⁴ für Wasserstoff steht, durch dem Fachmann bekannte Alkylierungs- oder Acylierungsreaktionen hergestellt werden.

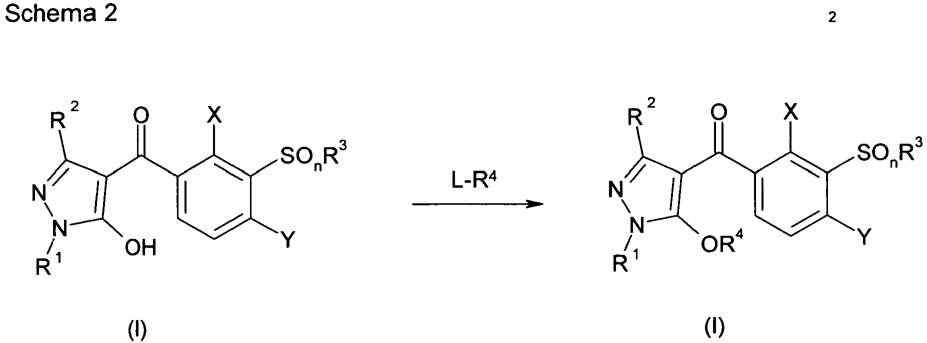

In Schema 2 steht die Gruppe L für eine Abgangsgruppe wie Halogenid oder Trifluormethylsulfonyl.

Die Benzoesäuren (II) können beispielsweise gemäß Schema 3 durch dem Fachmann bekannte Reaktionen aus den Verbindungen (VI) hergestellt werden.

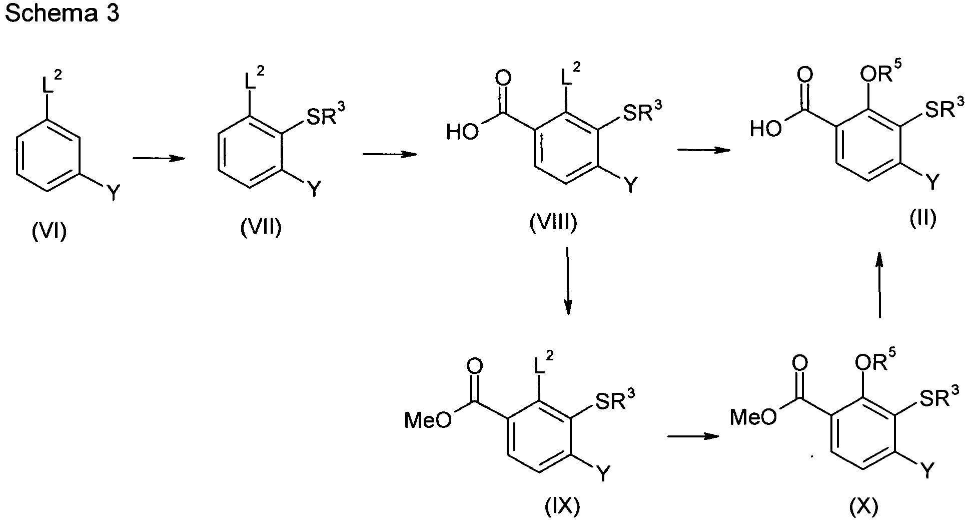

Beispielsweise können Verbindungen der Formel (VI), worin L² für einen orthodirigierenden Substituenten wie Fluor steht, mit Lithiumdiisopropylamid metalliert und dann mit einem Thiolierungsreagenz zu einer Verbindung der Formel (VII) umgesetzt werden. Durch eine weitere Metallierungreaktion mit beispielsweise n-Butyllithium und anschließender Carboxylierung gelangt man zu Benzoesäure (VIII). Solche Reaktionen sind beispielsweise aus Tetrahedron Letters 1992 (33), 49, S. 7499 - 7502; J. Heterocyclic Chem. 1999, 36, S. 1453 ff. und Angew. Chem. 2005, 117, 380 - 398) bekannt. Der Rest L² wird anschließend, gegebenenfalls nach Veresterung, gegen den Rest OR⁵ ausgetauscht. Durch Reaktion der Verbindungen (X) oder (II) mit Oxidationsmitteln wie meta-Chlorperbenzoesäure wird die Thiogruppe zu einer Sulfinyl- oder Sulfonylgruppe oxidiert.

Ein Austausch der Gruppe L² gegen OR⁵ kann gemäß Schema 4 auch auf der Stufe der 4-Benzoyl-5-hydroxypyrazole vorgenommen werden.

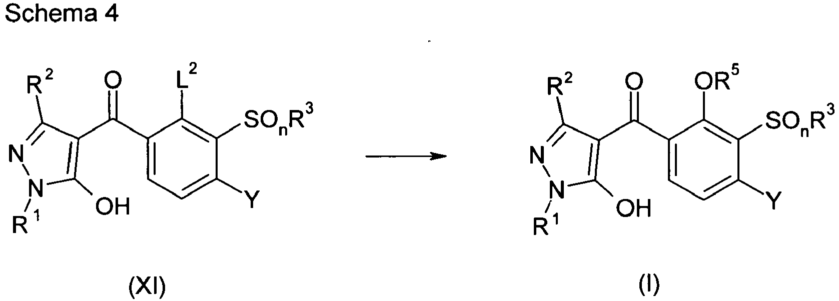

Der Thiorest in 3-Position kann gemäß Schema 5 auch via Metallierungsreaktionen aus Verbindungen (VI) eingeführt werden. Solche Reaktionen sind beispielsweise aus Synthesis 2006, 10, 1578 - 1589; Org. Lett. 8 (2006) 4, 765 - 768 und Angew. Chem. 2005, 117, 380 - 398 bekannt.

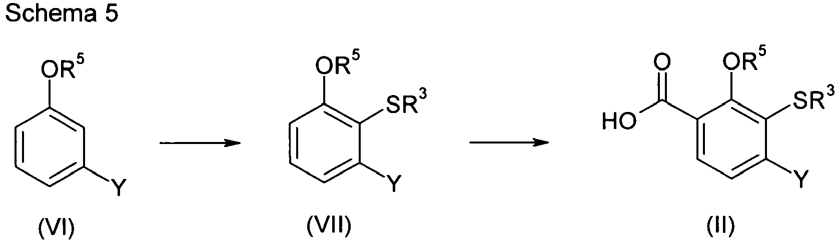

Gemäß Schema 6 können Verbindungen (II), worin X für Hydroxy steht, durch Acylierungsreaktionen Verbindungen (II), worin X für OCOR⁵ oder OSO₂R⁵ steht, hergestellt werden. Solche Reaktionen sind beispielsweise aus Houben-Weyl, Methoden der Organischen Chemie, Georg Thieme Verlag Stuttgart, Bd. VIII, vierte Auflage 1952, S. 543 ff. sowie Bd. IX, vierte Auflage 1955, S. 388 f. bekannt.

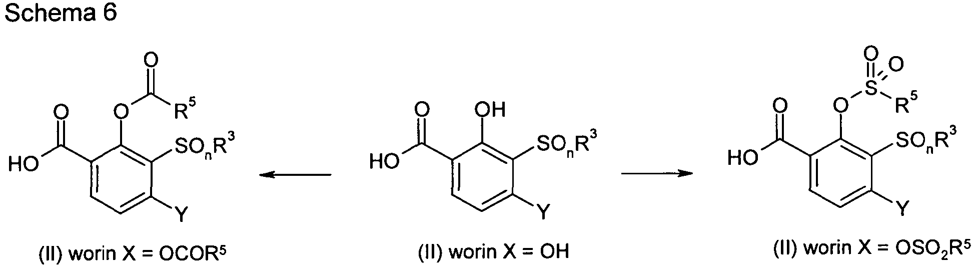

Der Alkylthiorest in 3-Position kann zum Sulfoxid oder Sulfon oxidiert werden. Hierfür bietet sich eine Anzahl an Oxidationssystemen an, beispielsweise Persäuren wie meta-Clorperbenzoesäure, die gegebenenfalls in situ erzeugt werden (zum Beispiel Peressigsäure im System Essigsäure/Wasserstoffperoxid/Natriumwolframat(VI)). Solche Reaktionen sind beispielsweise aus Houben-Weyl, Methoden der Organischen Chemie, Georg Thieme Verlag Stuttgart, Bd. E 11, Erweiterungs- und Folgebände zur vierten Auflage 1985, S. 702 ff., S. 718 ff. sowie S. 1194 ff.bekannt. Diese Oxidationsreaktionen können auch auf der Stufe der 4-Benzoyl-5-hydroxypyrazole oder der entsprechenden Enolester vorgenommen werden, siehe Schema 7.

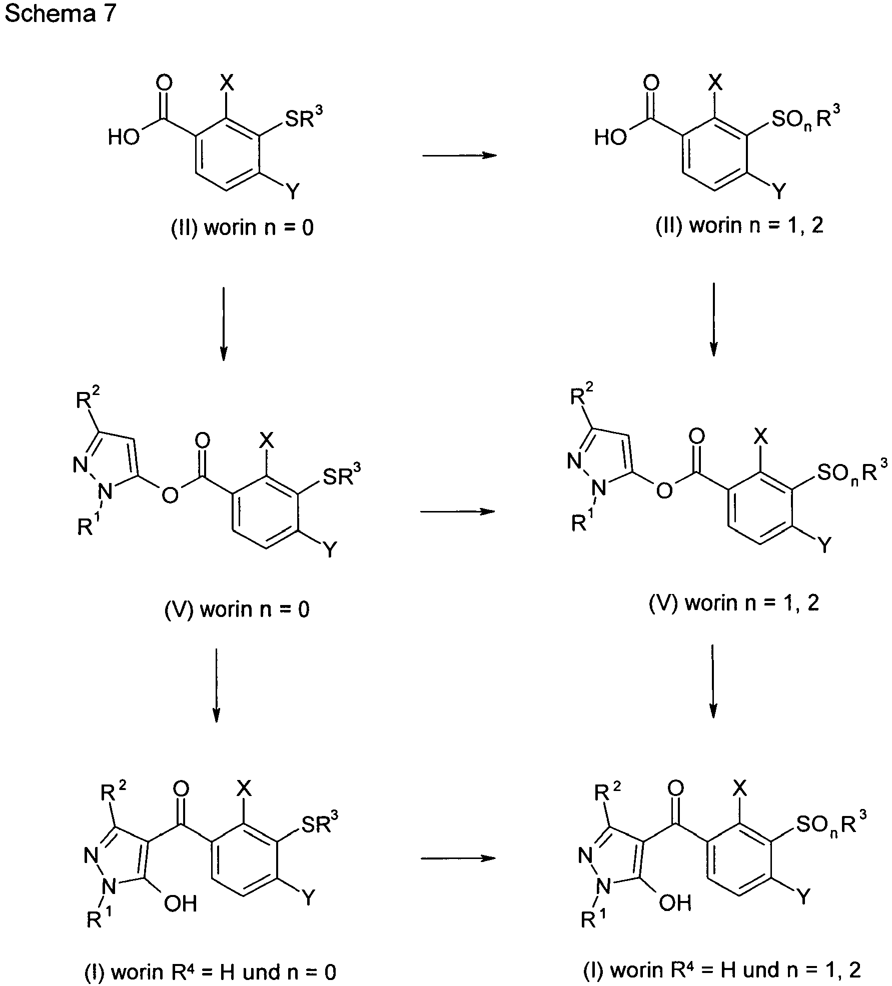

Es kann von Vorteil sein, die Reihenfolge der in den vorstehenden Schemata beschriebenen Reaktionsschritte zu vertauschen oder auch untereinander zu kombinieren. Die Aufarbeitung der jeweiligen Reaktionsmischungen erfolgt in der Regel nach bekannten Verfahren, beispielsweise durch Kristallisation, wässrigextraktive Aufarbeitung, durch chromatographische Methoden oder durch Kombination dieser Methoden.

Die Verbindungen der Formel (II) sind neu und ebenfalls Gegenstand vorliegender Erfindung.

Kollektionen aus Verbindungen der Formel (I) und/oder deren Salzen, die nach den oben genannten Reaktionen synthetisiert werden können, können auch in parallelisierter Weise hergestellt werden, wobei dies in manueller, teilweise automatisierter oder vollständig automatisierter Weise geschehen kann. Dabei ist es beispielsweise möglich, die Reaktionsdurchführung, die Aufarbeitung oder die Reinigung der Produkte bzw. Zwischenstufen zu automatisieren. Insgesamt wird hierunter eine Vorgehensweise verstanden, wie sie beispielsweise durch D. Tiebes in Combinatorial Chemistry - Synthesis, Analysis, Screening (Herausgeber Günther Jung), Verlag Wiley 1999, auf den Seiten 1 bis 34 beschrieben ist.

Zur parallelisierten Reaktionsdurchführung und Aufarbeitung können eine Reihe von im Handel erhältlichen Geräten verwendet werden, beispielsweise Calpyso-Reaktionsblöcke (Caylpso reaction blocks) der Firma Barnstead International, Dubuque, Iowa 52004-0797, USA oder Reaktionsstationen (reaction stations) der Firma Radleys, Shirehill, Saffron Walden, Essex, CB 11 3AZ, England oder MultiPROBE Automated Workstations der Firma Perkin Elmar, Waltham, Massachusetts 02451, USA. Für die parallelisierte Aufreinigung von Verbindungen der allgemeinen Formel (I) und deren Salzen beziehungsweise von bei der Herstellung anfallenden Zwischenprodukten stehen unter anderem Chromatographieapparaturen zur Verfügung, beispielsweise der Firma ISCO, Inc., 4700 Superior Street, Lincoln, NE 68504, USA.

Die aufgeführten Apparaturen führen zu einer modularen Vorgehensweise, bei der die einzelnen Arbeitsschritte automatisiert sind, zwischen den Arbeitsschritten jedoch manuelle Operationen durchgeführt werden müssen. Dies kann durch den Einsatz von teilweise oder vollständig integrierten Automationssystemen umgangen werden, bei denen die jeweiligen Automationsmodule beispielsweise durch Roboter bedient werden. Derartige Automationssysteme können zum Beispiel von der Firma Caliper, Hopkinton, MA 01748, USA bezogen werden.

Die Durchführung einzelner oder mehrerer Syntheseschritte kann durch den Einsatz von Polymer-supported reagents/Scavanger-Harze unterstützt werden. In der Fachliteratur sind eine Reihe von Versuchsprotokollen beschrieben, beispielsweise in ChemFiles, Vol. 4, No. 1, Polymer-Supported Scavengers and Reagents for Solution-Phase Synthesis (Sigma-Aldrich).

Neben den hier beschriebenen Methoden kann die Herstellung von Verbindungen der allgemeinen Formel (I) und deren Salzen vollständig oder partiell durch Festphasen unterstützte Methoden erfolgen. Zu diesem Zweck werden einzelne Zwischenstufen oder alle Zwischenstufen der Synthese oder einer für die entsprechende Vorgehensweise angepassten Synthese an ein Syntheseharz gebunden. Festphasen- unterstützte Synthesemethoden sind in der Fachliteratur hinreichend beschrieben, z.B. Barry A. Bunin in "The Combinatorial Index", Verlag Academic Press, 1998 und Combinatorial Chemistry - Synthesis, Analysis, Screening (Herausgeber Günther Jung), Verlag Wiley, 1999. Die Verwendung von Festphasen- unterstützten Synthesemethoden erlaubt eine Reihe von literaturbekannten Protokollen, die wiederum manuell oder automatisiert ausgeführt werden können. Die Reaktionen können beispielsweise mittels IRORI-Technologie in Mikroreaktoren (microreactors) der Firma Nexus Biosystems, 12140 Community Road, Poway, CA92064, USA durchgeführt werden.

Sowohl an fester als auch in flüssiger Phase kann die Durchführung einzelner oder mehrerer Syntheseschritte durch den Einsatz der Mikrowellen-Technologie unterstützt werden. In der Fachliteratur sind eine Reihe von Versuchsprotokollen beschrieben, beispielsweise in Microwaves in Organic and Medicinal Chemistry (Herausgeber C. O. Kappe und a. Stadler), Verlag Wiley, 2005.

Die Herstellung gemäß der hier beschriebenen Verfahren liefert Verbindungen der Formel (I) und deren Salze in Form von Substanzkollektionen, die Bibliotheken genannt werden. Gegenstand der vorliegenden Erfindung sind auch Bibliotheken, die mindestens zwei Verbindungen der Formel (I) und deren Salzen enthalten.

Die erfindungsgemäßen Verbindungen der Formel (I) (und/oder deren Salze), im folgenden zusammen als "erfindungsgemäße Verbindungen" bezeichnet, weisen eine ausgezeichnete herbizide Wirksamkeit gegen ein breites Spektrum wirtschaftlich wichtiger mono- und dikotyler annueller Schadpflanzen auf. Auch schwer bekämpfbare perennierende Schadpflanzen, die aus Rhizomen, Wurzelstöcken oder anderen Dauerorganen austreiben, werden durch die Wirkstoffe gut erfaßt.

Gegenstand der vorliegenden Erfindung ist daher auch ein Verfahren zur Bekämpfung von unerwünschten Pflanzen oder zur Wachstumsregulierung von Pflanzen, vorzugsweise in Pflanzenkulturen, worin eine oder mehrere erfindungsgemäße Verbindung(en) auf die Pflanzen (z.B. Schadpflanzen wie mono- oder dikotyle Unkräuter oder unerwünschte Kulturpflanzen), das Saatgut (z.B. Körner, Samen oder vegetative Vermehrungsorgane wie Knollen oder Sprossteile mit Knospen) oder die Fläche, auf der die Pflanzen wachsen (z.B. die Anbaufläche), ausgebracht werden. Dabei können die erfindungsgemäßen Verbindungen z.B. im Vorsaat- (ggf. auch durch Einarbeitung in den Boden), Vorauflauf- oder Nachauflaufverfahren ausgebracht werden. Im einzelnen seien beispielhaft einige Vertreter der mono- und dikotylen Unkrautflora genannt, die durch die erfindungsgemäßen Verbindungen kontrolliert werden können, ohne dass durch die Nennung eine Beschränkung auf bestimmte Arten erfolgen soll.

Monokotyle Schadpflanzen der Gattungen: Aegilops, Agropyron, Agrostis, Alopecurus, Apera, Avena, Brachiaria, Bromus, Cenchrus, Commelina, Cynodon, Cyperus, Dactyloctenium, Digitaria, Echinochloa, Eleocharis, Eleusine, Eragrostis, Eriochloa, Festuca, Fimbristylis, Heteranthera, Imperata, Ischaemum, Leptochloa, Lolium, Monochoria, Panicum, Paspalum, Phalaris, Phleum, Poa, Rottboellia, Sagittaria, Scirpus, Setaria, Sorghum.

Dikotyle Unkräuter der Gattungen: Abutilon, Amaranthus, Ambrosia, Anoda, Anthemis, Aphanes, Artemisia, Atriplex, Bellis, Bidens, Capsella, Carduus, Cassia, Centaurea, Chenopodium, Cirsium, Convolvulus, Datura, Desmodium, Emex, Erysimum, Euphorbia, Galeopsis, Galinsoga, Galium, Hibiscus, Ipomoea, Kochia, Lamium, Lepidium, Lindernia, Matricaria, Mentha, Mercurialis, Mullugo, Myosotis, Papaver, Pharbitis, Plantago, Polygonum, Portulaca, Ranunculus, Raphanus, Rorippa, Rotala, Rumex, Salsola, Senecio, Sesbania, Sida, Sinapis, Solanum, Sonchus, Sphenoclea, Stellaria, Taraxacum, Thlaspi, Trifolium, Urtica, Veronica, Viola, Xanthium.

Werden die erfindungsgemäßen Verbindungen vor dem Keimen auf die Erdoberfläche appliziert, so wird entweder das Auflaufen der Unkrautkeimlinge vollständig verhindert oder die Unkräuter wachsen bis zum Keimblattstadium heran, stellen jedoch dann ihr Wachstum ein und sterben schließlich nach Ablauf von drei bis vier Wochen vollkommen ab.

Bei Applikation der Wirkstoffe auf die grünen Pflanzenteile im Nachauflaufverfahren tritt nach der Behandlung Wachstumsstop ein und die Schadpflanzen bleiben in dem zum Applikationszeitpunkt vorhandenen Wachstumsstadium stehen oder sterben nach einer gewissen Zeit ganz ab, so dass auf diese Weise eine für die Kulturpflanzen schädliche Unkrautkonkurrenz sehr früh und nachhaltig beseitigt wird.

Obgleich die erfindungsgemäßen Verbindungen eine ausgezeichnete herbizide Aktivität gegenüber mono- und dikotylen Unkräutern aufweisen, werden Kulturpflanzen wirtschaftlich bedeutender Kulturen z.B. dikotyler Kulturen der Gattungen Arachis, Beta, Brassica, Cucumis, Cucurbita, Helianthus, Daucus, Glycine, Gossypium, Ipomoea, Lactuca, Linum, Lycopersicon, Nicotiana, Phaseolus, Pisum, Solanum, Vicia, oder monokotyler Kulturen der Gattungen Allium, Ananas, Asparagus, Avena, Hordeum, Oryza, Panicum, Saccharum, Secale, Sorghum, Triticale, Triticum, Zea, insbesondere Zea und Triticum, abhängig von der Struktur der jeweiligen erfindungsgemäßen Verbindung und deren Aufwandmenge nur unwesentlich oder gar nicht geschädigt. Die vorliegenden Verbindungen eignen sich aus diesen Gründen sehr gut zur selektiven Bekämpfung von unerwünschtem Pflanzenwuchs in Pflanzenkulturen wie landwirtschaftlichen Nutzpflanzungen oder Zierpflanzungen.

Darüberhinaus weisen die erfindungsgemäßen Verbindungen (abhängig von ihrer jeweiligen Struktur und der ausgebrachten Aufwandmenge) hervorragende wachstumsregulatorische Eigenschaften bei Kulturpflanzen auf. Sie greifen regulierend in den pflanzeneigenen Stoffwechsel ein und können damit zur gezielten Beeinflussung von Pflanzeninhaltsstoffen und zur Ernteerleichterung wie z.B. durch Auslösen von Desikkation und Wuchsstauchung eingesetzt werden. Desweiteren eignen sie sich auch zur generellen Steuerung und Hemmung von unerwünschtem vegetativen Wachstum, ohne dabei die Pflanzen abzutöten. Eine Hemmung des vegetativen Wachstums spielt bei vielen mono- und dikotylen Kulturen eine große Rolle, da beispielsweise die Lagerbildung hierdurch verringert oder völlig verhindert werden kann.

Aufgrund ihrer herbiziden und pflanzenwachstumsregulatorischen Eigenschaften können die Wirkstoffe auch zur Bekämpfung von Schadpflanzen in Kulturen von gentechnisch oder durch konventionelle Mutagenese veränderten Pflanzen eingesetzt werden. Die transgenen Pflanzen zeichnen sich in der Regel durch besondere vorteilhafte Eigenschaften aus, beispielsweise durch Resistenzen gegenüber bestimmten Pestiziden, vor allem bestimmten Herbiziden, Resistenzen gegenüber Pflanzenkrankheiten oder Erregern von Pflanzenkrankheiten wie bestimmten Insekten oder Mikroorganismen wie Pilzen, Bakterien oder Viren. Andere besondere Eigenschaften betreffen z. B. das Erntegut hinsichtlich Menge, Qualität, Lagerfähigkeit, Zusammensetzung und spezieller Inhaltsstoffe. So sind transgene Pflanzen mit erhöhtem Stärkegehalt oder veränderter Qualität der Stärke oder solche mit anderer Fettsäurezusammensetzung des Ernteguts bekannt.

Bevorzugt bezüglich transgener Kulturen ist die Anwendung der erfindungsgemäßen Verbindungen in wirtschaftlich bedeutenden transgenen Kulturen von Nutz- und Zierpflanzen, z. B. von Getreide wie Weizen, Gerste, Roggen, Hafer, Hirse, Reis und Mais oder auch Kulturen von Zuckerrübe, Baumwolle, Soja, Raps, Kartoffel, Tomate, Erbse und anderen Gemüsesorten.Vorzugsweise können die erfindungsgemäßen Verbindungen als Herbizide in Nutzpflanzenkulturen eingesetzt werden, welche gegenüber den phytotoxischen Wirkungen der Herbizide resistent sind bzw. gentechnisch resistent gemacht worden sind.

Bevorzugt ist die Anwendung der erfindungsgemäßen Verbindungen oder deren Salze in wirtschaftlich bedeutenden transgenen Kulturen von Nutz-und Zierpflanzen, z. B. von Getreide wie Weizen, Gerste, Roggen, Hafer, Hirse, Reis, Maniok und Mais oder auch Kulturen von Zuckerrübe, Baumwolle, Soja, Raps, Kartoffel, Tomate, Erbse und anderen Gemüsesorten. Vorzugsweise können die erfindungsgemäßen Verbindungen als Herbizide in Nutzpflanzenkulturen eingesetzt werden, welche gegenüber den phytotoxischen Wirkungen der Herbizide resistent sind bzw. gentechnisch resistent gemacht worden sind.

Herkömmliche Wege zur Herstellung neuer Pflanzen, die im Vergleich zu bisher vorkommenden Pflanzen modifizierte Eigenschaften aufweisen, bestehen beispielsweise in klassischen Züchtungsverfahren und der Erzeugung von Mutanten. Alternativ können neue Pflanzen mit veränderten Eigenschaften mit Hilfe gentechnischer Verfahren erzeugt werden (siehe z. B. EP-A-0221044, EP-A-0131624). Beschrieben wurden beispielsweise in mehreren Fällen
- gentechnische Veränderungen von Kulturpflanzen zwecks Modifikation der in den Pflanzen synthetisierten Stärke (z. B. WO 92/11376, WO 92/14827, WO 91/19806),
- transgene Kulturpflanzen, welche gegen bestimmte Herbizide vom Typ Glufosinate (vgl. z. B. EP-A-0242236, EP-A-242246) oder Glyphosate (WO 92/00377) oder der Sulfonylharnstoffe (EP-A-0257993, US-A-5013659) resistent sind,
- transgene Kulturpflanzen, beispielsweise Baumwolle, mit der Fähigkeit Bacillus thuringiensis-Toxine (Bt-Toxine) zu produzieren, welche die Pflanzen gegen bestimmte Schädlinge resistent machen (EP-A-0142924, EP-A-0193259).
- transgene Kulturpflanzen mit modifizierter Fettsäurezusammensetzung (WO 91/13972).
- gentechnisch veränderte Kulturpflanzen mit neuen Inhalts- oder Sekundärstoffen z. B. neuen Phytoalexinen, die eine erhöhte Krankheitsresistenz verursachen (EPA 309862, EPA0464461)
- gentechnisch veränderte Pflanzen mit reduzierter Photorespiration, die höhere Erträge und höhere Stresstoleranz aufweisen (EPA 0305398).
- Transgene Kulturpflanzen, die pharmazeutisch oder diagnostisch wichtige Proteine produzieren ("molecular pharming")
- transgene Kulturpflanzen, die sich durch höhere Erträge oder bessere Qualität auszeichnen
- transgene Kulturpflanzen die sich durch eine Kombinationen z. B. der o. g. neuen Eigenschaften auszeichnen ("gene stacking")

Zahlreiche molekularbiologische Techniken, mit denen neue transgene Pflanzen mit veränderten Eigenschaften hergestellt werden können, sind im Prinzip bekannt; siehe z. B. I. Potrykus und G. Spangenberg (eds.) Gene Transfer to Plants, Springer Lab Manual (1995), Springer Verlag Berlin, Heidelberg. oder Christou, "Trends in Plant Science" 1 (1996) 423-431).

Für derartige gentechnische Manipulationen können Nucleinsäuremoleküle in Plasmide eingebracht werden, die eine Mutagenese oder eine Sequenzveränderung durch Rekombination von DNA-Sequenzen erlauben. Mit Hilfe von Standardverfahren können z. B. Basenaustausche vorgenommen, Teilsequenzen entfernt oder natürliche oder synthetische Sequenzen hinzugefügt werden. Für die Verbindung der DNA-Fragmente untereinander können an die Fragmente Adaptoren oder Linker angesetzt werden, siehe z. B. Sambrook et al., 1989, Molecular Cloning, A Laboratory Manual, 2. Aufl. Cold Spring Harbor Laboratory Press, Cold Spring Harbor, NY; oder Winnacker "Gene und Klone", VCH Weinheim 2. Auflage 1996

Die Herstellung von Pflanzenzellen mit einer verringerten Aktivität eines Genprodukts kann beispielsweise erzielt werden durch die Expression mindestens einer entsprechenden antisense-RNA, einer sense-RNA zur Erzielung eines Cosuppressionseffektes oder die Expression mindestens eines entsprechend konstruierten Ribozyms, das spezifisch Transkripte des obengenannten Genprodukts spaltet. Hierzu können zum einen DNA-Moleküle verwendet werden, die die gesamte codierende Sequenz eines Genprodukts einschließlich eventuell vorhandener flankierender Sequenzen umfassen, als auch DNA-Moleküle, die nur Teile der codierenden Sequenz umfassen, wobei diese Teile lang genug sein müssen, um in den Zellen einen antisense-Effekt zu bewirken. Möglich ist auch die Verwendung von DNA-Sequenzen, die einen hohen Grad an Homologie zu den codiereden Sequenzen eines Genprodukts aufweisen, aber nicht vollkommen identisch sind.

Bei der Expression von Nucleinsäuremolekülen in Pflanzen kann das synthetisierte Protein in jedem beliebigen Kompartiment der pflanzlichen Zelle lokalisiert sein. Um aber die Lokalisation in einem bestimmten Kompartiment zu erreichen, kann z. B. die codierende Region mit DNA-Sequenzen verknüpft werden, die die Lokalisierung in einem bestimmten Kompartiment gewährleisten. Derartige Sequenzen sind dem Fachmann bekannt (siehe beispielsweise Braun et al., EMBO J. 11 (1992), 3219-3227; Wolter et al., Proc. Natl. Acad. Sci. USA 85 (1988), 846-850; Sonnewald et al., Plant J. 1 (1991), 95-106). Die Expression der Nukleinsäuremoleküle kann auch in den Organellen der Pflanzenzellen stattfinden.

Die transgenen Pflanzenzellen können nach bekannten Techniken zu ganzen Pflanzen regeneriert werden. Bei den transgenen Pflanzen kann es sich prinzipiell um Pflanzen jeder beliebigen Pflanzenspezies handeln, d.h., sowohl monokotyle als auch dikotyle Pflanzen.

So sind transgene Pflanzen erhältlich, die veränderte Eigenschaften durch Überexpression, Suppression oder Inhibierung homologer (= natürlicher) Gene oder Gensequenzen oder Expression heterologer (= fremder) Gene oder Gensequenzen aufweisen.

Vorzugsweise können die erfindungsgemäßen Verbindungen in transgenen Kulturen eingesetzt werden, welche gegen Wuchsstoffe, wie z. B. Dicamba oder gegen Herbizide, die essentielle Pflanzenenzyme, z. B. Acetolactatsynthasen (ALS), EPSP Synthasen, Glutaminsynthasen (GS) oder Hydroxyphenylpyruvat Dioxygenasen (HPPD) hemmen, respektive gegen Herbizide aus der Gruppe der Sulfonylharnstoffe, der Glyphosate, Glufosinate oder Benzoylisoxazole und analogen Wirkstoffe, resistent sind.

Bei der Anwendung der erfindungsgemäßen Wirkstoffe in transgenen Kulturen treten neben den in anderen Kulturen zu beobachtenden Wirkungen gegenüber Schadpflanzen oftmals Wirkungen auf, die für die Applikation in der jeweiligen transgenen Kultur spezifisch sind, beispielsweise ein verändertes oder speziell erweitertes Unkrautspektrum, das bekämpft werden kann, veränderte Aufwandmengen, die für die Applikation eingesetzt werden können, vorzugsweise gute Kombinierbarkeit mit den Herbiziden, gegenüber denen die transgene Kultur resistent ist, sowie Beeinflussung von Wuchs und Ertrag der transgenen Kulturpflanzen.

Gegenstand der Erfindung ist deshalb auch die Verwendung der erfindungsgemäßen Verbindungen als Herbizide zur Bekämpfung von Schadpflanzen in transgenen Kulturpflanzen.

Die erfindungsgemäßen Verbindungen können in Form von Spritzpulvern, emulgierbaren Konzentraten, versprühbaren Lösungen, Stäubemitteln oder Granulaten in den üblichen Zubereitungen angewendet werden. Gegenstand der Erfindung sind deshalb auch herbizide und pflanzenwachstumsregulierende Mittel, welche die erfindungsgemäßen Verbindungen enthalten.

Die erfindungsgemäßen Verbindungen können auf verschiedene Art formuliert werden, je nachdem welche biologischen und/oder chemisch-physikalischen Parameter vorgegeben sind. Als Formulierungsmöglichkeiten kommen beispielsweise in Frage: Spritzpulver (WP), wasserlösliche Pulver (SP), wasserlösliche Konzentrate, emulgierbare Konzentrate (EC), Emulsionen (EW), wie Öl-in-Wasser- und Wasser-in-Öl-Emulsionen, versprühbare Lösungen, Suspensionskonzentrate (SC), Dispersionen auf Öl- oder Wasserbasis, ölmischbare Lösungen, Kapselsuspensionen (CS), Stäubemittel (DP), Beizmittel, Granulate für die Streu- und Bodenapplikation, Granulate (GR) in Form von Mikro-, Sprüh-, Aufzugs- und Adsorptionsgranulaten, wasserdispergierbare Granulate (WG), wasserlösliche Granulate (SG), ULV-Formulierungen, Mikrokapseln und Wachse. Diese einzelnen Formulierungstypen sind im Prinzip bekannt und werden beispielsweise beschrieben in: Winnacker-Küchler, "Chemische Technologie", Band 7, C. Hanser Verlag München, 4. Aufl. 1986; Wade van Valkenburg, "Pesticide Formulations", Marcel Dekker, N.Y., 1973; K. Martens, "Spray Drying" Handbook, 3rd Ed. 1979, G. Goodwin Ltd. London.

Die notwendigen Formulierungshilfsmittel wie Inertmaterialien, Tenside, Lösungsmittel und weitere Zusatzstoffe sind ebenfalls bekannt und werden beispielsweise beschrieben in: Watkins, "Handbook of Insecticide Dust Diluents and Carriers", 2nd Ed., Darland Books, Caldwell N.J., H.v. Olphen, "Introduction to Clay Colloid Chemistry"; 2nd Ed., J. Wiley & Sons, N.Y.; C. Marsden, "Solvents Guide"; 2nd Ed., Interscience, N.Y. 1963; McCutcheon's "Detergents and Emulsifiers Annual", MC Publ. Corp., Ridgewood N.J.; Sisley and Wood, "Encyclopedia of Surface Active Agents", Chem. Publ. Co. Inc., N.Y. 1964; Schönfeldt, "Grenzflächenaktive Äthylenoxidaddukte", Wiss. Verlagsgesell., Stuttgart 1976; Winnacker-Küchler, "Chemische Technologie", Band 7, C. Hanser Verlag München, 4. Aufl. 1986.

Auf der Basis dieser Formulierungen lassen sich auch Kombinationen mit anderen pestizid wirksamen Stoffen, wie z.B. Insektiziden, Akariziden, Herbiziden, Fungiziden, sowie mit Safenern, Düngemitteln und/oder Wachstumsregulatoren herstellen, z.B. in Form einer Fertigformulierung oder als Tankmix.

Spritzpulver sind in Wasser gleichmäßig dispergierbare Präparate, die neben dem Wirkstoff außer einem Verdünnungs- oder Inertstoff noch Tenside ionischer und/oder nichtionischer Art (Netzmittel, Dispergiermittel), z. B. polyoxyethylierte Alkylphenole, polyoxethylierte Fettalkohole, polyoxethylierte Fettamine, Fettalkoholpolyglykolethersulfate, Alkansulfonate, Alkylbenzolsulfonate, ligninsulfonsaures Natrium, 2,2'-dinaphthylmethan-6,6'-disulfonsaures Natrium, dibutylnaphthalin-sulfonsaures Natrium oder auch oleoylmethyltaurinsaures Natrium enthalten. Zur Herstellung der Spritzpulver werden die herbiziden Wirkstoffe beispielsweise in üblichen Apparaturen wie Hammermühlen, Gebläsemühlen und Luftstrahlmühlen feingemahlen und gleichzeitig oder anschließend mit den Formulierungshilfsmitteln vermischt.

Emulgierbare Konzentrate werden durch Auflösen des Wirkstoffes in einem organischen Lösungsmittel z.B. Butanol, Cyclohexanon, Dimethylformamid, Xylol oder auch höhersiedenden Aromaten oder Kohlenwasserstoffen oder Mischungen der organischen Lösungsmittel unter Zusatz von einem oder mehreren Tensiden ionischer und/oder nichtionischer Art (Emulgatoren) hergestellt. Als Emulgatoren können beispielsweise verwendet werden: Alkylarylsulfonsaure Calzium-Salze wie Ca-Dodecylbenzolsulfonat oder nichtionische Emulgatoren wie Fettsäurepolyglykolester, Alkylarylpolyglykolether, Fettalkoholpolyglykolether, Propylenoxid-Ethylenoxid-Kondensationsprodukte, Alkylpolyether, Sorbitanester wie z.B. Sorbitanfettsäureester oder Polyoxethylensorbitanester wie z.B. Polyoxyethylensorbitanfettsäureester.

Stäubemittel erhält man durch Vermahlen des Wirkstoffes mit fein verteilten festen Stoffen, z.B. Talkum, natürlichen Tonen, wie Kaolin, Bentonit und Pyrophyllit, oder Diatomeenerde.

Suspensionskonzentrate können auf Wasser- oder Ölbasis sein. Sie können beispielsweise durch Naß-Vermahlung mittels handelsüblicher Perlmühlen und gegebenenfalls Zusatz von Tensiden, wie sie z.B. oben bei den anderen Formulierungstypen bereits aufgeführt sind, hergestellt werden.

Emulsionen, z.B. Öl-in-Wasser-Emulsionen (EW), lassen sich beispielsweise mittels Rührern, Kolloidmühlen und/oder statischen Mischern unter Verwendung von wäßrigen organischen Lösungsmitteln und gegebenenfalls Tensiden, wie sie z.B. oben bei den anderen Formulierungstypen bereits aufgeführt sind, herstellen.

Granulate können entweder durch Verdüsen des Wirkstoffes auf adsorptionsfähiges, granuliertes Inertmaterial hergestellt werden oder durch Aufbringen von Wirkstoffkonzentraten mittels Klebemitteln, z.B. Polyvinylalkohol, polyacrylsaurem Natrium oder auch Mineralölen, auf die Oberfläche von Trägerstoffen wie Sand, Kaolinite oder von granuliertem Inertmaterial. Auch können geeignete Wirkstoffe in der für die Herstellung von Düngemittelgranulaten üblichen Weise - gewünschtenfalls in Mischung mit Düngemitteln - granuliert werden.

Wasserdispergierbare Granulate werden in der Regel nach den üblichen Verfahren wie Sprühtrocknung, Wirbelbett-Granulierung, Teller-Granulierung, Mischung mit Hochgeschwindigkeitsmischern und Extrusion ohne festes Inertmaterial hergestellt.

Zur Herstellung von Teller-, Fließbett-, Extruder- und Sprühgranulate siehe z.B. Verfahren in "Spray-Drying Handbook" 3rd ed. 1979, G. Goodwin Ltd., London; J.E. Browning, "Agglomeration", Chemical and Engineering 1967, Seiten 147 ff; "Perry's Chemical Engineer's Handbook", 5th Ed., McGraw-Hill, New York 1973, S. 8-57.

Für weitere Einzelheiten zur Formulierung von Pflanzenschutzmitteln siehe z.B. G.C. Klingman, "Weed Control as a Science", John Wiley and Sons, Inc., New York, 1961, Seiten 81-96 und J.D. Freyer, S.A. Evans, "Weed Control Handbook", 5th Ed., Blackwell Scientific Publications, Oxford, 1968, Seiten 101-103.

Die agrochemischen Zubereitungen enthalten in der Regel 0.1 bis 99 Gew.-%, insbesondere 0.1 bis 95 Gew.-%, erfindungsgemäße Verbindungen. In Spritzpulvern beträgt die Wirkstoffkonzentration z.B. etwa 10 bis 90 Gew.-%, der Rest zu 100 Gew.-% besteht aus üblichen Formulierungsbestandteilen. Bei emulgierbaren Konzentraten kann die Wirkstoffkonzentration etwa 1 bis 90, vorzugsweise 5 bis 80 Gew.-% betragen. Staubförmige Formulierungen enthalten 1 bis 30 Gew.-% Wirkstoff, vorzugsweise meistens 5 bis 20 Gew.-% an Wirkstoff, versprühbare Lösungen enthalten etwa 0.05 bis 80, vorzugsweise 2 bis 50 Gew.-% Wirkstoff. Bei wasserdispergierbaren Granulaten hängt der Wirkstoffgehalt zum Teil davon ab, ob die wirksame Verbindung flüssig oder fest vorliegt und welche Granulierhilfsmittel, Füllstoffe usw. verwendet werden. Bei den in Wasser dispergierbaren Granulaten liegt der Gehalt an Wirkstoff beispielsweise zwischen 1 und 95 Gew.-%, vorzugsweise zwischen 10 und 80 Gew.-%.

Daneben enthalten die genannten Wirkstofformulierungen gegebenenfalls die jeweils üblichen Haft-, Netz-, Dispergier-, Emulgier-, Penetrations-, Konservierungs-, Frostschutz- und Lösungsmittel, Füll-, Träger- und Farbstoffe, Entschäumer, Verdunstungshemmer und den pH-Wert und die Viskosität beeinflussende Mittel.

Auf der Basis dieser Formulierungen lassen sich auch Kombinationen mit anderen pestizid wirksamen Stoffen, wie z.B. Insektiziden, Akariziden, Herbiziden, Fungiziden, sowie mit Safenern, Düngemitteln und/oder Wachstumsregulatoren herstellen, z.B. in Form einer Fertigformulierung oder als Tankmix.

Als Kombinationspartner für die erfindungsgemäßen Verbindungen in Mischungsformulierungen oder im Tank-Mix sind beispielsweise bekannte Wirkstoffe, die auf einer Inhibition von beispielsweise Acetolactat-Synthase, Acetyl-CoA-Carboxylase, Cellulose-Synthase, Enolpyruvylshikimat-3-phosphat-Synthase, Glutamin-Synthetase, p-Hydroxyphenylpyruvat-Dioxygenase, Phytoendesaturase, Photosystem I, Photosystem II, Protoporphyrinogen-Oxidase beruhen, einsetzbar, wie sie z.B. aus Weed Research 26 (1986) 441-445 oder "The Pesticide Manual", 14th edition, The British Crop Protection Council and the Royal Soc. of Chemistry, 2003 und dort zitierter Literatur beschrieben sind. Als bekannte Herbizide oder Pflanzenwachstumsregulatoren, die mit den erfindungsgemäßen Verbindungen kombiniert werden können, sind z.B. folgende Wirkstoffe zu nennen (die Verbindungen sind entweder mit dem "common name" nach der International Organization for Standardization (ISO) oder mit dem chemischen Namen oder mit der Codenummer bezeichnet) und umfassen stets sämtliche Anwendungsformen wie Säuren, Salze, Ester und Isomere wie Stereoisomere und optische Isomere. Dabei sind beispielhaft eine und zum Teil auch mehrere Anwendungsformen genannt:
Acetochlor, Acibenzolar, Acibenzolar-S-methyl, Acifluorfen, Acifluorfen-sodium, Aclonifen, Alachlor, Allidochlor, Alloxydim, Alloxydim-sodium, Ametryn, Amicarbazone, Amidochlor, Amidosulfuron, Aminocyclopyrachlor, Aminopyralid, Amitrole, Ammoniumsulfamat, Ancymidol, Anilofos, Asulam, Atrazine, Azafenidin, Azimsulfuron, Aziprotryn, BAH-043, BAS-140H, BAS-693H, BAS-714H, BAS-762H, BAS-776H, BAS-800H, Beflubutamid, Benazolin, Benazolin-ethyl, Bencarbazone, Benfluralin, Benfuresate, Bensulide, Bensulfuron-methyl, Bentazone, Benzfendizone, Benzobicyclon, Benzofenap, Benzofluor, Benzoylprop, Bicyclopyrone, Bifenox, Bilanafos, Bilanafos-natrium, Bispyribac, Bispyribac-natrium, Bromacil, Bromobutide, Bromofenoxim, Bromoxynil, Bromuron, Buminafos, Busoxinone, Butachlor, Butafenacil, Butamifos, Butenachlor, Butralin, Butroxydim, Butylate, Cafenstrole, Carbetamide, Carfentrazone, Carfentrazone-ethyl, Chlomethoxyfen, Chloramben, Chlorazifop, Chlorazifop-butyl, Chlorbromuron, Chlorbufam, Chlorfenac, Chlorfenacnatrium, Chlorfenprop, Chlorflurenol, Chlorflurenol-methyl, Chloridazon, Chlorimuron, Chlorimuron-ethyl, Chlormequat-chlorid, Chlornitrofen, Chlorophthalim, Chlorthaldimethyl, Chlorotoluron, Chlorsulfuron, Cinidon, Cinidon-ethyl, Cinmethylin, Cinosulfuron, Clethodim, Clodinafop Clodinafop-propargyl, Clofencet, Clomazone, Clomeprop, Cloprop, Clopyralid, Cloransulam, Cloransulam-methyl, Cumyluron, Cyanamide, Cyanazine, Cyclanilide, Cycloate, Cyclosulfamuron, Cycloxydim, Cycluron, Cyhalofop, Cyhalofop-butyl, Cyperquat, Cyprazine, Cyprazole, 2,4-D, 2,4-DB, Daimuron/Dymron, Dalapon, Daminozide, Dazomet, n-Decanol, Desmedipham, Desmetryn, Detosyl-Pyrazolate (DTP), Diallate, Dicamba, Dichlobenil, Dichlorprop, Dichlorprop-P, Diclofop, Diclofop-methyl, Diclofop-P-methyl, Diclosulam, Diethatyl, Diethatyl-ethyl, Difenoxuron, Difenzoquat, Diflufenican, Diflufenzopyr, Diflufenzopyrnatrium, Dimefuron, Dikegulac-sodium, Dimefuron, Dimepiperate, Dimethachlor, Dimethametryn, Dimethenamid, Dimethenamid-P, Dimethipin, Dimetrasulfuron, Dinitramine, Dinoseb, Dinoterb, Diphenamid, Dipropetryn, Diquat, Diquat-dibromide, Dithiopyr, Diuron, DNOC, Eglinazine-ethyl, Endothal, EPTC, Esprocarb, Ethalfluralin, Ethametsulfuron-methyl, Ethephon, Ethidimuron, Ethiozin, Ethofumesate, Ethoxyfen, Ethoxyfen-ethyl, Ethoxysulfuron, Etobenzanid, F-5331, d.h. N-[2-Chlor-4-fluor-5-[4-(3fluorpropyl)-4,5-dihydro-5-oxo-1H-tetrazol-1-yl]-phenyl]-ethansulfonamid, Fenoprop, Fenoxaprop, Fenoxaprop-P, Fenoxaprop-ethyl, Fenoxaprop-P-ethyl, Fenoxasulfone, Fentrazamide, Fenuron, Flamprop, Flamprop-M-isopropyl, Flamprop-M-methyl, Flazasulfuron, Florasulam, Fluazifop, Fluazifop-P, Fluazifopbutyl, Fluazifop-P-butyl, Fluazolate, Flucarbazone, Flucarbazone-sodium, Flucetosulfuron, Fluchloralin, Flufenacet (Thiafluamide), Flufenpyr, Flufenpyr-ethyl, Flumetralin, Flumetsulam, Flumiclorac, Flumiclorac-pentyl, Flumioxazin, Flumipropyn, Fluometuron, Fluorodifen, Fluoroglycofen, Fluoroglycofen-ethyl, Flupoxam, Flupropacil, Flupropanate, Flupyrsulfuron, Flupyrsulfuron-methyl-sodium, Flurenol, Flurenol-butyl, Fluridone, Flurochloridone, Fluroxypyr, Fluroxypyr-meptyl, Flurprimidol, Flurtamone, Fluthiacet, Fluthiacet-methyl, Fluthiamide, Fomesafen, Foramsulfuron, Forchlorfenuron, Fosamine, Furyloxyfen, Gibberellinsäure, Glufosinate, L-Glufosinate, L-Glufosinate-ammonium, Glufosinate-ammonium, Glyphosate, Glyphosate-isopropylammonium, H-9201, Halosafen, Halosulfuron, Halosulfuron-methyl, Haloxyfop, Haloxyfop-P, Haloxyfop-ethoxyethyl, Haloxyfop-P-ethoxyethyl, Haloxyfop-methyl, Haloxyfop-P-methyl, Hexazinone, HNPC-9908, HOK-201, HW-02, Imazamethabenz, Imazamethabenz-methyl, Imazamox, Imazapic, Imazapyr, Imazaquin, Imazethapyr, Imazosulfuron, Inabenfide, Indanofan, Indaziflam, Indolessigsäure (IAA), 4-Indol-3-ylbuttersäure (IBA), lodosulfuron, lodosulfuron-methyl-natrium, loxynil, Ipfencarbazone, Isocarbamid, Isopropalin, Isoproturon, Isouron, Isoxaben, Isoxachlortole, Isoxaflutole, Isoxapyrifop, IDH-100, KUH-043, KUH-071, Karbutilate, Ketospiradox, Lactofen, Lenacil, Linuron, Maleinsäurehydrazid, MCPA, MCPB, MCPB-methyl, -ethyl und -natrium, Mecoprop, Mecoprop-natrium, Mecoprop-butotyl, Mecoprop-P-butotyl, Mecoprop-P-dimethylammonium, Mecoprop-P-2-ethylhexyl, Mecoprop-P-kalium, Mefenacet, Mefluidide, Mepiquat-chlorid, Mesosulfuron, Mesosulfuron-methyl, Mesotrione, Methabenzthiazuron, Metam, Metamifop, Metamitron, Metazachlor, Metazosulfuron, Methazole, Methiozolin, Methoxyphenone, Methyldymron, 1-Methylcyclopropen, Methylisothiocyanat, Metobenzuron, Metobenzuron, Metobromuron, Metolachlor, S-Metolachlor, Metosulam, Metoxuron, Metribuzin, Metsulfuron, Metsulfuron-methyl, Molinate, Monalide, Monocarbamide, Monocarbamide-dihydrogensulfat, Monolinuron, Monosulfuron, Monuron, MT 128, MT-5950, d. h. N-[3-Chlor-4-(1-methylethyl)-phenyl]-2-methylpentanamid, NGGC-011, Naproanilide, Napropamide, Naptalam, NC-310, d.h. 4-(2,4-dichlorobenzoyl)-1-methyl-5-benzyloxypyrazole, Neburon, Nicosulfuron, Nipyraclofen, Nitralin, Nitrofen, Nitrophenolat-natrium (Isomerengemisch), Nitrofluorfen, Nonansäure, Norflurazon, Orbencarb, Orthosulfamuron, Oryzalin, Oxadiargyl, Oxadiazon, Oxasulfuron, Oxaziclomefone, Oxyfluorfen, Paclobutrazol, Paraquat, Paraquat-dichlorid, Pelargonsäure (Nonansäure), Pendimethalin, Pendralin, Penoxsulam, Pentanochlor, Pentoxazone, Perfluidone, Pethoxamid, Phenisopham, Phenmedipham, Phenmedipham-ethyl, Picloram, Picolinafen, Pinoxaden, Piperophos, Pirifenop, Pirifenop-butyl, Pretilachlor, Primisulfuron, Primisulfuron-methyl, Probenazole, Profluazol, Procyazine, Prodiamine, Prifluraline, Profoxydim, Prohexadione, Prohexadione-calcium, Prohydrojasmone, Prometon, Prometryn, Propachlor, Propanil, Propaquizafop, Propazine, Propham, Propisochlor, Propoxycarbazone, Propoxycarbazone-natrium, Propyrisulfuron, Propyzamide, Prosulfalin, Prosulfocarb, Prosulfuron, Prynachlor, Pyraclonil, Pyraflufen, Pyraflufen-ethyl, Pyrasulfotole, Pyrazolynate (Pyrazolate), Pyrazosulfuron-ethyl, Pyrazoxyfen, Pyribambenz, Pyribambenz-isopropyl, Pyribenzoxim, Pyributicarb, Pyridafol, Pyridate, Pyriftalid, Pyriminobac, Pyriminobacmethyl, Pyrimisulfan, Pyrithiobac, Pyrithiobac-natrium, Pyroxasulfone, Pyroxsulam, Quinclorac, Quinmerac, Quinoclamine, Quizalofop, Quizalofop-ethyl, Quizalofop-P, Quizalofop-P-ethyl, Quizalofop-P-tefuryl, Rimsulfuron, Saflufenacil, Secbumeton, Sethoxydim, Siduron, Simazine, Simetryn, SN-106279, Sulcotrione, Sulfallate (CDEC), Sulfentrazone, Sulfometuron, Sulfometuron-methyl, Sulfosate (Glyphosatetrimesium), Sulfosulfuron, SYN-523, SYP-249, SYP-298, SYP-300, Tebutam, Tebuthiuron, Tecnazene, Tefuryltrione, Tembotrione, Tepraloxydim, Terbacil, Terbucarb, Terbuchlor, Terbumeton, Terbuthylazine, Terbutryn, TH-547, Thenylchlor, Thiafluamide, Thiazafluron, Thiazopyr, Thidiazimin, Thidiazuron, Thiencarbazone, Thiencarbazone-methyl, Thifensulfuron, Thifensulfuron-methyl, Thiobencarb, Tiocarbazil, Topramezone, Tralkoxydim, Triallate, Triasulfuron, Triaziflam, Triazofenamide, Tribenuron, Tribenuron-methyl, Trichloressigsäure (TCA), Triclopyr, Tridiphane, Trietazine, Trifloxysulfuron, Trifloxysulfuron-natrium, Trifluralin, Triflusulfuron, Triflusulfuron-methyl, Trimeturon, Trinexapac, Trinexapacethyl, Tritosulfuron, Tsitodef, Uniconazole, Uniconazole-P, Vernolate, ZJ-0166, ZJ-0270, ZJ-0543, ZJ-0862 sowie die folgenden Verbindungen

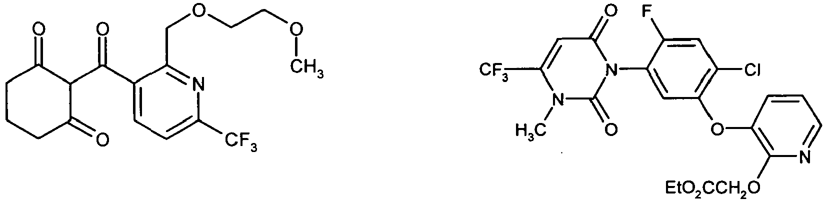

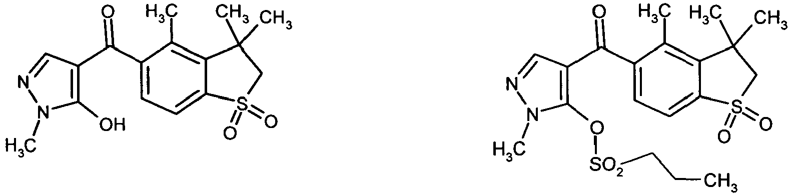

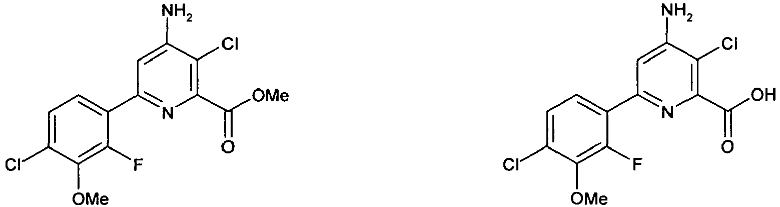

Zur Anwendung werden die in handelsüblicher Form vorliegenden Formulierungen gegebenenfalls in üblicher Weise verdünnt z.B. bei Spritzpulvern, emulgierbaren Konzentraten, Dispersionen und wasserdispergierbaren Granulaten mittels Wasser. Staubförmige Zubereitungen, Boden- bzw. Streugranulate sowie versprühbare Lösungen werden vor der Anwendung üblicherweise nicht mehr mit weiteren inerten Stoffen verdünnt.

Mit den äußeren Bedingungen wie Temperatur, Feuchtigkeit, der Art des verwendeten Herbizids, u.a. variiert die erforderliche Aufwandmenge der Verbindungen der Formel (I). Sie kann innerhalb weiter Grenzen schwanken, z.B. zwischen 0,001 und 1,0 kg/ha oder mehr Aktivsubstanz, vorzugsweise liegt sie jedoch zwischen 0,005 und 750 g/ha.

Die nachstehenden Beispiele erläutern die Erfindung.

### A. Chemische Beispiele

### Herstellung von 1-Ethyl-5-hydroxy-4-(2-methoxy-3-methylsulfonyl-4-(trifluormethyl) benzoyl)pyrazol (Tabellenbeispiel Nr. 2-21)

### Schritt 1: Synthese von 1-Fluor-2-methylthio-3-(trifluormethyl)benzol

Unter Inertgasatmosphäre wurden zu einer auf 0 °C gekühlten Lösung von 7.77 ml (55 mmol) Diisopropylamin in 100 ml wasserfreiem Tetrahydrofuran (THF) 32.8 ml (1.6M in Hexan, 52.5 mmol) n-Butyllithium getropft und die Lösung wurde nach 10 Minuten Rühren auf -78 °C abgekühlt. Bei dieser Temperatur wurden 8.21 g (50 mmol) 3-Fluorbenzotrifluorid zugegeben und die Reaktionsmischung wurde 1 h bei dieser Temperatur gerührt. Anschließend wurden 4.21 ml (55 mmol) Dimethyldisulfid zugetropft. Die Reaktionsmischung erwärmte sich innerhalb von ca. 3 h auf Raumtemperatur (RT), danach wurde sie erneut auf 0 °C gekühlt. Dann wurden 10 ml Wasser zugetropft und die Reaktionsmischung wurde auf ca. 1/4 ihres Volumens eingeengt. Der Rückstand wurde in Wasser und Dichlormethan aufgenommen, die Phasen wurden getrennt und die organische Phase wurde nacheinander mit Wasser, 10proz. Salzsäure, Wasser, gesättigter wässriger NaHCO₃-Lösung, Wasser und gesättigter wässriger NaCl-Lösung gewaschen und über Natriumsulfat getrocknet und filtriert. Das Lösungsmittel wurde abgetrennt und der Rückstand im Vakuum rektifiziert. Es wurden 8 g 1-Fluor-2-methylthio-3-(trifluormethyl)benzol mit einem Siedepunkt von 68 °C bei 6 mm Hg erhalten.

### Schritt 2: Synthese von 2-Fluor-3-methylthio-4-(trifluormethyl)benzoesäure

Unter Inertgasatmosphäre wurden zu einer auf -78 °C gekühlten Lösung von 7.98 g (38 mmol) 1-Fluor-2-methylthio-3-(trifluormethyl)benzol in 60 ml wasserfreiem Tetrahydrofuran 27.5 ml (1.6M in Hexan, 44 mmol) n-Butyllithium getropft, wobei die Temperatur der Reaktionsmischung -65 °C nicht übersteigen sollte. Das Gemisch rührte 3 h bei -78 °C und anschließend wurde bei dieser Temperatur ein Kohlendioxid-Strom in der Weise eingeleitet, dass die Temperatur der Reaktionsmischung -45 °C nicht überschritt. Schließlich wurde die Mischung auf RT erwärmt und danach erneut auf 0 °C abgekühlt. Zur Aufarbeitung wurde bei dieser Temperatur so lange Wasser tropfenweise zugegeben, bis sich der gebildete Niederschlag auflöste. Es wurde Diethylether zugegeben und die organische Phase wurde dreimal mit Wasser extrahiert. Die vereinigten wässrigen Phasen wurden mit 10proz. Salzsäure angesäuert. Die wässrige Phase wurde mehrfach mit Dichlormethan extrahiert, die vereinigten organischen Phase wurden mit gesättigter wässriger NaCl-Lösung gewaschen, über Natriumsulfat getrocknet und das Filtrat wurde schließlich vom Lösungsmittel befreit. Das so erhaltene Rohprodukt wurde aus Benzin/Essigsäureethylester umkristallisiert. Es wurden 6.8 g 2-Fluor-3-methylthio-4-(trifluormethyl)benzoesäure erhalten.

### Schritt 3: Synthese von 2-Fluor-3-methylthio-4-(trifluormethyl)benzoesäuremethylester

20.0 g (78.7 mmol) 2-Fluor-3-methylthio-4-(trifluormethyl)benzoesäure wurden in 200 ml Methanol mit 5 ml konzentrierter Schwefelsäure versetzt und das Gemisch wurde bis zum vollständigen Umsatz unter Rückfluss erhitzt. Das Gemisch wurde abgekühlt und das Lösungsmittel entfernt. Der Rückstand wurde in Wasser aufgenommen und das Gemisch zweimal mit Essigsäureethylester extrahiert. Die vereinigten organischen Phasen wurden einmal mit gesättigter wässriger NaHCO₃-Lösung gewaschen. Schließlich wurde die organische Phase getrocknet und das Filtrat wurde vom Lösungsmittel befreit. Als Rückstand wurden 20.5 g 2-Fluor-3-methylthio-4-(trifluormethyl)benzoesäuremethylester erhalten.

### Schritt 4: Synthese von 2-Methoxy-3-methylthio-4-(trifluormethyl)benzoesäuremethylester

Eine Mischung aus 19.9 g (74.2 mmol) 2-Fluor-3-methylthio-4-(trifluormethyl) benzoesäuremethylester und 40.1 g (30 Gew.-%, 223 mmol) Natriummethylat wurde in 250 ml Methanol 6 h unter Rückfluss erhitzt. Zur Aufarbeitung wurde das Gemisch eingeengt, der Rückstand in Wasser aufgenommen und das Gemisch mit Dichlormethan extrahiert. Die organische Phase wurde getrocknet und das Filtrat vom Lösungsmittel befreit. Als Rückstand wurden 15.9 g 2-Methoxy-3-methylthio-4-(trifluormethyl)benzoesäuremethylester gewonnen. Die wässrige Phase aus der extraktiven Aufarbeitung wurde mit verdünnter Salzsäure angesäuert und mit Essigsäureethylester extrahiert. Die organische Phase wurde getrocknet und das Filtrat vom Lösungsmittel befreit. Als Rückstand wurden zusätzlich 3.80 g 2-Methoxy-3-methylthio-4-(trifluormethyl)benzoesäuremethylester gewonnen.

### Schritt 5: Synthese von 2-Methoxy-3-methylthio-4-(trifluormethyl)benzoesäure (Tabellenbeispiel Nr. 7-13)

16.0 g (57.1 mmol) 2-Methoxy-3-methylthio-4-(trifluormethyl)benzoesäuremethylester wurden in 160 ml Methanol mit 16 ml 20proz. wässriger Natronlauge versetzt und 4 h bei RT gerührt. Zur Aufarbeitung wurde das Gemisch vom Lösungsmittel befreit und der Rückstand in wenig Wasser aufgenommen. Das Gemisch wurde im Eisbad abgekühlt und anschließend mit verdünnter HCl angesäuert. Das Gemisch wurde 5 Min. bei RT gerührt und anschließend der Inhalt filtriert. Als Rückstand wurden 15.3 g 2-Methoxy-3-methylthio-4-(trifluormethyl)benzoesäure gewonnen.

### Schritt 6: Synthese von 1-Ethyl-5-(2-methoxy-3-methylthio-4-(trifluormethyl) benzoyloxy)pyrazol

430 mg (80 Gew.-% Reinheit, 1.29 mmol) 2-Methoxy-3-methylthio-4-(trifluormethyl)-benzoesäure wurden in 20 ml Dichlormethan nacheinander mit 174 mg (1.55 mmol) 1-Ethyl-5-hydroxypyrazol und portionsweise mit 322 mg (1.68 mmol) 1-(3-Dimethylaminopropyl)-3-ethylcarbodiimidhydrochlorid versetzt. Das Gemisch wurde bis zum vollständigen Umsatz bei RT gerührt. Zur Aufarbeitung wurde das Gemisch mit 3 ml 1N Salzsäure versetzt und nach der Phasentrennung wurde die organische Phase vom Lösungsmittel befreit. Der Rückstand wurde chromatographisch gereinigt, wobei 450 mg 1-Ethyl-5-(2'-methoxy-3'-methylthio-4'-(trifluormethyl)-benzoyloxy)pyrazol mit einer Reinheit von 85 Gew.-% isoliert wurden.

### Schritt 7: Synthese von 1-Ethyl-5-(2-methoxy-3-methylsulfonyl-4-(trifluormethyl)-benzoyloxy)pyrazol

150 mg (85 Gew.-%, 0.354 mmol) 1-Ethyl-5-(2-methoxy-3'-methylthio-4-(trifluor methyl)benzoyloxy)pyrazol wurden in 10 ml Dichlormethan mit 262 mg (70 Gew.-%, 1.06 mmol) meta-Chlorperbenzoesäure versetzt. Das Gemisch wurde 16 h bei RT gerührt. Zur Aufarbeitung wurde 10proz. wässrige Natriumhydrogensulfit-Lösung gegeben. Nach dem Nachweis der Abwesenheit von Peroxiden wurde die organische Phase zweimal mit gesättigter wässriger NatriumhydrogencarbonatLösung gewaschen. Nach der Phasentrennung wurde das Lösungsmittel entfernt und der Rückstand wurde chromatographisch gereinigt. Es wurden 70 mg 1-Ethyl-5-(2-methoxy-3-methylsulfonyl-4-(trifluormethyl)benzoyloxy)pyrazol isoliert.

### Schritt 8: Synthese von 1-Ethyl-5-hydroxy-4-(2'-methoxy-3-methylsulfonyl-4-(trifluor-methyl)benzoyl)pyrazol (Tabellenbeispiel Nr. 2-21)

70 mg (0.178 mmol) 1-Ethyl-5-(2-methoxy-3-methylsulfonyl-4-(trifluormethyl)benzoyl-oxy)pyrazol wurden in 10 ml Acetonitril nacheinander mit 36 mg (0.357 mmol) Triethylamin, einer Spatelspitze Kaliumcyanid sowie sechs Tropfen Acetoncyanhydrin versetzt. Das Gemisch wurde 16 h bei RT gerührt. Zur Aufarbeitung wurde das Lösungsmittel abgetrennt. Der Rückstand wurde in 15 ml Dichlormethan aufgenommen und mit 3 ml 1 N Salzsäure versetzt. Nach der Phasentrennung wurde das Lösungsmittel abgetrennt und der Rückstand wurde chromatographisch gereinigt, wobei 34.7 mg 1-Ethyl-5-hydroxy-4-(2-methoxy-3-methylsulfonyl-4-(trifluormethyl)benzoyl)pyrazol gewonnen wurden.

Die in nachfolgenden Tabellen aufgeführten Beispiele wurden analog oben genannten Methoden hergestellt beziehungsweise sind analog oben genannten Methoden erhältlich. Diese Verbindungen sind ganz besonders bevorzugt.

Die verwendeten Abkürzungen bedeuten:

| | | | |
|---|---|---|---|
| Et = Ethyl | Me = Methyl | Pr = Propyl | Ph = Phenyl |

**Tabelle 1: Erfindungsgemäße Verbindungen der allgemeinen Formel (I), worin R¹ für Methyl und R² sowie R⁴ für jeweils Wasserstoff stehen.**

| | | | | | |
|---|---|---|---|---|---|
| 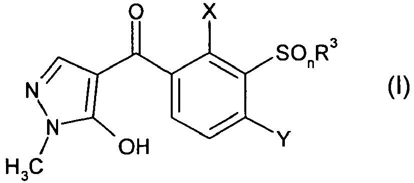 | | | | | |

| Nr. | X | R³ | n | Y | Physikalische Daten: ¹H-NMR: δ [CDCl₃] |
|---|---|---|---|---|---|
| 1-1 | OH | Me | 0 | CF₃ | |
| 1-2 | OH | Et | 0 | CF₃ | |
| 1-3 | OH | n-Pr | 0 | CF₃ | |
| 1-4 | OH | i-Pr | 0 | CF₃ | |
| 1-5 | OH | Me | 1 | CF₃ | |
| 1-6 | OH | Et | 1 | CF₃ | |
| 1-7 | OH | n-Pr | 1 | CF₃ | |
| 1-8 | OH | i-Pr | 1 | CF₃ | |
| 1-9 | OH | Me | 2 | CF₃ | |
| 1-10 | OH | Et | 2 | CF₃ | |
| 1-11 | OH | n-Pr | 2 | CF₃ | |
| 1-12 | OH | i-Pr | 2 | CF₃ | |
| 1-13 | OMe | Me | 0 | CF₃ | 7.56 (d, 1H), 7.51 (d, 1H), 7.43 (s, 1H), 3.89 (s, 3H), 3.71 (s, 3H), 2.49 (s, 3H) |
| 1-14 | OMe | Et | 0 | CF₃ | |
| 1-15 | OMe | n-Pr | 0 | CF₃ | |
| 1-16 | OMe | i-Pr | 0 | CF₃ | |
| 1-17 | OMe | Me | 1 | CF₃ | 7.72 (d, 1H), 7.60 (d, 1H), 7.50 (s, 1H), 3.92 (s, 3H), 3.72 (s, 3H), 3.18 (s, 3H) |
| 1-18 | OMe | Et | 1 | CF₃ | |
| 1-19 | OMe | n-Pr | 1 | CF₃ | |
| 1-20 | OMe | i-Pr | 1 | CF₃ | |
| 1-21 | OMe | Me | 2 | CF₃ | 7.82 (d, 1H), 7.77 (d, 1H), 7.47 (s, 1H), 3.90 (s, 3H), 3.72 (s, 3H), 3.40 (s, 3H) |
| 1-22 | OMe | Et | 2 | CF₃ | |
| 1-23 | OMe | n-Pr | 2 | CF₃ | |
| 1-24 | OMe | i-Pr | 2 | CF₃ | |
| 1-25 | OEt | Me | 0 | CF₃ | 7.54 (d, 1H), 7.51 (d, 1H), 7.47 (s, 1H), 4.08 (q, 2H), 3.71 (s, 3H), 2.51 (s, 3H), 1.31 (t, 3H) |
| 1-26 | OEt | Et | 0 | CF₃ | |
| 1-27 | OEt | n-Pr | 0 | CF₃ | |
| 1-28 | OEt | i-Pr | 0 | CF₃ | |
| 1-29 | OEt | Me | 1 | CF₃ | 7.71 (d, 1H), 7.60 (d, 1H), 7.52 (s, 1H), 4.25 (m, 1H), 3.96 (m, 1H), 3.72 (s, 3H), 3.18 (s, 3H), 1.31 (t, 3H) |
| 1-30 | OEt | Et | 1 | CF₃ | |
| 1-31 | OEt | n-Pr | 1 | CF₃ | |
| 1-32 | OEt | i-Pr | 1 | CF₃ | |
| 1-33 | OEt | Me | 2 | CF₃ | 7.80 (d, 1H), 7.75 (d, 1H), 7.47 (s, 1H), 4.11 (q, 2H), 3.72 (s, 3H), 3.42 (s, 3H), 1.32 (t, 3H) |
| 1-34 | OEt | Et | 2 | CF₃ | |
| 1-35 | OEt | n-Pr | 2 | CF₃ | |
| 1-36 | OEt | i-Pr | 2 | CF₃ | |
| 1-37 | O-CH₂₋c-Pr | Me | 0 | CF₃ | |
| 1-38 | O-CH₂₋c-Pr | Et | 0 | CF₃ | |
| 1-39 | O-CH₂₋c-Pr | n-Pr | 0 | CF₃ | |
| 1-40 | O-CH₂₋c-Pr | i-Pr | 0 | CF₃ | |
| 1-41 | O-CH₂₋c-Pr | Me | 1 | CF₃ | |
| 1-42 | O-CH₂₋c-Pr | Et | 1 | CF₃ | |
| 1-43 | O-CH₂₋c-Pr | n-Pr | 1 | CF₃ | |
| 1-44 | O-CH₂₋c-Pr | i-Pr | 1 | CF₃ | |
| 1-45 | O-CH₂₋c-Pr | Me | 2 | CF₃ | |
| 1-46 | O-CH₂₋c-Pr | Et | 2 | CF₃ | |
| 1-47 | O-CH₂₋c-Pr | n-Pr | 2 | CF₃ | |
| 1-48 | O-CH₂₋c-Pr | i-Pr | 2 | CF₃ | |
| 1-49 | OCH₂CH₂OMe | Me | 0 | CF₃ | 7.56 (d, 1H), 7.52 (d, 1H), 7.44 (s, 1H), 4.23 (m, 2H), 3.71 (s, 3H), 3.61 (m, 2H), 3.30 (s, 3H), 2.52 (s, 3H) |
| 1-50 | OCH₂CH₂OMe | Et | 0 | CF₃ | |
| 1-51 | OCH₂CH₂OMe | n-Pr | 0 | CF₃ | |
| 1-52 | OCH₂CH₂OMe | i-Pr | 0 | CF₃ | |
| 1-53 | OCH₂CH₂OMe | Me | 1 | CF₃ | 7.70 (d, 1H), 7.60 (d, 1H), 7.45 (s, 1H), 4.38 (m, 1H), 4.11 (m, 1H), |
| | | | | | 3.76 (m, 1H), 3.72 (s, 3H), 3.57 (m, 1H), 3.30 (s, 3H), 3.19 (s, 3H) |
| 1-54 | OCH₂CH₂OMe | Et | 1 | CF₃ | |
| 1-55 | OCH₂CH₂OMe | n-Pr | 1 | CF₃ | |
| 1-56 | OCH₂CH₂OMe | i-Pr | 1 | CF₃ | |
| 1-57 | OCH₂CH₂OMe | Me | 2 | CF₃ | 7.80 (d, 1H), 7.77 (d, 1H), 7.45 (s, 1H), 4.22 (t, 2H), 3.74 (s, 3H), 3.64 (m, 2H), 3.47 (s, 3H), 3.32 (s, 3H) |
| 1-58 | OCH₂CH₂OMe | Et | 2 | CF₃ | |
| 1-59 | OCH₂CH₂OMe | n-Pr | 2 | CF₃ | |
| 1-60 | OCH₂CH₂OMe | i-Pr | 2 | CF₃ | |
| 1-61 | OCH₂CH₂SMe | Me | 0 | CF₃ | |
| 1-62 | OCH₂CH₂SMe | Et | 0 | CF₃ | |
| 1-63 | OCH₂CH₂SMe | n-Pr | 0 | CF₃ | |
| 1-64 | OCH₂CH₂SMe | i-Pr | 0 | CF₃ | |
| 1-65 | OCH₂CH₂SMe | Me | 1 | CF₃ | |
| 1-66 | OCH₂CH₂SMe | Et | 1 | CF₃ | |
| 1-67 | OCH₂CH₂SMe | n-Pr | 1 | CF₃ | |
| 1-68 | OCH₂CH₂SMe | i-Pr | 1 | CF₃ | |
| 1-69 | OCH₂CH₂SMe | Me | 2 | CF₃ | |
| 1-70 | OCH₂CH₂SMe | Et | 2 | CF₃ | |
| 1-71 | OCH₂CH₂SMe | n-Pr | 2 | CF₃ | |
| 1-72 | OCH₂CH₂SMe | i-Pr | 2 | CF₃ | |
| 1-73 | OCH₂CH₂SO₂Me | Me | 0 | CF₃ | |
| 1-74 | OCH₂CH₂SO₂Me | Et | 0 | CF₃ | |
| 1-75 | OCH₂CH₂SO₂Me | n-Pr | 0 | CF₃ | |
| 1-76 | OCH₂CH₂SO₂Me | i-Pr | 0 | CF₃ | |
| 1-77 | OCH₂CH₂SO₂Me | Me | 1 | CF₃ | |
| 1-78 | OCH₂CH₂SO₂Me | Et | 1 | CF₃ | |
| 1-79 | OCH₂CH₂SO₂Me | n-Pr | 1 | CF₃ | |
| 1-80 | OCH₂CH₂SO₂Me | i-Pr | 1 | CF₃ | |
| 1-81 | OCH₂CH₂SO₂Me | Me | 2 | CF₃ | |
| 1-82 | OCH₂CH₂SO₂Me | Et | 2 | CF₃ | |
| 1-83 | OCH₂CH₂SO₂Me | n-Pr | 2 | CF₃ | |
| 1-84 | OCH₂CH₂SO₂Me | i-Pr | 2 | CF₃ | |
| 1-85 | OCOMe | Me | 0 | CF₃ | |
| 1-86 | OCOMe | Et | 0 | CF₃ | |
| 1-87 | OCOMe | n-Pr | 0 | CF₃ | |
| 1-88 | OCOMe | i-Pr | 0 | CF₃ | |
| 1-89 | OCOMe | Me | 1 | CF₃ | |
| 1-90 | OCOMe | Et | 1 | CF₃ | |
| 1-91 | OCOMe | n-Pr | 1 | CF₃ | |
| 1-92 | OCOMe | i-Pr | 1 | CF₃ | |
| 1-93 | OCOMe | Me | 2 | CF₃ | |
| 1-94 | OCOMe | Et | 2 | CF₃ | |
| 1-95 | OCOMe | n-Pr | 2 | CF₃ | |
| 1-96 | OCOMe | i-Pr | 2 | CF₃ | |
| 1-97 | OSO₂Me | Me | 0 | CF₃ | |
| 1-98 | OSO₂Me | Et | 0 | CF₃ | |
| 1-99 | OSO₂Me | n-Pr | 0 | CF₃ | |
| 1-100 | OSO₂Me | i-Pr | 0 | CF₃ | |
| 1-101 | OSO₂Me | Me | 1 | CF₃ | |
| 1-102 | OSO₂Me | Et | 1 | CF₃ | |
| 1-103 | OSO₂Me | n-Pr | 1 | CF₃ | |
| 1-104 | OSO₂Me | i-Pr | 1 | CF₃ | |
| 1-105 | OSO₂Me | Me | 2 | CF₃ | |
| 1-106 | OSO₂Me | Et | 2 | CF₃ | |
| 1-107 | OSO₂Me | n-Pr | 2 | CF₃ | |
| 1-108 | OSO₂Me | i-Pr | 2 | CF₃ | |
| 1-109 | OMe | Me | 0 | C₂F₅ | |
| 1-110 | OMe | Et | 0 | C₂F₅ | |
| 1-111 | OMe | n-Pr | 0 | C₂F₅ | |
| 1-112 | OMe | i-Pr | 0 | C₂F₅ | |
| 1-113 | OMe | Me | 1 | C₂F₅ | |
| 1-114 | OMe | Et | 1 | C₂F₅ | |
| 1-115 | OMe | n-Pr | 1 | C₂F₅ | |
| 1-116 | OMe | i-Pr | 1 | C₂F₅ | |
| 1-117 | OMe | Me | 2 | C₂F₅ | |
| 1-118 | OMe | Et | 2 | C₂F₅ | |
| 1-119 | OMe | n-Pr | 2 | C₂F₅ | |
| 1-120 | OMe | i-Pr | 2 | C₂F₅ | |
| 1-121 | OEt | Me | 0 | C₂F₅ | |
| 1-122 | OEt | Et | 0 | C₂F₅ | |
| 1-123 | OEt | n-Pr | 0 | C₂F₅ | |
| 1-124 | OEt | i-Pr | 0 | C₂F₅ | |
| 1-125 | OEt | Me | 1 | C₂F₅ | |
| 1-126 | OEt | Et | 1 | C₂F₅ | |
| 1-127 | OEt | n-Pr | 1 | C₂F₅ | |
| 1-128 | OEt | i-Pr | 1 | C₂F₅ | |
| 1-129 | OEt | Me | 2 | C₂F₅ | |
| 1-130 | OEt | Et | 2 | C₂F₅ | |
| 1-131 | OEt | n-Pr | 2 | C₂F₅ | |
| 1-132 | OEt | i-Pr | 2 | C₂F₅ | |
| 1-133 | O-CH₂₋c-Pr | Me | 0 | C₂F₅ | |
| 1-134 | O-CH₂₋c-Pr | Et | 0 | C₂F₅ | |
| 1-135 | O-CH₂₋c-Pr | n-Pr | 0 | C₂F₅ | |
| 1-136 | O-CH₂₋c-Pr | i-Pr | 0 | C₂F₅ | |
| 1-137 | O-CH₂₋c-Pr | Me | 1 | C₂F₅ | |
| 1-138 | O-CH₂₋c-Pr | Et | 1 | C₂F₅ | |
| 1-139 | O-CH₂₋c-Pr | n-Pr | 1 | C₂F₅ | |
| 1-140 | O-CH₂₋c-Pr | i-Pr | 1 | C₂F₅ | |
| 1-141 | O-CH₂₋c-Pr | Me | 2 | C₂F₅ | |
| 1-142 | O-CH₂₋c-Pr | Et | 2 | C₂F₅ | |
| 1-143 | O-CH₂₋c-Pr | n-Pr | 2 | C₂F₅ | |
| 1-144 | O-CH₂₋c-Pr | i-Pr | 2 | C₂F₅ | |
| 1-145 | OCH₂CH₂OMe | Me | 0 | C₂F₅ | |
| 1-146 | OCH₂CH₂OMe | Et | 0 | C₂F₅ | |
| 1-147 | OCH₂CH₂OMe | n-Pr | 0 | C₂F₅ | |
| 1-148 | OCH₂CH₂OMe | i-Pr | 0 | C₂F₅ | |
| 1-149 | OCH₂CH₂OMe | Me | 1 | C₂F₅ | |
| 1-150 | OCH₂CH₂OMe | Et | 1 | C₂F₅ | |
| 1-151 | OCH₂CH₂OMe | n-Pr | 1 | C₂F₅ | |
| 1-152 | OCH₂CH₂OMe | i-Pr | 1 | C₂F₅ | |
| 1-153 | OCH₂CH₂OMe | Me | 2 | C₂F₅ | |
| 1-154 | OCH₂CH₂OMe | Et | 2 | C₂F₅ | |
| 1-155 | OCH₂CH₂OMe | n-Pr | 2 | C₂F₅ | |
| 1-156 | OCH₂CH₂OMe | i-Pr | 2 | C₂F₅ | |
| 1-157 | OCH₂CH₂SMe | Me | 0 | C₂F₅ | |
| 1-158 | OCH₂CH₂SMe | Et | 0 | C₂F₅ | |
| 1-159 | OCH₂CH₂SMe | n-Pr | 0 | C₂F₅ | |
| 1-160 | OCH₂CH₂SMe | i-Pr | 0 | C₂F₅ | |
| 1-161 | OCH₂CH₂SMe | Me | 1 | C₂F₅ | |
| 1-162 | OCH₂CH₂SMe | Et | 1 | C₂F₅ | |
| 1-163 | OCH₂CH₂SMe | n-Pr | 1 | C₂F₅ | |
| 1-164 | OCH₂CH₂SMe | i-Pr | 1 | C₂F₅ | |
| 1-165 | OCH₂CH₂SMe | Me | 2 | C₂F₅ | |
| 1-166 | OCH₂CH₂SMe | Et | 2 | C₂F₅ | |
| 1-167 | OCH₂CH₂SMe | n-Pr | 2 | C₂F₅ | |
| 1-168 | OCH₂CH₂SMe | i-Pr | 2 | C₂F₅ | |
| 1-169 | OCH₂CH₂SO₂Me | Me | 0 | C₂F₅ | |
| 1-170 | OCH₂CH₂SO₂Me | Et | 0 | C₂F₅ | |
| 1-171 | OCH₂CH₂SO₂Me | n-Pr | 0 | C₂F₅ | |
| 1-172 | OCH₂CH₂SO₂Me | i-Pr | 0 | C₂F₅ | |
| 1-173 | OCH₂CH₂SO₂Me | Me | 1 | C₂F₅ | |
| 1-174 | OCH₂CH₂SO₂Me | Et | 1 | C₂F₅ | |
| 1-175 | OCH₂CH₂SO₂Me | n-Pr | 1 | C₂F₅ | |
| 1-176 | OCH₂CH₂SO₂Me | i-Pr | 1 | C₂F₅ | |
| 1-177 | OCH₂CH₂SO₂Me | Me | 2 | C₂F₅ | |
| 1-178 | OCH₂CH₂SO₂Me | Et | 2 | C₂F₅ | |
| 1-179 | OCH₂CH₂SO₂Me | n-Pr | 2 | C₂F₅ | |
| 1-180 | OCH₂CH₂SO₂Me | i-Pr | 2 | C₂F₅ | |
| 1-181 | OMe | Me | 0 | CCl₃ | |
| 1-182 | OMe | Et | 0 | CCl₃ | |
| 1-183 | OMe | n-Pr | 0 | CCl₃ | |
| 1-184 | OMe | i-Pr | 0 | CCl₃ | |
| 1-185 | OMe | Me | 1 | CCl₃ | |
| 1-186 | OMe | Et | 1 | CCl₃ | |
| 1-187 | OMe | n-Pr | 1 | CCl₃ | |
| 1-188 | OMe | i-Pr | 1 | CCl₃ | |
| 1-189 | OMe | Me | 2 | CCl₃ | |
| 1-190 | OMe | Et | 2 | CCl₃ | |
| 1-191 | OMe | n-Pr | 2 | CCl₃ | |
| 1-192 | OMe | i-Pr | 2 | CCl₃ | |
| 1-193 | OEt | Me | 0 | CCl₃ | |
| 1-194 | OEt | Et | 0 | CCl₃ | |
| 1-195 | OEt | n-Pr | 0 | CCl₃ | |
| 1-196 | OEt | i-Pr | 0 | CCl₃ | |
| 1-197 | OEt | Me | 1 | CCl₃ | |
| 1-198 | OEt | Et | 1 | CCl₃ | |
| 1-199 | OEt | n-Pr | 1 | CCl₃ | |
| 1-200 | OEt | i-Pr | 1 | CCl₃ | |
| 1-201 | OEt | Me | 2 | CCl₃ | |
| 1-202 | OEt | Et | 2 | CCl₃ | |
| 1-203 | OEt | n-Pr | 2 | CCl₃ | |
| 1-204 | OEt | i-Pr | 2 | CCl₃ | |
| 1-205 | O-CH₂₋c-Pr | Me | 0 | CCl₃ | |
| 1-206 | O-CH₂₋c-Pr | Et | 0 | CCl₃ | |
| 1-207 | O-CH₂₋c-Pr | n-Pr | 0 | CCl₃ | |
| 1-208 | O-CH₂₋c-Pr | i-Pr | 0 | CCl₃ | |
| 1-209 | O-CH₂₋c-Pr | Me | 1 | CCl₃ | |
| 1-210 | O-CH₂₋c-Pr | Et | 1 | CCl₃ | |
| 1-211 | O-CH₂₋c-Pr | n-Pr | 1 | CCl₃ | |
| 1-212 | O-CH₂₋c-Pr | i-Pr | 1 | CCl₃ | |
| 1-213 | O-CH₂₋c-Pr | Me | 2 | CCl₃ | |
| 1-214 | O-CH₂₋c-Pr | Et | 2 | CCl₃ | |
| 1-215 | O-CH₂₋c-Pr | n-Pr | 2 | CCl₃ | |
| 1-216 | O-CH₂₋c-Pr | i-Pr | 2 | CCl₃ | |
| 1-217 | OCH₂CH₂OMe | Me | 0 | CCl₃ | |
| 1-218 | OCH₂CH₂OMe | Et | 0 | CCl₃ | |
| 1-219 | OCH₂CH₂OMe | n-Pr | 0 | CCl₃ | |
| 1-220 | OCH₂CH₂OMe | i-Pr | 0 | CCl₃ | |
| 1-221 | OCH₂CH₂OMe | Me | 1 | CCl₃ | |
| 1-222 | OCH₂CH₂OMe | Et | 1 | CCl₃ | |
| 1-223 | OCH₂CH₂OMe | n-Pr | 1 | CCl₃ | |
| 1-224 | OCH₂CH₂OMe | i-Pr | 1 | CCl₃ | |
| 1-225 | OCH₂CH₂OMe | Me | 2 | CCl₃ | |
| 1-226 | OCH₂CH₂OMe | Et | 2 | CCl₃ | |
| 1-227 | OCH₂CH₂OMe | n-Pr | 2 | CCl₃ | |
| 1-228 | OCH₂CH₂OMe | i-Pr | 2 | CCl₃ | |
| 1-229 | OCH₂CH₂SMe | Me | 0 | CCl₃ | |
| 1-230 | OCH₂CH₂SMe | Et | 0 | CCl₃ | |
| 1-231 | OCH₂CH₂SMe | n-Pr | 0 | CCl₃ | |
| 1-232 | OCH₂CH₂SMe | i-Pr | 0 | CCl₃ | |
| 1-233 | OCH₂CH₂SMe | Me | 1 | CCl₃ | |
| 1-234 | OCH₂CH₂SMe | Et | 1 | CCl₃ | |
| 1-235 | OCH₂CH₂SMe | n-Pr | 1 | CCl₃ | |
| 1-236 | OCH₂CH₂SMe | i-Pr | 1 | CCl₃ | |
| 1-237 | OCH₂CH₂SMe | Me | 2 | CCl₃ | |
| 1-238 | OCH₂CH₂SMe | Et | 2 | CCl₃ | |
| 1-239 | OCH₂CH₂SMe | n-Pr | 2 | CCl₃ | |
| 1-240 | OCH₂CH₂SMe | i-Pr | 2 | CCl₃ | |
| 1-241 | OCH₂CH₂SO₂Me | Me | 0 | CCl₃ | |
| 1-242 | OCH₂CH₂SO₂Me | Et | 0 | CCl₃ | |
| 1-243 | OCH₂CH₂SO₂Me | n-Pr | 0 | CCl₃ | |
| 1-244 | OCH₂CH₂SO₂Me | i-Pr | 0 | CCl₃ | |
| 1-245 | OCH₂CH₂SO₂Me | Me | 1 | CCl₃ | |
| 1-246 | OCH₂CH₂SO₂Me | Et | 1 | CCl₃ | |
| 1-247 | OCH₂CH₂SO₂Me | n-Pr | 1 | CCl₃ | |
| 1-248 | OCH₂CH₂SO₂Me | i-Pr | 1 | CCl₃ | |
| 1-249 | OCH₂CH₂SO₂Me | Me | 2 | CCl₃ | |
| 1-250 | OCH₂CH₂SO₂Me | Et | 2 | CCl₃ | |
| 1-251 | OCH₂CH₂SO₂Me | n-Pr | 2 | CCl₃ | |
| 1-252 | OCH₂CH₂SO₂Me | i-Pr | 2 | CCl₃ | |
| 1-253 | OMe | Me | 0 | CHF₂ | |
| 1-254 | OMe | Et | 0 | CHF₂ | |
| 1-255 | OMe | n-Pr | 0 | CHF₂ | |
| 1-256 | OMe | i-Pr | 0 | CHF₂ | |
| 1-257 | OMe | Me | 1 | CHF₂ | |
| 1-258 | OMe | Et | 1 | CHF₂ | |
| 1-259 | OMe | n-Pr | 1 | CHF₂ | |
| 1-260 | OMe | i-Pr | 1 | CHF₂ | |
| 1-261 | OMe | Me | 2 | CHF₂ | |
| 1-262 | OMe | Et | 2 | CHF₂ | |
| 1-263 | OMe | n-Pr | 2 | CHF₂ | |
| 1-264 | OMe | i-Pr | 2 | CHF₂ | |
| 1-265 | OEt | Me | 0 | CHF₂ | |
| 1-266 | OEt | Et | 0 | CHF₂ | |
| 1-267 | OEt | n-Pr | 0 | CHF₂ | |
| 1-268 | OEt | i-Pr | 0 | CHF₂ | |
| 1-269 | OEt | Me | 1 | CHF₂ | |
| 1-270 | OEt | Et | 1 | CHF₂ | |
| 1-271 | OEt | n-Pr | 1 | CHF₂ | |
| 1-272 | OEt | i-Pr | 1 | CHF₂ | |
| 1-273 | OEt | Me | 2 | CHF₂ | |
| 1-274 | OEt | Et | 2 | CHF₂ | |
| 1-275 | OEt | n-Pr | 2 | CHF₂ | |
| 1-276 | OEt | i-Pr | 2 | CHF₂ | |
| 1-277 | O-CH₂-c-Pr | Me | 0 | CHF₂ | |
| 1-278 | O-CH₂-c-Pr | Et | 0 | CHF₂ | |
| 1-279 | O-CH₂-c-Pr | n-Pr | 0 | CHF₂ | |
| 1-280 | O-CH₂-c-Pr | i-Pr | 0 | CHF₂ | |
| 1-281 | O-CH₂-c-Pr | Me | 1 | CHF₂ | |
| 1-282 | O-CH₂-c-Pr | Et | 1 | CHF₂ | |
| 1-283 | O-CH₂-c-Pr | n-Pr | 1 | CHF₂ | |
| 1-284 | O-CH₂-c-Pr | i-Pr | 1 | CHF₂ | |
| 1-285 | O-CH₂-c-Pr | Me | 2 | CHF₂ | |
| 1-286 | O-CH₂-c-Pr | Et | 2 | CHF₂ | |
| 1-287 | O-CH₂-c-Pr | n-Pr | 2 | CHF₂ | |
| 1-288 | O-CH₂-c-Pr | i-Pr | 2 | CHF₂ | |
| 1-289 | OCH₂CH₂OMe | Me | 0 | CHF₂ | |
| 1-290 | OCH₂CH₂OMe | Et | 0 | CHF₂ | |
| 1-291 | OCH₂CH₂OMe | n-Pr | 0 | CHF₂ | |
| 1-292 | OCH₂CH₂OMe | i-Pr | 0 | CHF₂ | |
| 1-293 | OCH₂CH₂OMe | Me | 1 | CHF₂ | |
| 1-294 | OCH₂CH₂OMe | Et | 1 | CHF₂ | |
| 1-295 | OCH₂CH₂OMe | n-Pr | 1 | CHF₂ | |
| 1-296 | OCH₂CH₂OMe | i-Pr | 1 | CHF₂ | |
| 1-297 | OCH₂CH₂OMe | Me | 2 | CHF₂ | |
| 1-298 | OCH₂CH₂OMe | Et | 2 | CHF₂ | |
| 1-299 | OCH₂CH₂OMe | n-Pr | 2 | CHF₂ | |
| 1-300 | OCH₂CH₂OMe | i-Pr | 2 | CHF₂ | |
| 1-301 | OCH₂CH₂SMe | Me | 0 | CHF₂ | |
| 1-302 | OCH₂CH₂SMe | Et | 0 | CHF₂ | |
| 1-303 | OCH₂CH₂SMe | n-Pr | 0 | CHF₂ | |
| 1-304 | OCH₂CH₂SMe | i-Pr | 0 | CHF₂ | |
| 1-305 | OCH₂CH₂SMe | Me | 1 | CHF₂ | |
| 1-306 | OCH₂CH₂SMe | Et | 1 | CHF₂ | |
| 1-307 | OCH₂CH₂SMe | n-Pr | 1 | CHF₂ | |
| 1-308 | OCH₂CH₂SMe | i-Pr | 1 | CHF₂ | |
| 1-309 | OCH₂CH₂SMe | Me | 2 | CHF₂ | |
| 1-310 | OCH₂CH₂SMe | Et | 2 | CHF₂ | |
| 1-311 | OCH₂CH₂SMe | n-Pr | 2 | CHF₂ | |
| 1-312 | OCH₂CH₂SMe | i-Pr | 2 | CHF₂ | |
| 1-313 | OCH₂CH₂SO₂Me | Me | 0 | CHF₂ | |
| 1-314 | OCH₂CH₂SO₂Me | Et | 0 | CHF₂ | |
| 1-315 | OCH₂CH₂SO₂Me | n-Pr | 0 | CHF₂ | |
| 1-316 | OCH₂CH₂SO₂Me | i-Pr | 0 | CHF₂ | |
| 1-317 | OCH₂CH₂SO₂Me | Me | 1 | CHF₂ | |
| 1-318 | OCH₂CH₂SO₂Me | Et | 1 | CHF₂ | |
| 1-319 | OCH₂CH₂SO₂Me | n-Pr | 1 | CHF₂ | |
| 1-320 | OCH₂CH₂SO₂Me | i-Pr | 1 | CHF₂ | |
| 1-321 | OCH₂CH₂SO₂Me | Me | 2 | CHF₂ | |
| 1-322 | OCH₂CH₂SO₂Me | Et | 2 | CHF₂ | |
| 1-323 | OCH₂CH₂SO₂Me | n-Pr | 2 | CHF₂ | |
| 1-324 | OCH₂CH₂SO₂Me | i-Pr | 2 | CHF₂ | |
| 1-325 | OMe | Me | 0 | CF(CF₃)₂ | |
| 1-326 | OMe | Et | 0 | CF(CF₃)₂ | |
| 1-327 | OMe | n-Pr | 0 | CF(CF₃)₂ | |
| 1-328 | OMe | i-Pr | 0 | CF(CF₃)₂ | |
| 1-329 | OMe | Me | 1 | CF(CF₃)₂ | |
| 1-330 | OMe | Et | 1 | CF(CF₃)₂ | |
| 1-331 | OMe | n-Pr | 1 | CF(CF₃)₂ | |
| 1-332 | OMe | i-Pr | 1 | CF(CF₃)₂ | |
| 1-333 | OMe | Me | 2 | CF(CF₃)₂ | |
| 1-334 | OMe | Et | 2 | CF(CF₃)₂ | |
| 1-335 | OMe | n-Pr | 2 | CF(CF₃)₂ | |
| 1-336 | OMe | i-Pr | 2 | CF(CF₃)₂ | |
| 1-337 | OEt | Me | 0 | CF(CF₃)₂ | |
| 1-338 | OEt | Et | 0 | CF(CF₃)₂ | |
| 1-339 | OEt | n-Pr | 0 | CF(CF₃)₂ | |
| 1-340 | OEt | i-Pr | 0 | CF(CF₃)₂ | |
| 1-341 | OEt | Me | 1 | CF(CF₃)₂ | |
| 1-342 | OEt | Et | 1 | CF(CF₃)₂ | |
| 1-343 | OEt | n-Pr | 1 | CF(CF₃)₂ | |
| 1-344 | OEt | i-Pr | 1 | CF(CF₃)₂ | |
| 1-345 | OEt | Me | 2 | CF(CF₃)₂ | |
| 1-346 | OEt | Et | 2 | CF(CF₃)₂ | |
| 1-347 | OEt | n-Pr | 2 | CF(CF₃)₂ | |
| 1-348 | OEt | i-Pr | 2 | CF(CF₃)₂ | |
| 1-349 | O-CH₂-c-Pr | Me | 0 | CF(CF₃)₂ | |
| 1-350 | O-CH₂-c-Pr | Et | 0 | CF(CF₃)₂ | |
| 1-351 | O-CH₂-c-Pr | n-Pr | 0 | CF(CF₃)₂ | |
| 1-352 | O-CH₂-c-Pr | i-Pr | 0 | CF(CF₃)₂ | |
| 1-353 | O-CH₂-c-Pr | Me | 1 | CF(CF₃)₂ | |
| 1-354 | O-CH₂-c-Pr | Et | 1 | CF(CF₃)₂ | |
| 1-355 | O-CH₂-c-Pr | n-Pr | 1 | CF(CF₃)₂ | |
| 1-356 | O-CH₂-c-Pr | i-Pr | 1 | CF(CF₃)₂ | |
| 1-357 | O-CH₂-c-Pr | Me | 2 | CF(CF₃)₂ | |
| 1-358 | O-CH₂-c-Pr | Et | 2 | CF(CF₃)₂ | |
| 1-359 | O-CH₂-c-Pr | n-Pr | 2 | CF(CF₃)₂ | |
| 1-360 | O-CH₂-c-Pr | i-Pr | 2 | CF(CF₃)₂ | |
| 1-361 | OCH₂CH₂OMe | Me | 0 | CF(CF₃)₂ | |
| 1-362 | OCH₂CH₂OMe | Et | 0 | CF(CF₃)₂ | |
| 1-363 | OCH₂CH₂OMe | n-Pr | 0 | CF(CF₃)₂ | |
| 1-364 | OCH₂CH₂OMe | i-Pr | 0 | CF(CF₃)₂ | |
| 1-365 | OCH₂CH₂OMe | Me | 1 | CF(CF₃)₂ | |
| 1-366 | OCH₂CH₂OMe | Et | 1 | CF(CF₃)₂ | |
| 1-367 | OCH₂CH₂OMe | n-Pr | 1 | CF(CF₃)₂ | |
| 1-368 | OCH₂CH₂OMe | i-Pr | 1 | CF(CF₃)₂ | |
| 1-369 | OCH₂CH₂OMe | Me | 2 | CF(CF₃)₂ | |
| 1-370 | OCH₂CH₂OMe | Et | 2 | CF(CF₃)₂ | |
| 1-371 | OCH₂CH₂OMe | n-Pr | 2 | CF(CF₃)₂ | |
| 1-372 | OCH₂CH₂OMe | i-Pr | 2 | CF(CF₃)₂ | |
| 1-373 | OCH₂CH₂SMe | Me | 0 | CF(CF₃)₂ | |
| 1-374 | OCH₂CH₂SMe | Et | 0 | CF(CF₃)₂ | |
| 1-375 | OCH₂CH₂SMe | n-Pr | 0 | CF(CF₃)₂ | |
| 1-376 | OCH₂CH₂SMe | i-Pr | 0 | CF(CF₃)₂ | |
| 1-377 | OCH₂CH₂SMe | Me | 1 | CF(CF₃)₂ | |
| 1-378 | OCH₂CH₂SMe | Et | 1 | CF(CF₃)₂ | |
| 1-379 | OCH₂CH₂SMe | n-Pr | 1 | CF(CF₃)₂ | |
| 1-380 | OCH₂CH₂SMe | i-Pr | 1 | CF(CF₃)₂ | |
| 1-381 | OCH₂CH₂SMe | Me | 2 | CF(CF₃)₂ | |
| 1-382 | OCH₂CH₂SMe | Et | 2 | CF(CF₃)₂ | |
| 1-383 | OCH₂CH₂SMe | n-Pr | 2 | CF(CF₃)₂ | |
| 1-384 | OCH₂CH₂SMe | i-Pr | 2 | CF(CF₃)₂ | |
| 1-385 | OCH₂CH₂SO₂Me | Me | 0 | CF(CF₃)₂ | |
| 1-386 | OCH₂CH₂SO₂Me | Et | 0 | CF(CF₃)₂ | |
| 1-387 | OCH₂CH₂SO₂Me | n-Pr | 0 | CF(CF₃)₂ | |
| 1-388 | OCH₂CH₂SO₂Me | i-Pr | 0 | CF(CF₃)₂ | |
| 1-389 | OCH₂CH₂SO₂Me | Me | 1 | CF(CF₃)₂ | |
| 1-390 | OCH₂CH₂SO₂Me | Et | 1 | CF(CF₃)₂ | |
| 1-391 | OCH₂CH₂SO₂Me | n-Pr | 1 | CF(CF₃)₂ | |
| 1-392 | OCH₂CH₂SO₂Me | i-Pr | 1 | CF(CF₃)₂ | |
| 1-393 | OCH₂CH₂SO₂Me | Me | 2 | CF(CF₃)₂ | |
| 1-394 | OCH₂CH₂SO₂Me | Et | 2 | CF(CF₃)₂ | |
| 1-395 | OCH₂CH₂SO₂Me | n-Pr | 2 | CF(CF₃)₂ | |
| 1-396 | OCH₂CH₂SO₂Me | i-Pr | 2 | CF(CF₃)₂ | |

**Tabelle 2: Erfindungsgemäße Verbindungen der allgemeinen Formel (I), worin R¹ für Ethyl und R² sowie R⁴ für jeweils Wasserstoff stehen.**

| | | | | | |
|---|---|---|---|---|---|
| 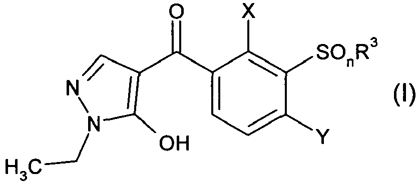 | | | | | |

| Nr. | X | R³ | n | Y | Physikalische Daten: ¹H-NMR: δ [CDCl₃] |
|---|---|---|---|---|---|
| 2-1 | OH | Me | 0 | CF₃ | |
| 2-2 | OH | Et | 0 | CF₃ | |
| 2-3 | OH | n-Pr | 0 | CF₃ | |
| 2-4 | OH | i-Pr | 0 | CF₃ | |
| 2-5 | OH | Me | 1 | CF₃ | |
| 2-6 | OH | Et | 1 | CF₃ | |
| 2-7 | OH | n-Pr | 1 | CF₃ | |
| 2-8 | OH | i-Pr | 1 | CF₃ | |
| 2-9 | OH | Me | 2 | CF₃ | |
| 2-10 | OH | Et | 2 | CF₃ | |
| 2-11 | OH | n-Pr | 2 | CF₃ | |
| 2-12 | OH | i-Pr | 2 | CF₃ | |
| 2-13 | OMe | Me | 0 | CF₃ | 7.57 (d, 1H), 7.51 (d, 1H), 7.44 (s, 1H), 4.07 (q, 2H), 3.89 (s, 3H), 2.49 (s, 3H), 1.45 (t, 3H) |
| 2-14 | OMe | Et | 0 | CF₃ | |
| 2-15 | OMe | n-Pr | 0 | CF₃ | |
| 2-16 | OMe | i-Pr | 0 | CF₃ | |
| 2-17 | OMe | Me | 1 | CF₃ | 7.72 (d, 1H), 7.60 (d, 1H), 7.49 (s, 1H), 4.08 (q, 2H), 3.92 (s, 3H), 3.17 (s, 3H), 1.47 (t, 3H) |
| 2-18 | OMe | Et | 1 | CF₃ | |
| 2-19 | OMe | n-Pr | 1 | CF₃ | |
| 2-20 | OMe | i-Pr | 1 | CF₃ | |
| 2-21 | OMe | Me | 2 | CF₃ | 7.83 (d, 1H), 7.77 (d, 1H), 7.47 (s, 1H), 4.10 (q, 2H), 3.91 (s, 3H), 3.41 (s, 3H), 1.47 (t, 3H) |
| 2-22 | OMe | Et | 2 | CF₃ | |
| 2-23 | OMe | n-Pr | 2 | CF₃ | |
| 2-24 | OMe | i-Pr | 2 | CF₃ | |
| 2-25 | OEt | Me | 0 | CF₃ | 7.55 (d, 1H), 7.52 (d, 1H), 7.47 (s, 1H), 4.07 (m, 4H), 2.50 (s, 3H), 1.44 (t, 3H), 1.30 (t, 3H) |
| 2-26 | OEt | Et | 0 | CF₃ | |
| 2-27 | OEt | n-Pr | 0 | CF₃ | |
| 2-28 | OEt | i-Pr | 0 | CF₃ | |
| 2-29 | OEt | Me | 1 | CF₃ | 7.70 (d, 1H), 7.58 (d, 1H), 7.50 (s, 1H), 4.27 (m, 1H), 4.08 (q, 2H), 3.96 (m, 1H), 3.17 (s, 3H), 1.46 (t, 3H), 1.31 (t, 3H) |
| 2-30 | OEt | Et | 1 | CF₃ | |
| 2-31 | OEt | n-Pr | 1 | CF₃ | |
| 2-32 | OEt | i-Pr | 1 | CF₃ | |
| 2-33 | OEt | Me | 2 | CF₃ | 7.81 (d, 1H), 7.75 (d, 1H), 7.47 (s, 1H), 4.10 (m, 4H), 3.42 (s, 3H), 1.46 (t, 3H), 1.31 (t, 3H) |
| 2-34 | OEt | Et | 2 | CF₃ | |
| 2-35 | OEt | n-Pr | 2 | CF₃ | |
| 2-36 | OEt | i-Pr | 2 | CF₃ | |
| 2-37 | O-CH₂₋c-Pr | Me | 0 | CF₃ | |
| 2-38 | O-CH₂₋c-Pr | Et | 0 | CF₃ | |
| 2-39 | O-CH₂₋c-Pr | n-Pr | 0 | CF₃ | |
| 2-40 | O-CH₂₋c-Pr | i-Pr | 0 | CF₃ | |
| 2-41 | O-CH₂₋c-Pr | Me | 1 | CF₃ | |
| 2-42 | O-CH₂₋c-Pr | Et | 1 | CF₃ | |
| 2-43 | O-CH₂₋c-Pr | n-Pr | 1 | CF₃ | |
| 2-44 | O-CH₂₋c-Pr | i-Pr | 1 | CF₃ | |
| 2-45 | O-CH₂₋c-Pr | Me | 2 | CF₃ | |
| 2-46 | O-CH₂₋c-Pr | Et | 2 | CF₃ | |
| 2-47 | O-CH₂₋c-Pr | n-Pr | 2 | CF₃ | |
| 2-48 | O-CH₂₋c-Pr | i-Pr | 2 | CF₃ | |
| 2-49 | OCH₂CH₂OMe | Me | 0 | CF₃ | |
| 2-50 | OCH₂CH₂OMe | Et | 0 | CF₃ | |
| 2-51 | OCH₂CH₂OMe | n-Pr | 0 | CF₃ | |
| 2-52 | OCH₂CH₂OMe | i-Pr | 0 | CF₃ | |
| 2-53 | OCH₂CH₂OMe | Me | 1 | CF₃ | |
| 2-54 | OCH₂CH₂OMe | Et | 1 | CF₃ | |
| 2-55 | OCH₂CH₂OMe | n-Pr | 1 | CF₃ | |
| 2-56 | OCH₂CH₂OMe | i-Pr | 1 | CF₃ | |
| 2-57 | OCH₂CH₂OMe | Me | 2 | CF₃ | |
| 2-58 | OCH₂CH₂OMe | Et | 2 | CF₃ | |
| 2-59 | OCH₂CH₂OMe | n-Pr | 2 | CF₃ | |
| 2-60 | OCH₂CH₂OMe | i-Pr | 2 | CF₃ | |
| 2-61 | OCH₂CH₂SMe | Me | 0 | CF₃ | |
| 2-62 | OCH₂CH₂SMe | Et | 0 | CF₃ | |
| 2-63 | OCH₂CH₂SMe | n-Pr | 0 | CF₃ | |
| 2-64 | OCH₂CH₂SMe | i-Pr | 0 | CF₃ | |
| 2-65 | OCH₂CH₂SMe | Me | 1 | CF₃ | |
| 2-66 | OCH₂CH₂SMe | Et | 1 | CF₃ | |
| 2-67 | OCH₂CH₂SMe | n-Pr | 1 | CF₃ | |
| 2-68 | OCH₂CH₂SMe | i-Pr | 1 | CF₃ | |
| 2-69 | OCH₂CH₂SMe | Me | 2 | CF₃ | |
| 2-70 | OCH₂CH₂SMe | Et | 2 | CF₃ | |
| 2-71 | OCH₂CH₂SMe | n-Pr | 2 | CF₃ | |
| 2-72 | OCH₂CH₂SMe | i-Pr | 2 | CF₃ | |
| 2-73 | OCH₂CH₂SO₂Me | Me | 0 | CF₃ | |
| 2-74 | OCH₂CH₂SO₂Me | Et | 0 | CF₃ | |
| 2-75 | OCH₂CH₂SO₂Me | n-Pr | 0 | CF₃ | |
| 2-76 | OCH₂CH₂SO₂Me | i-Pr | 0 | CF₃ | |
| 2-77 | OCH₂CH₂SO₂Me | Me | 1 | CF₃ | |
| 2-78 | OCH₂CH₂SO₂Me | Et | 1 | CF₃ | |
| 2-79 | OCH₂CH₂SO₂Me | n-Pr | 1 | CF₃ | |
| 2-80 | OCH₂CH₂SO₂Me | i-Pr | 1 | CF₃ | |
| 2-81 | OCH₂CH₂SO₂Me | Me | 2 | CF₃ | |
| 2-82 | OCH₂CH₂SO₂Me | Et | 2 | CF₃ | |
| 2-83 | OCH₂CH₂SO₂Me | n-Pr | 2 | CF₃ | |
| 2-84 | OCH₂CH₂SO₂Me | i-Pr | 2 | CF₃ | |
| 2-85 | OCOMe | Me | 0 | CF₃ | |
| 2-86 | OCOMe | Et | 0 | CF₃ | |
| 2-87 | OCOMe | n-Pr | 0 | CF₃ | |
| 2-88 | OCOMe | i-Pr | 0 | CF₃ | |
| 2-89 | OCOMe | Me | 1 | CF₃ | |
| 2-90 | OCOMe | Et | 1 | CF₃ | |
| 2-91 | OCOMe | n-Pr | 1 | CF₃ | |
| 2-92 | OCOMe | i-Pr | 1 | CF₃ | |
| 2-93 | OCOMe | Me | 2 | CF₃ | |
| 2-94 | OCOMe | Et | 2 | CF₃ | |
| 2-95 | OCOMe | n-Pr | 2 | CF₃ | |
| 2-96 | OCOMe | i-Pr | 2 | CF₃ | |
| 2-97 | OSO₂Me | Me | 0 | CF₃ | |
| 2-98 | OSO₂Me | Et | 0 | CF₃ | |
| 2-99 | OSO₂Me | n-Pr | 0 | CF₃ | |
| 2-100 | OSO₂Me | i-Pr | 0 | CF₃ | |
| 2-101 | OSO₂Me | Me | 1 | CF₃ | |
| 2-102 | OSO₂Me | Et | 1 | CF₃ | |
| 2-103 | OSO₂Me | n-Pr | 1 | CF₃ | |
| 2-104 | OSO₂Me | i-Pr | 1 | CF₃ | |
| 2-105 | OSO₂Me | Me | 2 | CF₃ | |
| 2-106 | OSO₂Me | Et | 2 | CF₃ | |
| 2-107 | OSO₂Me | n-Pr | 2 | CF₃ | |
| 2-108 | OSO₂Me | i-Pr | 2 | CF₃ | |
| 2-109 | OMe | Me | 0 | C₂F₅ | |
| 2-110 | OMe | Et | 0 | C₂F₅ | |
| 2-111 | OMe | n-Pr | 0 | C₂F₅ | |
| 2-112 | OMe | i-Pr | 0 | C₂F₅ | |
| 2-113 | OMe | Me | 1 | C₂F₅ | |
| 2-114 | OMe | Et | 1 | C₂F₅ | |
| 2-115 | OMe | n-Pr | 1 | C₂F₅ | |
| 2-116 | OMe | i-Pr | 1 | C₂F₅ | |
| 2-117 | OMe | Me | 2 | C₂F₅ | |
| 2-118 | OMe | Et | 2 | C₂F₅ | |
| 2-119 | OMe | n-Pr | 2 | C₂F₅ | |
| 2-120 | OMe | i-Pr | 2 | C₂F₅ | |
| 2-121 | OEt | Me | 0 | C₂F₅ | |
| 2-122 | OEt | Et | 0 | C₂F₅ | |
| 2-123 | OEt | n-Pr | 0 | C₂F₅ | |
| 2-124 | OEt | i-Pr | 0 | C₂F₅ | |
| 2-125 | OEt | Me | 1 | C₂F₅ | |
| 2-126 | OEt | Et | 1 | C₂F₅ | |
| 2-127 | OEt | n-Pr | 1 | C₂F₅ | |
| 2-128 | OEt | i-Pr | 1 | C₂F₅ | |
| 2-129 | OEt | Me | 2 | C₂F₅ | |
| 2-130 | OEt | Et | 2 | C₂F₅ | |
| 2-131 | OEt | n-Pr | 2 | C₂F₅ | |
| 2-132 | OEt | i-Pr | 2 | C₂F₅ | |
| 2-133 | O-CH₂₋c-Pr | Me | 0 | C₂F₅ | |
| 2-134 | O-CH₂₋c-Pr | Et | 0 | C₂F₅ | |
| 2-135 | O-CH₂₋c-Pr | n-Pr | 0 | C₂F₅ | |
| 2-136 | O-CH₂₋c-Pr | i-Pr | 0 | C₂F₅ | |
| 2-137 | O-CH₂₋c-Pr | Me | 1 | C₂F₅ | |
| 2-138 | O-CH₂₋c-Pr | Et | 1 | C₂F₅ | |
| 2-139 | O-CH₂₋c-Pr | n-Pr | 1 | C₂F₅ | |
| 2-140 | O-CH₂₋c-Pr | i-Pr | 1 | C₂F₅ | |
| 2-141 | O-CH₂₋c-Pr | Me | 2 | C₂F₅ | |
| 2-142 | O-CH₂₋c-Pr | Et | 2 | C₂F₅ | |
| 2-143 | O-CH₂₋c-Pr | n-Pr | 2 | C₂F₅ | |
| 2-144 | O-CH₂₋c-Pr | i-Pr | 2 | C₂F₅ | |
| 2-145 | OCH₂CH₂OMe | Me | 0 | C₂F₅ | |
| 2-146 | OCH₂CH₂OMe | Et | 0 | C₂F₅ | |
| 2-147 | OCH₂CH₂OMe | n-Pr | 0 | C₂F₅ | |
| 2-148 | OCH₂CH₂OMe | i-Pr | 0 | C₂F₅ | |
| 2-149 | OCH₂CH₂OMe | Me | 1 | C₂F₅ | |
| 2-150 | OCH₂CH₂OMe | Et | 1 | C₂F₅ | |
| 2-151 | OCH₂CH₂OMe | n-Pr | 1 | C₂F₅ | |
| 2-152 | OCH₂CH₂OMe | i-Pr | 1 | C₂F₅ | |
| 2-153 | OCH₂CH₂OMe | Me | 2 | C₂F₅ | |
| 2-154 | OCH₂CH₂OMe | Et | 2 | C₂F₅ | |
| 2-155 | OCH₂CH₂OMe | n-Pr | 2 | C₂F₅ | |
| 2-156 | OCH₂CH₂OMe | i-Pr | 2 | C₂F₅ | |
| 2-157 | OCH₂CH₂SMe | Me | 0 | C₂F₅ | |
| 2-158 | OCH₂CH₂SMe | Et | 0 | C₂F₅ | |
| 2-159 | OCH₂CH₂SMe | n-Pr | 0 | C₂F₅ | |
| 2-160 | OCH₂CH₂SMe | i-Pr | 0 | C₂F₅ | |
| 2-161 | OCH₂CH₂SMe | Me | 1 | C₂F₅ | |
| 2-162 | OCH₂CH₂SMe | Et | 1 | C₂F₅ | |
| 2-163 | OCH₂CH₂SMe | n-Pr | 1 | C₂F₅ | |
| 2-164 | OCH₂CH₂SMe | i-Pr | 1 | C₂F₅ | |
| 2-165 | OCH₂CH₂SMe | Me | 2 | C₂F₅ | |
| 2-166 | OCH₂CH₂SMe | Et | 2 | C₂F₅ | |
| 2-167 | OCH₂CH₂SMe | n-Pr | 2 | C₂F₅ | |
| 2-168 | OCH₂CH₂SMe | i-Pr | 2 | C₂F₅ | |
| 2-169 | OCH₂CH₂SO₂Me | Me | 0 | C₂F₅ | |
| 2-170 | OCH₂CH₂SO₂Me | Et | 0 | C₂F₅ | |
| 2-171 | OCH₂CH₂SO₂Me | n-Pr | 0 | C₂F₅ | |
| 2-172 | OCH₂CH₂SO₂Me | i-Pr | 0 | C₂F₅ | |
| 2-173 | OCH₂CH₂SO₂Me | Me | 1 | C₂F₅ | |
| 2-174 | OCH₂CH₂SO₂Me | Et | 1 | C₂F₅ | |
| 2-175 | OCH₂CH₂SO₂Me | n-Pr | 1 | C₂F₅ | |
| 2-176 | OCH₂CH₂SO₂Me | i-Pr | 1 | C₂F₅ | |
| 2-177 | OCH₂CH₂SO₂Me | Me | 2 | C₂F₅ | |
| 2-178 | OCH₂CH₂SO₂Me | Et | 2 | C₂F₅ | |
| 2-179 | OCH₂CH₂SO₂Me | n-Pr | 2 | C₂F₅ | |
| 2-180 | OCH₂CH₂SO₂Me | i-Pr | 2 | C₂F₅ | |
| 2-181 | OMe | Me | 0 | CCl₃ | |
| 2-182 | OMe | Et | 0 | CCl₃ | |
| 2-183 | OMe | n-Pr | 0 | CCl₃ | |
| 2-184 | OMe | i-Pr | 0 | CCl₃ | |
| 2-185 | OMe | Me | 1 | CCl₃ | |
| 2-186 | OMe | Et | 1 | CCl₃ | |
| 2-187 | OMe | n-Pr | 1 | CCl₃ | |
| 2-188 | OMe | i-Pr | 1 | CCl₃ | |
| 2-189 | OMe | Me | 2 | CCl₃ | |
| 2-190 | OMe | Et | 2 | CCl₃ | |
| 2-191 | OMe | n-Pr | 2 | CCl₃ | |
| 2-192 | OMe | i-Pr | 2 | CCl₃ | |
| 2-193 | OEt | Me | 0 | CCl₃ | |
| 2-194 | OEt | Et | 0 | CCl₃ | |
| 2-195 | OEt | n-Pr | 0 | CCl₃ | |
| 2-196 | OEt | i-Pr | 0 | CCl₃ | |
| 2-197 | OEt | Me | 1 | CCl₃ | |
| 2-198 | OEt | Et | 1 | CCl₃ | |
| 2-199 | OEt | n-Pr | 1 | CCl₃ | |
| 2-200 | OEt | i-Pr | 1 | CCl₃ | |
| 2-201 | OEt | Me | 2 | CCl₃ | |
| 2-202 | OEt | Et | 2 | CCl₃ | |
| 2-203 | OEt | n-Pr | 2 | CCl₃ | |
| 2-204 | OEt | i-Pr | 2 | CCl₃ | |
| 2-205 | O-CH₂₋c-Pr | Me | 0 | CCl₃ | |
| 2-206 | O-CH₂₋c-Pr | Et | 0 | CCl₃ | |
| 2-207 | O-CH₂₋c-Pr | n-Pr | 0 | CCl₃ | |
| 2-208 | O-CH₂₋c-Pr | i-Pr | 0 | CCl₃ | |
| 2-209 | O-CH₂₋c-Pr | Me | 1 | CCl₃ | |
| 2-210 | O-CH₂₋c-Pr | Et | 1 | CCl₃ | |
| 2-211 | O-CH₂₋c-Pr | n-Pr | 1 | CCl₃ | |
| 2-212 | O-CH₂₋c-Pr | i-Pr | 1 | CCl₃ | |
| 2-213 | O-CH₂₋c-Pr | Me | 2 | CCl₃ | |
| 2-214 | O-CH₂₋c-Pr | Et | 2 | CCl₃ | |
| 2-215 | O-CH₂₋c-Pr | n-Pr | 2 | CCl₃ | |
| 2-216 | O-CH₂₋c-Pr | i-Pr | 2 | CCl₃ | |
| 2-217 | OCH₂CH₂OMe | Me | 0 | CCl₃ | |
| 2-218 | OCH₂CH₂OMe | Et | 0 | CCl₃ | |
| 2-219 | OCH₂CH₂OMe | n-Pr | 0 | CCl₃ | |
| 2-220 | OCH₂CH₂OMe | i-Pr | 0 | CCl₃ | |
| 2-221 | OCH₂CH₂OMe | Me | 1 | CCl₃ | |
| 2-222 | OCH₂CH₂OMe | Et | 1 | CCl₃ | |
| 2-223 | OCH₂CH₂OMe | n-Pr | 1 | CCl₃ | |
| 2-224 | OCH₂CH₂OMe | i-Pr | 1 | CCl₃ | |
| 2-225 | OCH₂CH₂OMe | Me | 2 | CCl₃ | |
| 2-226 | OCH₂CH₂OMe | Et | 2 | CCl₃ | |
| 2-227 | OCH₂CH₂OMe | n-Pr | 2 | CCl₃ | |
| 2-228 | OCH₂CH₂OMe | i-Pr | 2 | CCl₃ | |
| 2-229 | OCH₂CH₂SMe | Me | 0 | CCl₃ | |
| 2-230 | OCH₂CH₂SMe | Et | 0 | CCl₃ | |
| 2-231 | OCH₂CH₂SMe | n-Pr | 0 | CCl₃ | |
| 2-232 | OCH₂CH₂SMe | i-Pr | 0 | CCl₃ | |
| 2-233 | OCH₂CH₂SMe | Me | 1 | CCl₃ | |
| 2-234 | OCH₂CH₂SMe | Et | 1 | CCl₃ | |
| 2-235 | OCH₂CH₂SMe | n-Pr | 1 | CCl₃ | |
| 2-236 | OCH₂CH₂SMe | i-Pr | 1 | CCl₃ | |
| 2-237 | OCH₂CH₂SMe | Me | 2 | CCl₃ | |
| 2-238 | OCH₂CH₂SMe | Et | 2 | CCl₃ | |
| 2-239 | OCH₂CH₂SMe | n-Pr | 2 | CCl₃ | |
| 2-240 | OCH₂CH₂SMe | i-Pr | 2 | CCl₃ | |
| 2-241 | OCH₂CH₂SO₂Me | Me | 0 | CCl₃ | |
| 2-242 | OCH₂CH₂SO₂Me | Et | 0 | CCl₃ | |
| 2-243 | OCH₂CH₂SO₂Me | n-Pr | 0 | CCl₃ | |
| 2-244 | OCH₂CH₂SO₂Me | i-Pr | 0 | CCl₃ | |
| 2-245 | OCH₂CH₂SO₂Me | Me | 1 | CCl₃ | |
| 2-246 | OCH₂CH₂SO₂Me | Et | 1 | CCl₃ | |
| 2-247 | OCH₂CH₂SO₂Me | n-Pr | 1 | CCl₃ | |
| 2-248 | OCH₂CH₂SO₂Me | i-Pr | 1 | CCl₃ | |
| 2-249 | OCH₂CH₂SO₂Me | Me | 2 | CCl₃ | |
| 2-250 | OCH₂CH₂SO₂Me | Et | 2 | CCl₃ | |
| 2-251 | OCH₂CH₂SO₂Me | n-Pr | 2 | CCl₃ | |
| 2-252 | OCH₂CH₂SO₂Me | i-Pr | 2 | CCl₃ | |
| 2-253 | OMe | Me | 0 | CHF₂ | |
| 2-254 | OMe | Et | 0 | CHF₂ | |
| 2-255 | OMe | n-Pr | 0 | CHF₂ | |
| 2-256 | OMe | i-Pr | 0 | CHF₂ | |
| 2-257 | OMe | Me | 1 | CHF₂ | |
| 2-258 | OMe | Et | 1 | CHF₂ | |
| 2-259 | OMe | n-Pr | 1 | CHF₂ | |
| 2-260 | OMe | i-Pr | 1 | CHF₂ | |
| 2-261 | OMe | Me | 2 | CHF₂ | |
| 2-262 | OMe | Et | 2 | CHF₂ | |
| 2-263 | OMe | n-Pr | 2 | CHF₂ | |
| 2-264 | OMe | i-Pr | 2 | CHF₂ | |
| 2-265 | OEt | Me | 0 | CHF₂ | |
| 2-266 | OEt | Et | 0 | CHF₂ | |
| 2-267 | OEt | n-Pr | 0 | CHF₂ | |
| 2-268 | OEt | i-Pr | 0 | CHF₂ | |
| 2-269 | OEt | Me | 1 | CHF₂ | |
| 2-270 | OEt | Et | 1 | CHF₂ | |
| 2-271 | OEt | n-Pr | 1 | CHF₂ | |
| 2-272 | OEt | i-Pr | 1 | CHF₂ | |
| 2-273 | OEt | Me | 2 | CHF₂ | |
| 2-274 | OEt | Et | 2 | CHF₂ | |
| 2-275 | OEt | n-Pr | 2 | CHF₂ | |
| 2-276 | OEt | i-Pr | 2 | CHF₂ | |
| 2-277 | O - CH₂ - c-Pr | Me | 0 | CHF₂ | |
| 2-278 | O - CH₂ - c-Pr | Et | 0 | CHF₂ | |
| 2-279 | O - CH₂ - c-Pr | n-Pr | 0 | CHF₂ | |
| 2-280 | O - CH₂ - c-Pr | i-Pr | 0 | CHF₂ | |
| 2-281 | O - CH₂ - c-Pr | Me | 1 | CHF₂ | |
| 2-282 | O - CH₂ - c-Pr | Et | 1 | CHF₂ | |
| 2-283 | O - CH₂ - c-Pr | n-Pr | 1 | CHF₂ | |
| 2-284 | O - CH₂ - c-Pr | i-Pr | 1 | CHF₂ | |
| 2-285 | O - CH₂ - c-Pr | Me | 2 | CHF₂ | |
| 2-286 | O - CH₂ - c-Pr | Et | 2 | CHF₂ | |
| 2-287 | O - CH₂ - c-Pr | n-Pr | 2 | CHF₂ | |
| 2-288 | O - CH₂ - c-Pr | i-Pr | 2 | CHF₂ | |
| 2-289 | OCH₂CH₂OMe | Me | 0 | CHF₂ | |
| 2-290 | OCH₂CH₂OMe | Et | 0 | CHF₂ | |
| 2-291 | OCH₂CH₂OMe | n-Pr | 0 | CHF₂ | |
| 2-292 | OCH₂CH₂OMe | i-Pr | 0 | CHF₂ | |
| 2-293 | OCH₂CH₂OMe | Me | 1 | CHF₂ | |
| 2-294 | OCH₂CH₂OMe | Et | 1 | CHF₂ | |
| 2-295 | OCH₂CH₂OMe | n-Pr | 1 | CHF₂ | |
| 2-296 | OCH₂CH₂OMe | i-Pr | 1 | CHF₂ | |
| 2-297 | OCH₂CH₂OMe | Me | 2 | CHF₂ | |
| 2-298 | OCH₂CH₂OMe | Et | 2 | CHF₂ | |
| 2-299 | OCH₂CH₂OMe | n-Pr | 2 | CHF₂ | |
| 2-300 | OCH₂CH₂OMe | i-Pr | 2 | CHF₂ | |
| 2-301 | OCH₂CH₂SMe | Me | 0 | CHF₂ | |
| 2-302 | OCH₂CH₂SMe | Et | 0 | CHF₂ | |
| 2-303 | OCH₂CH₂SMe | n-Pr | 0 | CHF₂ | |
| 2-304 | OCH₂CH₂SMe | i-Pr | 0 | CHF₂ | |
| 2-305 | OCH₂CH₂SMe | Me | 1 | CHF₂ | |
| 2-306 | OCH₂CH₂SMe | Et | 1 | CHF₂ | |
| 2-307 | OCH₂CH₂SMe | n-Pr | 1 | CHF₂ | |
| 2-308 | OCH₂CH₂SMe | i-Pr | 1 | CHF₂ | |
| 2-309 | OCH₂CH₂SMe | Me | 2 | CHF₂ | |
| 2-310 | OCH₂CH₂SMe | Et | 2 | CHF₂ | |
| 2-311 | OCH₂CH₂SMe | n-Pr | 2 | CHF₂ | |
| 2-312 | OCH₂CH₂SMe | i-Pr | 2 | CHF₂ | |
| 2-313 | OCH₂CH₂SO₂Me | Me | 0 | CHF₂ | |
| 2-314 | OCH₂CH₂SO₂Me | Et | 0 | CHF₂ | |
| 2-315 | OCH₂CH₂SO₂Me | n-Pr | 0 | CHF₂ | |
| 2-316 | OCH₂CH₂SO₂Me | i-Pr | 0 | CHF₂ | |
| 2-317 | OCH₂CH₂SO₂Me | Me | 1 | CHF₂ | |
| 2-318 | OCH₂CH₂SO₂Me | Et | 1 | CHF₂ | |
| 2-319 | OCH₂CH₂SO₂Me | n-Pr | 1 | CHF₂ | |
| 2-320 | OCH₂CH₂SO₂Me | i-Pr | 1 | CHF₂ | |
| 2-321 | OCH₂CH₂SO₂Me | Me | 2 | CHF₂ | |
| 2-322 | OCH₂CH₂SO₂Me | Et | 2 | CHF₂ | |
| 2-323 | OCH₂CH₂SO₂Me | n-Pr | 2 | CHF₂ | |
| 2-324 | OCH₂CH₂SO₂Me | i-Pr | 2 | CHF₂ | |
| 2-325 | OMe | Me | 0 | CF(CF₃)₂ | |
| 2-326 | OMe | Et | 0 | CF(CF₃)₂ | |
| 2-327 | OMe | n-Pr | 0 | CF(CF₃)₂ | |
| 2-328 | OMe | i-Pr | 0 | CF(CF₃)₂ | |
| 2-329 | OMe | Me | 1 | CF(CF₃)₂ | |
| 2-330 | OMe | Et | 1 | CF(CF₃)₂ | |
| 2-331 | OMe | n-Pr | 1 | CF(CF₃)₂ | |
| 2-332 | OMe | i-Pr | 1 | CF(CF₃)₂ | |
| 2-333 | OMe | Me | 2 | CF(CF₃)₂ | |
| 2-334 | OMe | Et | 2 | CF(CF₃)₂ | |
| 2-335 | OMe | n-Pr | 2 | CF(CF₃)₂ | |
| 2-336 | OMe | i-Pr | 2 | CF(CF₃)₂ | |
| 2-337 | OEt | Me | 0 | CF(CF₃)₂ | |
| 2-338 | OEt | Et | 0 | CF(CF₃)₂ | |
| 2-339 | OEt | n-Pr | 0 | CF(CF₃)₂ | |
| 2-340 | OEt | i-Pr | 0 | CF(CF₃)₂ | |
| 2-341 | OEt | Me | 1 | CF(CF₃)₂ | |
| 2-342 | OEt | Et | 1 | CF(CF₃)₂ | |
| 2-343 | OEt | n-Pr | 1 | CF(CF₃)₂ | |
| 2-344 | OEt | i-Pr | 1 | CF(CF₃)₂ | |
| 2-345 | OEt | Me | 2 | CF(CF₃)₂ | |
| 2-346 | OEt | Et | 2 | CF(CF₃)₂ | |
| 2-347 | OEt | n-Pr | 2 | CF(CF₃)₂ | |
| 2-348 | OEt | i-Pr | 2 | CF(CF₃)₂ | |
| 2-349 | O - CH₂ - c-Pr | Me | 0 | CF(CF₃)₂ | |
| 2-350 | O - CH₂ - c-Pr | Et | 0 | CF(CF₃)₂ | |
| 2-351 | O - CH₂ - c-Pr | n-Pr | 0 | CF(CF₃)₂ | |
| 2-352 | O - CH₂ - c-Pr | i-Pr | 0 | CF(CF₃)₂ | |
| 2-353 | O - CH₂ - c-Pr | Me | 1 | CF(CF₃)₂ | |
| 2-354 | O - CH₂ - c-Pr | Et | 1 | CF(CF₃)₂ | |
| 2-355 | O - CH₂ - c-Pr | n-Pr | 1 | CF(CF₃)₂ | |
| 2-356 | O - CH₂ - c-Pr | i-Pr | 1 | CF(CF₃)₂ | |
| 2-357 | O - CH₂ - c-Pr | Me | 2 | CF(CF₃)₂ | |
| 2-358 | O - CH₂ - c-Pr | Et | 2 | CF(CF₃)₂ | |
| 2-359 | O - CH₂ - c-Pr | n-Pr | 2 | CF(CF₃)₂ | |
| 2-360 | O - CH₂ - c-Pr | i-Pr | 2 | CF(CF₃)₂ | |
| 2-361 | OCH₂CH₂OMe | Me | 0 | CF(CF₃)₂ | |
| 2-362 | OCH₂CH₂OMe | Et | 0 | CF(CF₃)₂ | |
| 2-363 | OCH₂CH₂OMe | n-Pr | 0 | CF(CF₃)₂ | |
| 2-364 | OCH₂CH₂OMe | i-Pr | 0 | CF(CF₃)₂ | |
| 2-365 | OCH₂CH₂OMe | Me | 1 | CF(CF₃)₂ | |
| 2-366 | OCH₂CH₂OMe | Et | 1 | CF(CF₃)₂ | |
| 2-367 | OCH₂CH₂OMe | n-Pr | 1 | CF(CF₃)₂ | |
| 2-368 | OCH₂CH₂OMe | i-Pr | 1 | CF(CF₃)₂ | |
| 2-369 | OCH₂CH₂OMe | Me | 2 | CF(CF₃)₂ | |
| 2-370 | OCH₂CH₂OMe | Et | 2 | CF(CF₃)₂ | |
| 2-371 | OCH₂CH₂OMe | n-Pr | 2 | CF(CF₃)₂ | |
| 2-372 | OCH₂CH₂OMe | i-Pr | 2 | CF(CF₃)₂ | |
| 2-373 | OCH₂CH₂SMe | Me | 0 | CF(CF₃)₂ | |
| 2-374 | OCH₂CH₂SMe | Et | 0 | CF(CF₃)₂ | |
| 2-375 | OCH₂CH₂SMe | n-Pr | 0 | CF(CF₃)₂ | |
| 2-376 | OCH₂CH₂SMe | i-Pr | 0 | CF(CF₃)₂ | |
| 2-377 | OCH₂CH₂SMe | Me | 1 | CF(CF₃)₂ | |
| 2-378 | OCH₂CH₂SMe | Et | 1 | CF(CF₃)₂ | |
| 2-379 | OCH₂CH₂SMe | n-Pr | 1 | CF(CF₃)₂ | |
| 2-380 | OCH₂CH₂SMe | i-Pr | 1 | CF(CF₃)₂ | |
| 2-381 | OCH₂CH₂SMe | Me | 2 | CF(CF₃)₂ | |
| 2-382 | OCH₂CH₂SMe | Et | 2 | CF(CF₃)₂ | |
| 2-383 | OCH₂CH₂SMe | n-Pr | 2 | CF(CF₃)₂ | |
| 2-384 | OCH₂CH₂SMe | i-Pr | 2 | CF(CF₃)₂ | |
| 2-385 | OCH₂CH₂SO₂Me | Me | 0 | CF(CF₃)₂ | |
| 2-386 | OCH₂CH₂SO₂Me | Et | 0 | CF(CF₃)₂ | |
| 2-387 | OCH₂CH₂SO₂Me | n-Pr | 0 | CF(CF₃)₂ | |
| 2-388 | OCH₂CH₂SO₂Me | i-Pr | 0 | CF(CF₃)₂ | |
| 2-389 | OCH₂CH₂SO₂Me | Me | 1 | CF(CF₃)₂ | |
| 2-390 | OCH₂CH₂SO₂Me | Et | 1 | CF(CF₃)₂ | |
| 2-391 | OCH₂CH₂SO₂Me | n-Pr | 1 | CF(CF₃)₂ | |
| 2-392 | OCH₂CH₂SO₂Me | i-Pr | 1 | CF(CF₃)₂ | |
| 2-393 | OCH₂CH₂SO₂Me | Me | 2 | CF(CF₃)₂ | |
| 2-394 | OCH₂CH₂SO₂Me | Et | 2 | CF(CF₃)₂ | |
| 2-395 | OCH₂CH₂SO₂Me | n-Pr | 2 | CF(CF₃)₂ | |
| 2-396 | OCH₂CH₂SO₂Me | i-Pr | 2 | CF(CF₃)₂ | |

**Tabelle 3: Erfindungsgemäße Verbindungen der allgemeinen Formel (I), worin R⁴ für Wasserstoff sowie R¹ und R² jeweils für Methyl stehen.**

| | | | | | |
|---|---|---|---|---|---|
| 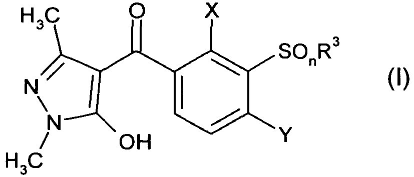 | | | | | |

| Nr. | X | R³ | n | Y | Physikalische Daten: ¹H-NMR: δ [CDCl₃] |
|---|---|---|---|---|---|
| 3-1 | OH | Me | 0 | CF₃ | |
| 3-2 | OH | Et | 0 | CF₃ | |
| 3-3 | OH | n-Pr | 0 | CF₃ | |
| 3-4 | OH | i-Pr | 0 | CF₃ | |
| 3-5 | OH | Me | 1 | CF₃ | |
| 3-6 | OH | Et | 1 | CF₃ | |
| 3-7 | OH | n-Pr | 1 | CF₃ | |
| 3-8 | OH | i-Pr | 1 | CF₃ | |
| 3-9 | OH | Me | 2 | CF₃ | |
| 3-10 | OH | Et | 2 | CF₃ | |
| 3-11 | OH | n-Pr | 2 | CF₃ | |
| 3-12 | OH | i-Pr | 2 | CF₃ | |
| 3-13 | OMe | Me | 0 | CF₃ | 7.57 (d, 1H), 7.31 (d, 1H), 3.88 (s, 3H), 3.63 (s, 3H), 2.44 (s, 3H), 1.77 (s, 3H) |
| 3-14 | OMe | Et | 0 | CF₃ | |
| 3-15 | OMe | n-Pr | 0 | CF₃ | |
| 3-16 | OMe | i-Pr | 0 | CF₃ | |
| 3-17 | OMe | Me | 1 | CF₃ | 7.62 (d, 1H), 7.53 (d, 1H), 3.94 (s, 3H), 3.66 (s, 3H), 3.13 (s, 3H), 1.84 (s, 3H) |
| 3-18 | OMe | Et | 1 | CF₃ | |
| 3-19 | OMe | n-Pr | 1 | CF₃ | |
| 3-20 | OMe | i-Pr | 1 | CF₃ | |
| 3-21 | OMe | Me | 2 | CF₃ | 7.78 (d, 1H), 7.62 (d, 1H), 3.92 (s, 3H), 3.66 (s, 3H), 3.33 (s, 3H), 1.82 (s, 3H) |
| 3-22 | OMe | Et | 2 | CF₃ | |
| 3-23 | OMe | n-Pr | 2 | CF₃ | |
| 3-24 | OMe | i-Pr | 2 | CF₃ | |
| 3-25 | OEt | Me | 0 | CF₃ | 7.55 (d, 1H), 7.31 (d, 1H), 4.11 (q, 2H), 3.63 (s, 3H), 2.46 (s, 3H), 1.78 (s, 3H), 1.30 (t, 3H) |
| 3-26 | OEt | Et | 0 | CF₃ | |
| 3-27 | OEt | n-Pr | 0 | CF₃ | |
| 3-28 | OEt | i-Pr | 0 | CF₃ | |
| 3-29 | OEt | Me | 1 | CF₃ | 7.59 (d, 1H), 7.51 (d, 1H), 4.30 (m, 1H), 4.00 (m, 1H), 3.66 (s, 3H), 3.14 (s, 3H), 1.84 (s, 3H), 1.30 (t, 3H) |
| 3-30 | OEt | Et | 1 | CF₃ | |
| 3-31 | OEt | n-Pr | 1 | CF₃ | |
| 3-32 | OEt | i-Pr | 1 | CF₃ | |
| 3-33 | OEt | Me | 2 | CF₃ | 7.76 (d, 1H), 7.61 (d, 1H), 4.13 (q, 2H), 3.67 (s, 3H), 3.37 (s, 3H), 1.82 (s, 3H), 1.30 (t, 3H) |
| 3-34 | OEt | Et | 2 | CF₃ | |
| 3-35 | OEt | n-Pr | 2 | CF₃ | |
| 3-36 | OEt | i-Pr | 2 | CF₃ | |
| 3-37 | O-CH₂₋c-Pr | Me | 0 | CF₃ | |
| 3-38 | O-CH₂₋c-Pr | Et | 0 | CF₃ | |
| 3-39 | O-CH₂₋c-Pr | n-Pr | 0 | CF₃ | |
| 3-40 | O-CH₂₋c-Pr | i-Pr | 0 | CF₃ | |
| 3-41 | O-CH₂₋c-Pr | Me | 1 | CF₃ | |
| 3-42 | O-CH₂₋c-Pr | Et | 1 | CF₃ | |
| 3-43 | O-CH₂₋c-Pr | n-Pr | 1 | CF₃ | |
| 3-44 | O-CH₂₋c-Pr | i-Pr | 1 | CF₃ | |
| 3-45 | O-CH₂₋c-Pr | Me | 2 | CF₃ | |
| 3-46 | O-CH₂₋c-Pr | Et | 2 | CF₃ | |
| 3-47 | O-CH₂₋c-Pr | n-Pr | 2 | CF₃ | |
| 3-48 | O-CH₂₋c-Pr | i-Pr | 2 | CF₃ | |
| 3-49 | OCH₂CH₂OMe | Me | 0 | CF₃ | 7.56 (d, 1H), 7.33 (d, 1H), 4.23 (m, 2H), 3.64 (s, 3H), 3.60 (t, 2H), 3.31 (s, 3H), 2.49 (s, 3H), 1.79 (s, 3H) |
| 3-50 | OCH₂CH₂OMe | Et | 0 | CF₃ | |
| 3-51 | OCH₂CH₂OMe | n-Pr | 0 | CF₃ | |
| 3-52 | OCH₂CH₂OMe | i-Pr | 0 | CF₃ | |
| 3-53 | OCH₂CH₂OMe | Me | 1 | CF₃ | 7.61 (d, 1H), 7.52 (d, 1H), 4.42 (m, 1H), 4.10 (m, 1H), 3.75 (m, 1H), 3.66 (s, 3H), 3.55 (m, 1H), 3.28 (s, 3H), 3.16 (s, 3H), 1.83 (s, 3H) |
| 3-54 | OCH₂CH₂OMe | Et | 1 | CF₃ | |
| 3-55 | OCH₂CH₂OMe | n-Pr | 1 | CF₃ | |
| 3-56 | OCH₂CH₂OMe | i-Pr | 1 | CF₃ | |
| 3-57 | OCH₂CH₂OMe | Me | 2 | CF₃ | 7.77 (d, 1H), 7.61 (d, 1H), 4.25 (t, 2H), 3.65 (s, 3H), 3.62 (t, 2H), 3.42 (s, 3H), 3.30 (s, 3H), 1.82 (s, 3H) |
| 3-58 | OCH₂CH₂OMe | Et | 2 | CF₃ | |
| 3-59 | OCH₂CH₂OMe | n-Pr | 2 | CF₃ | |
| 3-60 | OCH₂CH₂OMe | i-Pr | 2 | CF₃ | |
| 3-61 | OCH₂CH₂SMe | Me | 0 | CF₃ | |
| 3-62 | OCH₂CH₂SMe | Et | 0 | CF₃ | |
| 3-63 | OCH₂CH₂SMe | n-Pr | 0 | CF₃ | |
| 3-64 | OCH₂CH₂SMe | i-Pr | 0 | CF₃ | |
| 3-65 | OCH₂CH₂SMe | Me | 1 | CF₃ | |
| 3-66 | OCH₂CH₂SMe | Et | 1 | CF₃ | |
| 3-67 | OCH₂CH₂SMe | n-Pr | 1 | CF₃ | |
| 3-68 | OCH₂CH₂SMe | i-Pr | 1 | CF₃ | |
| 3-69 | OCH₂CH₂SMe | Me | 2 | CF₃ | |
| 3-70 | OCH₂CH₂SMe | Et | 2 | CF₃ | |
| 3-71 | OCH₂CH₂SMe | n-Pr | 2 | CF₃ | |
| 3-72 | OCH₂CH₂SMe | i-Pr | 2 | CF₃ | |
| 3-73 | OCH₂CH₂SO₂Me | Me | 0 | CF₃ | |
| 3-74 | OCH₂CH₂SO₂Me | Et | 0 | CF₃ | |
| 3-75 | OCH₂CH₂SO₂Me | n-Pr | 0 | CF₃ | |
| 3-76 | OCH₂CH₂SO₂Me | i-Pr | 0 | CF₃ | |
| 3-77 | OCH₂CH₂SO₂Me | Me | 1 | CF₃ | |
| 3-78 | OCH₂CH₂SO₂Me | Et | 1 | CF₃ | |
| 3-79 | OCH₂CH₂SO₂Me | n-Pr | 1 | CF₃ | |
| 3-80 | OCH₂CH₂SO₂Me | i-Pr | 1 | CF₃ | |
| 3-81 | OCH₂CH₂SO₂Me | Me | 2 | CF₃ | |
| 3-82 | OCH₂CH₂SO₂Me | Et | 2 | CF₃ | |
| 3-83 | OCH₂CH₂SO₂Me | n-Pr | 2 | CF₃ | |
| 3-84 | OCH₂CH₂SO₂Me | i-Pr | 2 | CF₃ | |
| 3-85 | OCOMe | Me | 0 | CF₃ | |
| 3-86 | OCOMe | Et | 0 | CF₃ | |
| 3-87 | OCOMe | n-Pr | 0 | CF₃ | |
| 3-88 | OCOMe | i-Pr | 0 | CF₃ | |
| 3-89 | OCOMe | Me | 1 | CF₃ | |
| 3-90 | OCOMe | Et | 1 | CF₃ | |
| 3-91 | OCOMe | n-Pr | 1 | CF₃ | |
| 3-92 | OCOMe | i-Pr | 1 | CF₃ | |
| 3-93 | OCOMe | Me | 2 | CF₃ | |
| 3-94 | OCOMe | Et | 2 | CF₃ | |
| 3-95 | OCOMe | n-Pr | 2 | CF₃ | |
| 3-96 | OCOMe | i-Pr | 2 | CF₃ | |
| 3-97 | OSO₂Me | Me | 0 | CF₃ | |
| 3-98 | OSO₂Me | Et | 0 | CF₃ | |
| 3-99 | OSO₂Me | n-Pr | 0 | CF₃ | |
| 3-100 | OSO₂Me | i-Pr | 0 | CF₃ | |
| 3-101 | OSO₂Me | Me | 1 | CF₃ | |
| 3-102 | OSO₂Me | Et | 1 | CF₃ | |
| 3-103 | OSO₂Me | n-Pr | 1 | CF₃ | |
| 3-104 | OSO₂Me | i-Pr | 1 | CF₃ | |
| 3-105 | OSO₂Me | Me | 2 | CF₃ | |
| 3-106 | OSO₂Me | Et | 2 | CF₃ | |
| 3-107 | OSO₂Me | n-Pr | 2 | CF₃ | |
| 3-108 | OSO₂Me | i-Pr | 2 | CF₃ | |
| 3-109 | OMe | Me | 0 | C₂F₅ | |
| 3-110 | OMe | Et | 0 | C₂F₅ | |
| 3-111 | OMe | n-Pr | 0 | C₂F₅ | |
| 3-112 | OMe | i-Pr | 0 | C₂F₅ | |
| 3-113 | OMe | Me | 1 | C₂F₅ | |
| 3-114 | OMe | Et | 1 | C₂F₅ | |
| 3-115 | OMe | n-Pr | 1 | C₂F₅ | |
| 3-116 | OMe | i-Pr | 1 | C₂F₅ | |
| 3-117 | OMe | Me | 2 | C₂F₅ | |
| 3-118 | OMe | Et | 2 | C₂F₅ | |
| 3-119 | OMe | n-Pr | 2 | C₂F₅ | |
| 3-120 | OMe | i-Pr | 2 | C₂F₅ | |
| 3-121 | OEt | Me | 0 | C₂F₅ | |
| 3-122 | OEt | Et | 0 | C₂F₅ | |
| 3-123 | OEt | n-Pr | 0 | C₂F₅ | |
| 3-124 | OEt | i-Pr | 0 | C₂F₅ | |
| 3-125 | OEt | Me | 1 | C₂F₅ | |
| 3-126 | OEt | Et | 1 | C₂F₅ | |
| 3-127 | OEt | n-Pr | 1 | C₂F₅ | |
| 3-128 | OEt | i-Pr | 1 | C₂F₅ | |
| 3-129 | OEt | Me | 2 | C₂F₅ | |
| 3-130 | OEt | Et | 2 | C₂F₅ | |
| 3-131 | OEt | n-Pr | 2 | C₂F₅ | |
| 3-132 | OEt | i-Pr | 2 | C₂F₅ | |
| 3-133 | O-CH₂₋c-Pr | Me | 0 | C₂F₅ | |
| 3-134 | O-CH₂₋c-Pr | Et | 0 | C₂F₅ | |
| 3-135 | O-CH₂₋c-Pr | n-Pr | 0 | C₂F₅ | |
| 3-136 | O-CH₂₋c-Pr | i-Pr | 0 | C₂F₅ | |
| 3-137 | O-CH₂₋c-Pr | Me | 1 | C₂F₅ | |
| 3-138 | O-CH₂₋c-Pr | Et | 1 | C₂F₅ | |
| 3-139 | O-CH₂₋c-Pr | n-Pr | 1 | C₂F₅ | |
| 3-140 | O-CH₂₋c-Pr | i-Pr | 1 | C₂F₅ | |
| 3-141 | O-CH₂₋c-Pr | Me | 2 | C₂F₅ | |
| 3-142 | O-CH₂₋c-Pr | Et | 2 | C₂F₅ | |
| 3-143 | O-CH₂₋c-Pr | n-Pr | 2 | C₂F₅ | |
| 3-144 | O-CH₂₋c-Pr | i-Pr | 2 | C₂F₅ | |
| 3-145 | OCH₂CH₂OMe | Me | 0 | C₂F₅ | |
| 3-146 | OCH₂CH₂OMe | Et | 0 | C₂F₅ | |
| 3-147 | OCH₂CH₂OMe | n-Pr | 0 | C₂F₅ | |
| 3-148 | OCH₂CH₂OMe | i-Pr | 0 | C₂F₅ | |
| 3-149 | OCH₂CH₂OMe | Me | 1 | C₂F₅ | |
| 3-150 | OCH₂CH₂OMe | Et | 1 | C₂F₅ | |
| 3-151 | OCH₂CH₂OMe | n-Pr | 1 | C₂F₅ | |
| 3-152 | OCH₂CH₂OMe | i-Pr | 1 | C₂F₅ | |
| 3-153 | OCH₂CH₂OMe | Me | 2 | C₂F₅ | |
| 3-154 | OCH₂CH₂OMe | Et | 2 | C₂F₅ | |
| 3-155 | OCH₂CH₂OMe | n-Pr | 2 | C₂F₅ | |
| 3-156 | OCH₂CH₂OMe | i-Pr | 2 | C₂F₅ | |
| 3-157 | OCH₂CH₂SMe | Me | 0 | C₂F₅ | |
| 3-158 | OCH₂CH₂SMe | Et | 0 | C₂F₅ | |
| 3-159 | OCH₂CH₂SMe | n-Pr | 0 | C₂F₅ | |
| 3-160 | OCH₂CH₂SMe | i-Pr | 0 | C₂F₅ | |
| 3-161 | OCH₂CH₂SMe | Me | 1 | C₂F₅ | |
| 3-162 | OCH₂CH₂SMe | Et | 1 | C₂F₅ | |
| 3-163 | OCH₂CH₂SMe | n-Pr | 1 | C₂F₅ | |
| 3-164 | OCH₂CH₂SMe | i-Pr | 1 | C₂F₅ | |
| 3-165 | OCH₂CH₂SMe | Me | 2 | C₂F₅ | |
| 3-166 | OCH₂CH₂SMe | Et | 2 | C₂F₅ | |
| 3-167 | OCH₂CH₂SMe | n-Pr | 2 | C₂F₅ | |
| 3-168 | OCH₂CH₂SMe | i-Pr | 2 | C₂F₅ | |
| 3-169 | OCH₂CH₂SO₂Me | Me | 0 | C₂F₅ | |
| 3-170 | OCH₂CH₂SO₂Me | Et | 0 | C₂F₅ | |
| 3-171 | OCH₂CH₂SO₂Me | n-Pr | 0 | C₂F₅ | |
| 3-172 | OCH₂CH₂SO₂Me | i-Pr | 0 | C₂F₅ | |
| 3-173 | OCH₂CH₂SO₂Me | Me | 1 | C₂F₅ | |
| 3-174 | OCH₂CH₂SO₂Me | Et | 1 | C₂F₅ | |
| 3-175 | OCH₂CH₂SO₂Me | n-Pr | 1 | C₂F₅ | |
| 3-176 | OCH₂CH₂SO₂Me | i-Pr | 1 | C₂F₅ | |
| 3-177 | OCH₂CH₂SO₂Me | Me | 2 | C₂F₅ | |
| 3-178 | OCH₂CH₂SO₂Me | Et | 2 | C₂F₅ | |
| 3-179 | OCH₂CH₂SO₂Me | n-Pr | 2 | C₂F₅ | |
| 3-180 | OCH₂CH₂SO₂Me | i-Pr | 2 | C₂F₅ | |
| 3-181 | OMe | Me | 0 | CCl₃ | |
| 3-182 | OMe | Et | 0 | CCl₃ | |
| 3-183 | OMe | n-Pr | 0 | CCl₃ | |
| 3-184 | OMe | i-Pr | 0 | CCl₃ | |
| 3-185 | OMe | Me | 1 | CCl₃ | |
| 3-186 | OMe | Et | 1 | CCl₃ | |
| 3-187 | OMe | n-Pr | 1 | CCl₃ | |
| 3-188 | OMe | i-Pr | 1 | CCl₃ | |
| 3-189 | OMe | Me | 2 | CCl₃ | |
| 3-190 | OMe | Et | 2 | CCl₃ | |
| 3-191 | OMe | n-Pr | 2 | CCl₃ | |
| 3-192 | OMe | i-Pr | 2 | CCl₃ | |
| 3-193 | OEt | Me | 0 | CCl₃ | |
| 3-194 | OEt | Et | 0 | CCl₃ | |
| 3-195 | OEt | n-Pr | 0 | CCl₃ | |
| 3-196 | OEt | i-Pr | 0 | CCl₃ | |
| 3-197 | OEt | Me | 1 | CCl₃ | |
| 3-198 | OEt | Et | 1 | CCl₃ | |
| 3-199 | OEt | n-Pr | 1 | CCl₃ | |
| 3-200 | OEt | i-Pr | 1 | CCl₃ | |
| 3-201 | OEt | Me | 2 | CCl₃ | |
| 3-202 | OEt | Et | 2 | CCl₃ | |
| 3-203 | OEt | n-Pr | 2 | CCl₃ | |
| 3-204 | OEt | i-Pr | 2 | CCl₃ | |
| 3-205 | O-CH₂₋c-Pr | Me | 0 | CCl₃ | |
| 3-206 | O-CH₂₋c-Pr | Et | 0 | CCl₃ | |
| 3-207 | O-CH₂₋c-Pr | n-Pr | 0 | CCl₃ | |
| 3-208 | O-CH₂₋c-Pr | i-Pr | 0 | CCl₃ | |
| 3-209 | O-CH₂₋c-Pr | Me | 1 | CCl₃ | |
| 3-210 | O-CH₂₋c-Pr | Et | 1 | CCl₃ | |
| 3-211 | O-CH₂₋c-Pr | n-Pr | 1 | CCl₃ | |
| 3-212 | O-CH₂₋c-Pr | i-Pr | 1 | CCl₃ | |
| 3-213 | O-CH₂₋c-Pr | Me | 2 | CCl₃ | |
| 3-214 | O-CH₂₋c-Pr | Et | 2 | CCl₃ | |
| 3-215 | O-CH₂₋c-Pr | n-Pr | 2 | CCl₃ | |
| 3-216 | O-CH₂₋c-Pr | i-Pr | 2 | CCl₃ | |
| 3-217 | OCH₂CH₂OMe | Me | 0 | CCl₃ | |
| 3-218 | OCH₂CH₂OMe | Et | 0 | CCl₃ | |
| 3-219 | OCH₂CH₂OMe | n-Pr | 0 | CCl₃ | |
| 3-220 | OCH₂CH₂OMe | i-Pr | 0 | CCl₃ | |
| 3-221 | OCH₂CH₂OMe | Me | 1 | CCl₃ | |
| 3-222 | OCH₂CH₂OMe | Et | 1 | CCl₃ | |
| 3-223 | OCH₂CH₂OMe | n-Pr | 1 | CCl₃ | |
| 3-224 | OCH₂CH₂OMe | i-Pr | 1 | CCl₃ | |
| 3-225 | OCH₂CH₂OMe | Me | 2 | CCl₃ | |
| 3-226 | OCH₂CH₂OMe | Et | 2 | CCl₃ | |
| 3-227 | OCH₂CH₂OMe | n-Pr | 2 | CCl₃ | |
| 3-228 | OCH₂CH₂OMe | i-Pr | 2 | CCl₃ | |
| 3-229 | OCH₂CH₂SMe | Me | 0 | CCl₃ | |
| 3-230 | OCH₂CH₂SMe | Et | 0 | CCl₃ | |
| 3-231 | OCH₂CH₂SMe | n-Pr | 0 | CCl₃ | |
| 3-232 | OCH₂CH₂SMe | i-Pr | 0 | CCl₃ | |
| 3-233 | OCH₂CH₂SMe | Me | 1 | CCl₃ | |
| 3-234 | OCH₂CH₂SMe | Et | 1 | CCl₃ | |
| 3-235 | OCH₂CH₂SMe | n-Pr | 1 | CCl₃ | |
| 3-236 | OCH₂CH₂SMe | i-Pr | 1 | CCl₃ | |
| 3-237 | OCH₂CH₂SMe | Me | 2 | CCl₃ | |
| 3-238 | OCH₂CH₂SMe | Et | 2 | CCl₃ | |
| 3-239 | OCH₂CH₂SMe | n-Pr | 2 | CCl₃ | |
| 3-240 | OCH₂CH₂SMe | i-Pr | 2 | CCl₃ | |
| 3-241 | OCH₂CH₂SO₂Me | Me | 0 | CCl₃ | |
| 3-242 | OCH₂CH₂SO₂Me | Et | 0 | CCl₃ | |
| 3-243 | OCH₂CH₂SO₂Me | n-Pr | 0 | CCl₃ | |
| 3-244 | OCH₂CH₂SO₂Me | i-Pr | 0 | CCl₃ | |
| 3-245 | OCH₂CH₂SO₂Me | Me | 1 | CCl₃ | |
| 3-246 | OCH₂CH₂SO₂Me | Et | 1 | CCl₃ | |
| 3-247 | OCH₂CH₂SO₂Me | n-Pr | 1 | CCl₃ | |
| 3-248 | OCH₂CH₂SO₂Me | i-Pr | 1 | CCl₃ | |
| 3-249 | OCH₂CH₂SO₂Me | Me | 2 | CCl₃ | |
| 3-250 | OCH₂CH₂SO₂Me | Et | 2 | CCl₃ | |
| 3-251 | OCH₂CH₂SO₂Me | n-Pr | 2 | CCl₃ | |
| 3-252 | OCH₂CH₂SO₂Me | i-Pr | 2 | CCl₃ | |
| 3-253 | OMe | Me | 0 | CHF₂ | |
| 3-254 | OMe | Et | 0 | CHF₂ | |
| 3-255 | OMe | n-Pr | 0 | CHF₂ | |
| 3-256 | OMe | i-Pr | 0 | CHF₂ | |
| 3-257 | OMe | Me | 1 | CHF₂ | |
| 3-258 | OMe | Et | 1 | CHF₂ | |
| 3-259 | OMe | n-Pr | 1 | CHF₂ | |
| 3-260 | OMe | i-Pr | 1 | CHF₂ | |
| 3-261 | OMe | Me | 2 | CHF₂ | |
| 3-262 | OMe | Et | 2 | CHF₂ | |
| 3-263 | OMe | n-Pr | 2 | CHF₂ | |
| 3-264 | OMe | i-Pr | 2 | CHF₂ | |
| 3-265 | OEt | Me | 0 | CHF₂ | |
| 3-266 | OEt | Et | 0 | CHF₂ | |
| 3-267 | OEt | n-Pr | 0 | CHF₂ | |
| 3-268 | OEt | i-Pr | 0 | CHF₂ | |
| 3-269 | OEt | Me | 1 | CHF₂ | |
| 3-270 | OEt | Et | 1 | CHF₂ | |
| 3-271 | OEt | n-Pr | 1 | CHF₂ | |
| 3-272 | OEt | i-Pr | 1 | CHF₂ | |
| 3-273 | OEt | Me | 2 | CHF₂ | |
| 3-274 | OEt | Et | 2 | CHF₂ | |
| 3-275 | OEt | n-Pr | 2 | CHF₂ | |
| 3-276 | OEt | i-Pr | 2 | CHF₂ | |
| 3-277 | O-CH₂-c-Pr | Me | 0 | CHF₂ | |
| 3-278 | O-CH₂-c-Pr | Et | 0 | CHF₂ | |
| 3-279 | O-CH₂-c-Pr | n-Pr | 0 | CHF₂ | |
| 3-280 | O-CH₂-c-Pr | i-Pr | 0 | CHF₂ | |
| 3-281 | O-CH₂-c-Pr | Me | 1 | CHF₂ | |
| 3-282 | O-CH₂-c-Pr | Et | 1 | CHF₂ | |
| 3-283 | O-CH₂-c-Pr | n-Pr | 1 | CHF₂ | |
| 3-284 | O-CH₂-c-Pr | i-Pr | 1 | CHF₂ | |
| 3-285 | O-CH₂-c-Pr | Me | 2 | CHF₂ | |
| 3-286 | O-CH₂-c-Pr | Et | 2 | CHF₂ | |
| 3-287 | O-CH₂-c-Pr | n-Pr | 2 | CHF₂ | |
| 3-288 | O-CH₂-c-Pr | i-Pr | 2 | CHF₂ | |
| 3-289 | OCH₂CH₂OMe | Me | 0 | CHF₂ | |
| 3-290 | OCH₂CH₂OMe | Et | 0 | CHF₂ | |
| 3-291 | OCH₂CH₂OMe | n-Pr | 0 | CHF₂ | |
| 3-292 | OCH₂CH₂OMe | i-Pr | 0 | CHF₂ | |
| 3-293 | OCH₂CH₂OMe | Me | 1 | CHF₂ | |
| 3-294 | OCH₂CH₂OMe | Et | 1 | CHF₂ | |
| 3-295 | OCH₂CH₂OMe | n-Pr | 1 | CHF₂ | |
| 3-296 | OCH₂CH₂OMe | i-Pr | 1 | CHF₂ | |
| 3-297 | OCH₂CH₂OMe | Me | 2 | CHF₂ | |
| 3-298 | OCH₂CH₂OMe | Et | 2 | CHF₂ | |
| 3-299 | OCH₂CH₂OMe | n-Pr | 2 | CHF₂ | |
| 3-300 | OCH₂CH₂OMe | i-Pr | 2 | CHF₂ | |
| 3-301 | OCH₂CH₂SMe | Me | 0 | CHF₂ | |
| 3-302 | OCH₂CH₂SMe | Et | 0 | CHF₂ | |
| 3-303 | OCH₂CH₂SMe | n-Pr | 0 | CHF₂ | |
| 3-304 | OCH₂CH₂SMe | i-Pr | 0 | CHF₂ | |
| 3-305 | OCH₂CH₂SMe | Me | 1 | CHF₂ | |
| 3-306 | OCH₂CH_{z}SMe | Et | 1 | CHF₂ | |
| 3-307 | OCH₂CH₂SMe | n-Pr | 1 | CHF₂ | |
| 3-308 | OCH₂CH₂SMe | i-Pr | 1 | CHF₂ | |
| 3-309 | OCH₂CH₂SMe | Me | 2 | CHF₂ | |
| 3-310 | OCH₂CH₂SMe | Et | 2 | CHF₂ | |
| 3-311 | OCH₂CH₂SMe | n-Pr | 2 | CHF₂ | |
| 3-312 | OCH₂CH₂SMe | i-Pr | 2 | CHF₂ | |
| 3-313 | OCH₂CH₂SO₂Me | Me | 0 | CHF₂ | |
| 3-314 | OCH₂CH₂SO₂Me | Et | 0 | CHF₂ | |
| 3-315 | OCH₂CH₂SO₂Me | n-Pr | 0 | CHF₂ | |
| 3-316 | OCH₂CH₂SO₂Me | i-Pr | 0 | CHF₂ | |
| 3-317 | OCH₂CH₂SO₂Me | Me | 1 | CHF₂ | |
| 3-318 | OCH₂CH₂SO₂Me | Et | 1 | CHF₂ | |
| 3-319 | OCH₂CH₂SO₂Me | n-Pr | 1 | CHF₂ | |
| 3-320 | OCH₂CH₂SO₂Me | i-Pr | 1 | CHF₂ | |
| 3-321 | OCH₂CH₂SO₂Me | Me | 2 | CHF₂ | |
| 3-322 | OCH₂CH₂SO₂Me | Et | 2 | CHF₂ | |
| 3-323 | OCH₂CH₂SO₂Me | n-Pr | 2 | CHF₂ | |
| 3-324 | OCH₂CH₂SO₂Me | i-Pr | 2 | CHF₂ | |
| 3-325 | OMe | Me | 0 | CF(CF₃)₂ | |
| 3-326 | OMe | Et | 0 | CF(CF₃)₂ | |
| 3-327 | OMe | n-Pr | 0 | CF(CF₃)₂ | |
| 3-328 | OMe | i-Pr | 0 | CF(CF₃)₂ | |
| 3-329 | OMe | Me | 1 | CF(CF₃)₂ | |
| 3-330 | OMe | Et | 1 | CF(CF₃)₂ | |
| 3-331 | OMe | n-Pr | 1 | CF(CF₃)₂ | |
| 3-332 | OMe | i-Pr | 1 | CF(CF₃)₂ | |
| 3-333 | OMe | Me | 2 | CF(CF₃)₂ | |
| 3-334 | OMe | Et | 2 | CF(CF₃)₂ | |
| 3-335 | OMe | n-Pr | 2 | CF(CF₃)₂ | |
| 3-336 | OMe | i-Pr | 2 | CF(CF₃)₂ | |
| 3-337 | OEt | Me | 0 | CF(CF₃)₂ | |
| 3-338 | OEt | Et | 0 | CF(CF₃)₂ | |
| 3-339 | OEt | n-Pr | 0 | CF(CF₃)₂ | |
| 3-340 | OEt | i-Pr | 0 | CF(CF₃)₂ | |
| 3-341 | OEt | Me | 1 | CF(CF₃)₂ | |
| 3-342 | OEt | Et | 1 | CF(CF₃)₂ | |
| 3-343 | OEt | n-Pr | 1 | CF(CF₃)₂ | |
| 3-344 | OEt | i-Pr | 1 | CF(CF₃)₂ | |
| 3-345 | OEt | Me | 2 | CF(CF₃)₂ | |
| 3-346 | OEt | Et | 2 | CF(CF₃)₂ | |
| 3-347 | OEt | n-Pr | 2 | CF(CF₃)₂ | |
| 3-348 | OEt | i-Pr | 2 | CF(CF₃)₂ | |
| 3-349 | O-CH₂-c-Pr | Me | 0 | CF(CF₃)₂ | |
| 3-350 | O-CH₂-c-Pr | Et | 0 | CF(CF₃)₂ | |
| 3-351 | O-CH₂-c-Pr | n-Pr | 0 | CF(CF₃)₂ | |
| 3-352 | O-CH₂-c-Pr | i-Pr | 0 | CF(CF₃)₂ | |
| 3-353 | O-CH₂-c-Pr | Me | 1 | CF(CF₃)₂ | |
| 3-354 | O-CH₂-c-Pr | Et | 1 | CF(CF₃)₂ | |
| 3-355 | O-CH₂-c-Pr | n-Pr | 1 | CF(CF₃)₂ | |
| 3-356 | O-CH₂-c-Pr | i-Pr | 1 | CF(CF₃)₂ | |
| 3-357 | O-CH₂-c-Pr | Me | 2 | CF(CF₃)₂ | |
| 3-358 | O-CH₂-c-Pr | Et | 2 | CF(CF₃)₂ | |
| 3-359 | O-CH₂-c-Pr | n-Pr | 2 | CF(CF₃)₂ | |
| 3-360 | O-CH₂-c-Pr | i-Pr | 2 | CF(CF₃)₂ | |
| 3-361 | OCH₂CH₂OMe | Me | 0 | CF(CF₃)₂ | |
| 3-362 | OCH₂CH₂OMe | Et | 0 | CF(CF₃)₂ | |
| 3-363 | OCH₂CH₂OMe | n-Pr | 0 | CF(CF₃)₂ | |
| 3-364 | OCH₂CH₂OMe | i-Pr | 0 | CF(CF₃)₂ | |
| 3-365 | OCH₂CH₂OMe | Me | 1 | CF(CF₃)₂ | |
| 3-366 | OCH₂CH₂OMe | Et | 1 | CF(CF₃)₂ | |
| 3-367 | OCH₂CH₂OMe | n-Pr | 1 | CF(CF₃)₂ | |
| 3-368 | OCH₂CH₂OMe | i-Pr | 1 | CF(CF₃)₂ | |
| 3-369 | OCH₂CH₂OMe | Me | 2 | CF(CF₃)₂ | |
| 3-370 | OCH₂CH₂OMe | Et | 2 | CF(CF₃)₂ | |
| 3-371 | OCH₂CH₂OMe | n-Pr | 2 | CF(CF₃)₂ | |
| 3-372 | OCH₂CH₂OMe | i-Pr | 2 | CF(CF₃)₂ | |
| 3-373 | OCH₂CH₂SMe | Me | 0 | CF(CF₃)₂ | |
| 3-374 | OCH₂CH₂SMe | Et | 0 | CF(CF₃)₂ | |
| 3-375 | OCH₂CH₂SMe | n-Pr | 0 | CF(CF₃)₂ | |
| 3-376 | OCH₂CH₂SMe | i-Pr | 0 | CF(CF₃)₂ | |
| 3-377 | OCH₂CH₂SMe | Me | 1 | CF(CF₃)₂ | |
| 3-378 | OCH₂CH₂SMe | Et | 1 | CF(CF₃)₂ | |
| 3-379 | OCH₂CH₂SMe | n-Pr | 1 | CF(CF₃)₂ | |
| 3-380 | OCH₂CH₂SMe | i-Pr | 1 | CF(CF₃)₂ | |
| 3-381 | OCH₂CH₂SMe | Me | 2 | CF(CF₃)₂ | |
| 3-382 | OCH₂CH₂SMe | Et | 2 | CF(CF₃)₂ | |
| 3-383 | OCH₂CH₂SMe | n-Pr | 2 | CF(CF₃)₂ | |
| 3-384 | OCH₂CH₂SMe | i-Pr | 2 | CF(CF₃)₂ | |
| 3-385 | OCH₂CH₂SO₂Me | Me | 0 | CF(CF₃)₂ | |
| 3-386 | OCH₂CH₂SO₂Me | Et | 0 | CF(CF₃)₂ | |
| 3-387 | OCH₂CH₂SO₂Me | n-Pr | 0 | CF(CF₃)₂ | |
| 3-388 | OCH₂CH₂SO₂Me | i-Pr | 0 | CF(CF₃)₂ | |
| 3-389 | OCH₂CH₂SO₂Me | Me | 1 | CF(CF₃)₂ | |
| 3-390 | OCH₂CH₂SO₂Me | Et | 1 | CF(CF₃)₂ | |
| 3-391 | OCH₂CH₂SO₂Me | n-Pr | 1 | CF(CF₃)₂ | |
| 3-392 | OCH₂CH₂SO₂Me | i-Pr | 1 | CF(CF₃)₂ | |
| 3-393 | OCH₂CH₂SO₂Me | Me | 2 | CF(CF₃)₂ | |
| 3-394 | OCH₂CH₂SO₂Me | Et | 2 | CF(CF₃)₂ | |
| 3-395 | OCH₂CH₂SO₂Me | n-Pr | 2 | CF(CF₃)₂ | |
| 3-396 | OCH₂CH₂SO₂Me | i-Pr | 2 | CF(CF₃)₂ | |

**Tabelle 4: Erfindungsgemäße Verbindungen der allgemeinen Formel (I), worin R¹ für Methyl und R² für Wasserstoff steht**

| | | | | | | |
|---|---|---|---|---|---|---|
| 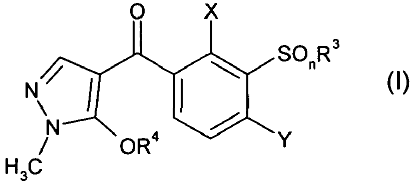 | | | | | | |

| Nr. | X | R³ | n | Y | R⁴ | Physikalische Daten: ¹H-NMR: δ [CDCl₃] |
|---|---|---|---|---|---|---|
| 4-1 | OMe | Me | 0 | CF₃ | SO₂-n-P | |
| 4-2 | OMe | Et | 0 | CF₃ | SO₂-n-Pr | |
| 4-3 | OMe | n-Pr | 0 | CF₃ | SO₂-n-P | |
| 4-4 | OMe | i-Pr | 0 | CF₃ | SO₂-n-Pr | |
| 4-5 | OMe | Me | 0 | CF₃ | SO₂-CH₂)₂OMe | |
| 4-6 | OMe | Et | 0 | CF₃ | SO₂-(CH₂)₂OMe | |
| 4-7 | OMe | n-Pr | 0 | CF₃ | SO₂-(CH₂)₂OMe | |
| 4-8 | OMe | i-Pr | 0 | CF₃ | SO₂-(CH₂)₂OMe | |
| 4-9 | OMe | Me | 0 | CF₃ | SO₂-Ph | |
| 4-10 | OMe | Et | 0 | CF₃ | SO₂-Ph | |
| 4-11 | OMe | n-Pr | 0 | CF₃ | SO₂-Ph | |
| 4-12 | OMe | i-Pr | 0 | CF₃ | SO₂-Ph | |
| 4-13 | OMe | Me | 0 | CF₃ | SO₂-(4-Me-Ph) | |
| 4-14 | OMe | Et | 0 | CF₃ | SO₂-(4-Me-Ph) | |
| 4-15 | OMe | n-Pr | 0 | CF₃ | SO₂-(4-Me-Ph) | |
| 4-16 | OMe | i-Pr | 0 | CF₃ | SO₂-(4-Me-Ph) | |
| 4-17 | OMe | Me | 0 | CF₃ | SO₂-(Thien-2-yl | |
| 4-18 | OMe | Et | 0 | CF₃ | SO₂-(Thien-2-yl) | |
| 4-19 | OMe | n-Pr | 0 | CF₃ | SO₂-(Thien-2-yl) | |
| 4-20 | OMe | i-Pr | 0 | CF₃ | SO₂-(Thien-2-yl) | |
| 4-21 | OMe | Me | 0 | CF₃ | CO-Ph | |
| 4-22 | OMe | Et | 0 | CF₃ | CO-Ph | |
| 4-23 | OMe | n-Pr | 0 | CF₃ | CO-Ph | |
| 4-24 | OMe | i-Pr | 0 | CF₃ | CO-Ph | |
| 4-25 | OMe | Me | 0 | CF₃ | CH₂-CO-Ph | |
| 4-26 | OMe | Et | 0 | CF₃ | CH₂-CO-Ph | |
| 4-27 | OMe | n-Pr | 0 | CF₃ | CH₂-CO-Ph | |
| 4-28 | OMe | i-Pr | 0 | CF₃ | CH₂-CO-Ph | |
| 4-29 | OMe | Me | 0 | CF₃ | CH₂-CO-(4-Me-Ph) | |
| 4-30 | OMe | Et | 0 | CF₃ | CH₂-CO-(4-Me-Ph) | |
| 4-31 | OMe | n-Pr | 0 | CF₃ | CH₂-CO-(4-Me-Ph) | |
| 4-32 | OMe | i-Pr | 0 | CF₃ | CH₂-CO-(4-Me-Ph) | |
| 4-33 | OMe | Me | 1 | CF₃ | SO₂-n-Pr | |
| 4-34 | OMe | Et | 1 | CF₃ | SO₂-n-Pr | |
| 4-35 | OMe | n-Pr | 1 | CF₃ | SO₂-n-Pr | |
| 4-36 | OMe | i-Pr | 1 | CF₃ | SO₂-n-Pr | |
| 4-37 | OMe | Me | 1 | CF₃ | SO₂-(CH₂)₂OMe | |
| 4-38 | OMe | Et | 1 | CF₃ | SO₂-(CH₂)₂OMe | |
| 4-39 | OMe | n-Pr | 1 | CF₃ | SO₂-(CH₂)₂OMe | |
| 4-40 | OMe | i-Pr | 1 | CF₃ | SO₂-(CH₂)₂OMe | |
| 4-41 | OMe | Me | 1 | CF₃ | SO₂-Ph | |
| 4-42 | OMe | Et | 1 | CF₃ | SO₂-Ph | |
| 4-43 | OMe | n-Pr | 1 | CF₃ | SO₂-Ph | |
| 4-44 | OMe | i-Pr | 1 | CF₃ | SO₂-Ph | |
| 4-45 | OMe | Me | 1 | CF₃ | SO₂-(4-Me-Ph) | |
| 4-46 | OMe | Et | 1 | CF₃ | SO₂-(4-Me-Ph) | |
| 4-47 | OMe | n-Pr | 1 | CF₃ | SO₂-(4-Me-Ph) | |
| 4-48 | OMe | i-Pr | 1 | CF₃ | SO₂-(4-Me-Ph) | |
| 4-49 | OMe | Me | 1 | CF₃ | SO₂-(Thien-2-yl) | |
| 4-50 | OMe | Et | 1 | CF₃ | SO₂-(Thien-2-yl) | |
| 4-51 | OMe | n-Pr | 1 | CF₃ | SO₂-(Thien-2-yl) | |
| 4-52 | OMe | i-Pr | 1 | CF₃ | SO₂-(Thien-2-yl) | |
| 4-53 | OMe | Me | 1 | CF₃ | CO-Ph | |
| 4-54 | OMe | Et | 1 | CF₃ | CO-Ph | |
| 4-55 | OMe | n-Pr | 1 | CF₃ | CO-Ph | |
| 4-56 | OMe | i-Pr | 1 | CF₃ | CO-Ph | |
| 4-57 | OMe | Me | 1 | CF₃ | CH₂-CO-Ph | |
| 4-58 | OMe | Et | 1 | CF₃ | CH₂-CO-Ph | |
| 4-59 | OMe | n-Pr | 1 | CF₃ | CH₂-CO-Ph | |
| 4-60 | OMe | i-Pr | 1 | CF₃ | CH₂-CO-Ph | |
| 4-61 | OMe | Me | 1 | CF₃ | CH₂-CO-(4-Me-Ph) | |
| 4-62 | OMe | Et | 1 | CF₃ | CH₂-CO-(4-Me-Ph) | |
| 4-63 | OMe | n-Pr | 1 | CF₃ | CH₂-CO-(4-Me-Ph) | |
| 4-64 | OMe | i-Pr | 1 | CF₃ | CH₂-CO-(4-Me-Ph) | |
| 4-65 | OMe | Me | 2 | CF₃ | SO₂-n-Pr | |
| 4-66 | OMe | Et | 2 | CF₃ | SO₂-n-Pr | |
| 4-67 | OMe | n-Pr | 2 | CF₃ | SO₂-n-Pr | |
| 4-68 | OMe | i-Pr | 2 | CF₃ | SO₂-n-Pr | |
| 4-69 | OMe | Me | 2 | CF₃ | SO₂-(CH₂)₂OMe | |
| 4-70 | OMe | Et | 2 | CF₃ | SO₂-(CH₂)₂OMe | |
| 4-71 | OMe | n-Pr | 2 | CF₃ | SO₂-(CH₂)₂OMe | |
| 4-72 | OMe | i-Pr | 2 | CF₃ | SO₂-(CH₂)₂OMe | |
| 4-73 | OMe | Me | 2 | CF₃ | SO₂-Ph | |
| 4-74 | OMe | Et | 2 | CF₃ | SO₂-Ph | |
| 4-75 | OMe | n-Pr | 2 | CF₃ | SO₂-Ph | |
| 4-76 | OMe | i-Pr | 2 | CF₃ | SO₂-Ph | |
| 4-77 | OMe | Me | 2 | CF₃ | SO₂-(4-Me-Ph) | |
| 4-78 | OMe | Et | 2 | CF₃ | SO₂-(4-Me-Ph) | |
| 4-79 | OMe | n-Pr | 2 | CF₃ | S0₂-(4-Me-Ph) | |
| 4-80 | OMe | i-Pr | 2 | CF₃ | SO₂-(4-Me-Ph) | |
| 4-81 | OMe | Me | 2 | CF₃ | SO₂-(Thien-2-yl) | |
| 4-82 | OMe | Et | 2 | CF₃ | SO₂-(Thien-2-yl) | |
| 4-83 | OMe | n-Pr | 2 | CF₃ | SO₂-(Thien-2-yl) | |
| 4-84 | OMe | i-Pr | 2 | CF₃ | SO₂-(Thien-2-yl) | |
| 4-85 | OMe | Me | 2 | CF₃ | CO-Ph | |
| 4-86 | OMe | Et | 2 | CF₃ | CO-Ph | |
| 4-87 | OMe | n-Pr | 2 | CF₃ | CO-Ph | |
| 4-88 | OMe | i-Pr | 2 | CF₃ | CO-Ph | |
| 4-89 | OMe | Me | 2 | CF₃ | CH₂-CO-Ph | |
| 4-90 | OMe | Et | 2 | CF₃ | CH₂-CO-Ph | |
| 4-91 | OMe | n-Pr | 2 | CF₃ | CH₂-CO-Ph | |
| 4-92 | OMe | i-Pr | 2 | CF₃ | CH₂-CO-Ph | |
| 4-93 | OMe | Me | 2 | CF₃ | CH₂-CO-(4-Me-Ph) | |
| 4-94 | OMe | Et | 2 | CF₃ | CH₂-CO-(4-Me-Ph) | |
| 4-95 | OMe | n-Pr | 2 | CF₃ | CH₂-CO-(4-Me-Ph) | |
| 4-96 | OMe | i-Pr | 2 | CF₃ | CH₂-CO-(4-Me-Ph) | |
| 4-97 | OEt | Me | 0 | CF₃ | SO₂-n-Pr | |
| 4-98 | OEt | Et | 0 | CF₃ | SO₂-n-Pr | |
| 4-99 | OEt | n-Pr | 0 | CF₃ | SO₂-n-Pr | |
| 4-100 | OEt | i-Pr | 0 | CF₃ | SO₂-n-Pr | |
| 4-101 | OEt | Me | 0 | CF₃ | SO₂-(CH₂)₂OMe | |
| 4-102 | OEt | Et | 0 | CF₃ | SO₂-(CH₂)₂OMe | |
| 4-103 | OEt | n-Pr | 0 | CF₃ | SO₂-(CH₂)₂OMe | |
| 4-104 | OEt | i-Pr | 0 | CF₃ | SO₂-(CH₂)₂OMe | |
| 4-105 | OEt | Me | 0 | CF₃ | SO₂-Ph | |
| 4-106 | OEt | Et | 0 | CF₃ | SO₂-Ph | |
| 4-107 | OEt | n-Pr | 0 | CF₃ | SO₂-Ph | |
| 4-108 | OEt | i-Pr | 0 | CF₃ | SO₂-Ph | |
| 4-109 | OEt | Me | 0 | CF₃ | SO₂-(4-Me-Ph) | |
| 4-110 | OEt | Et | 0 | CF₃ | SO₂-(4-Me-Ph) | |
| 4-111 | OEt | n-Pr | 0 | CF₃ | SO₂-(4-Me-Ph) | |
| 4-112 | OEt | i-Pr | 0 | CF₃ | SO₂-(4-Me-Ph) | |
| 4-113 | OEt | Me | 0 | CF₃ | SO₂-(Thien-2-yl) | |
| 4-114 | OEt | Et | 0 | CF₃ | SO₂-(Thien-2-yl) | |
| 4-115 | OEt | n-Pr | 0 | CF₃ | SO₂-(Thien-2-yl) | |
| 4-116 | OEt | i-Pr | 0 | CF₃ | SO₂-(Thien-2-yl) | |
| 4-117 | OEt | Me | 0 | CF₃ | CO-Ph | |
| 4-118 | OEt | Et | 0 | CF₃ | CO-Ph | |
| 4-119 | OEt | n-Pr | 0 | CF₃ | CO-Ph | |
| 4-120 | OEt | i-Pr | 0 | CF₃ | CO-Ph | |
| 4-121 | OEt | Me | 0 | CF₃ | CH₂-CO-Ph | |
| 4-122 | OEt | Et | 0 | CF₃ | CH₂-CO-Ph | |
| 4-123 | OEt | n-Pr | 0 | CF₃ | CH₂-CO-Ph | |
| 4-124 | OEt | i-Pr | 0 | CF₃ | CH₂-CO-Ph | |
| 4-125 | OEt | Me | 0 | CF₃ | CH₂-CO-(4-Me-Ph) | |
| 4-126 | OEt | Et | 0 | CF₃ | CH₂-CO-(4-Me-Ph) | |
| 4-127 | OEt | n-Pr | 0 | CF₃ | CH₂-CO-(4-Me-Ph) | |
| 4-128 | OEt | i-Pr | 0 | CF₃ | CH₂-CO-(4-Me-Ph) | |
| 4-129 | OEt | Me | 1 | CF₃ | SO₂-n-Pr | |
| 4-130 | OEt | Et | 1 | CF₃ | SO₂-n-Pr | |
| 4-131 | OEt | n-Pr | 1 | CF₃ | SO₂-n-Pr | |
| 4-132 | OEt | i-Pr | 1 | CF₃ | SO₂-n-Pr | |
| 4-133 | OEt | Me | 1 | CF₃ | SO₂-(CH₂)₂OMe | |
| 4-134 | OEt | Et | 1 | CF₃ | SO₂-(CH₂)₂OMe | |
| 4-135 | OEt | n-Pr | 1 | CF₃ | SO₂-(CH₂)₂OMe | |
| 4-136 | OEt | i-Pr | 1 | CF₃ | SO₂-(CH₂)₂OMe | |
| 4-137 | OEt | Me | 1 | CF₃ | SO₂-Ph | |
| 4-138 | OEt | Et | 1 | CF₃ | SO₂-Ph | |
| 4-139 | OEt | n-Pr | 1 | CF₃ | SO₂-Ph | |
| 4-140 | OEt | i-Pr | 1 | CF₃ | SO₂-Ph | |
| 4-141 | OEt | Me | 1 | CF₃ | SO₂-(4-Me-Ph) | |
| 4-142 | OEt | Et | 1 | CF₃ | SO₂-(4-Me-Ph) | |
| 4-143 | OEt | n-Pr | 1 | CF₃ | SO₂-(4-Me-Ph) | |
| 4-144 | OEt | i-Pr | 1 | CF₃ | SO₂-(4-Me-Ph) | |
| 4-145 | OEt | Me | 1 | CF₃ | SO₂-(Thien-2-yl) | |
| 4-146 | OEt | Et | 1 | CF₃ | SO₂-(Thien-2-yl) | |
| 4-147 | OEt | n-Pr | 1 | CF₃ | SO₂-(Thien-2-yl) | |
| 4-148 | OEt | i-Pr | 1 | CF₃ | SO₂-(Thien-2-yl) | |
| 4-149 | OEt | Me | 1 | CF₃ | CO-Ph | |
| 4-150 | OEt | Et | 1 | CF₃ | CO-Ph | |
| 4-151 | OEt | n-Pr | 1 | CF₃ | CO-Ph | |
| 4-152 | OEt | i-Pr | 1 | CF₃ | CO-Ph | |
| 4-153 | OEt | Me | 1 | CF₃ | CH₂-CO-Ph | |
| 4-154 | OEt | Et | 1 | CF₃ | CH₂-CO-Ph | |
| 4-155 | OEt | n-Pr | 1 | CF₃ | CH₂-CO-Ph | |
| 4-156 | OEt | i-Pr | 1 | CF₃ | CH₂-CO-Ph | |
| 4-157 | OEt | Me | 1 | CF₃ | CH₂-CO-(4-Me-Ph) | |
| 4-158 | OEt | Et | 1 | CF₃ | CH₂-CO-(4-Me-Ph) | |
| 4-159 | OEt | n-Pr | 1 | CF₃ | CH₂-CO-(4-Me-Ph) | |
| 4-160 | OEt | i-Pr | 1 | CF₃ | CH₂-CO-(4-Me-Ph) | |
| 4-161 | OEt | Me | 2 | CF₃ | SO₂-n-Pr | |
| 4-162 | OEt | Et | 2 | CF₃ | SO₂-n-Pr | |
| 4-163 | OEt | n-Pr | 2 | CF₃ | SO₂-n-Pr | |
| 4-164 | OEt | i-Pr | 2 | CF₃ | SO₂-n-Pr | |
| 4-165 | OEt | Me | 2 | CF₃ | SO₂-(CH₂)₂OMe | |
| 4-166 | OEt | Et | 2 | CF₃ | SO₂-(CH₂)₂OMe | |
| 4-167 | OEt | n-Pr | 2 | CF₃ | SO₂-(CH₂)₂OMe | |
| 4-168 | OEt | i-Pr | 2 | CF₃ | SO₂-(CH₂)₂OMe | |
| 4-169 | OEt | Me | 2 | CF₃ | SO₂-Ph | |
| 4-170 | OEt | Et | 2 | CF₃ | SO₂-Ph | |
| 4-171 | OEt | n-Pr | 2 | CF₃ | SO₂-Ph | |
| 4-172 | OEt | i-Pr | 2 | CF₃ | SO₂-Ph | |
| 4-173 | OEt | Me | 2 | CF₃ | SO₂-(4-Me-Ph) | |
| 4-174 | OEt | Et | 2 | CF₃ | SO₂-(4-Me-Ph) | |
| 4-175 | OEt | n-Pr | 2 | CF₃ | SO₂-(4-Me-Ph) | |
| 4-176 | OEt | i-Pr | 2 | CF₃ | SO₂-(4-Me-Ph) | |
| 4-177 | OEt | Me | 2 | CF₃ | SO₂-(Thien-2-yl) | |
| 4-178 | OEt | Et | 2 | CF₃ | SO₂-(Thien-2-yl) | |
| 4-179 | OEt | n-Pr | 2 | CF₃ | SO₂-(Thien-2-yl) | |
| 4-180 | OEt | i-Pr | 2 | CF₃ | SO₂-(Thien-2-yl) | |
| 4-181 | OEt | Me | 2 | CF₃ | CO-Ph | |
| 4-182 | OEt | Et | 2 | CF₃ | CO-Ph | |
| 4-183 | OEt | n-Pr | 2 | CF₃ | CO-Ph | |
| 4-184 | OEt | i-Pr | 2 | CF₃ | CO-Ph | |
| 4-185 | OEt | Me | 2 | CF₃ | CH₂-CO-Ph | |
| 4-186 | OEt | Et | 2 | CF₃ | CH₂-CO-Ph | |
| 4-187 | OEt | n-Pr | 2 | CF₃ | CH₂-CO-Ph | |
| 4-188 | OEt | i-Pr | 2 | CF₃ | CH₂-CO-Ph | |
| 4-189 | OEt | Me | 2 | CF₃ | CH₂-CO-(4-Me-Ph) | |
| 4-190 | OEt | Et | 2 | CF₃ | CH₂-CO-(4-Me-Ph) | |
| 4-191 | OEt | n-Pr | 2 | CF₃ | CH₂-CO-(4-Me-Ph) | |
| 4-192 | OEt | i-Pr | 2 | CF₃ | CH₂-CO-(4-Me-Ph) | |
| 4-193 | OCH₂CH₂OMe | Me | 0 | CF₃ | SO₂-n-Pr | |
| 4-194 | OCH₂CH₂OMe | Et | 0 | CF₃ | SO₂-n-Pr | |
| 4-195 | OCH₂CH₂OMe | n-Pr | 0 | CF₃ | SO₂-n-Pr | |
| 4-196 | OCH₂CH₂OMe | i-Pr | 0 | CF₃ | SO₂-n-Pr | |
| 4-197 | OCH₂CH₂OMe | Me | 0 | CF₃ | SO₂-(CH₂)₂OMe | |
| 4-198 | OCH₂CH₂OMe | Et | 0 | CF₃ | SO₂-(CH₂)₂OMe | |
| 4-199 | OCH₂CH₂OMe | n-Pr | 0 | CF₃ | SO₂-(CH₂)₂OMe | |
| 4-200 | OCH₂CH₂OMe | i-Pr | 0 | CF₃ | SO₂-(CH₂)₂OMe | |
| 4-201 | OCH₂CH₂OMe | Me | 0 | CF₃ | SO₂-Ph | |
| 4-202 | OCH₂CH₂OMe | Et | 0 | CF₃ | SO₂-Ph | |
| 4-203 | OCH₂CH₂OMe | n-Pr | 0 | CF₃ | SO₂-Ph | |
| 4-204 | OCH₂CH₂OMe | i-Pr | 0 | CF₃ | SO₂-Ph | |
| 4-205 | OCH₂CH₂OMe | Me | 0 | CF₃ | SO₂-(4-Me-Ph) | |
| 4-206 | OCH₂CH₂OMe | Et | 0 | CF₃ | SO₂-(4-Me-Ph) | |
| 4-207 | OCH₂CH₂OMe | n-Pr | 0 | CF₃ | SO₂-(4-Me-Ph) | |
| 4-208 | OCH₂CH₂OMe | i-Pr | 0 | CF₃ | SO₂-(4-Me-Ph) | |
| 4-209 | OCH₂CH₂OMe | Me | 0 | CF₃ | SO₂-(Thien-2-yl) | |
| 4-210 | OCH₂CH₂OMe | Et | 0 | CF₃ | SO₂-(Thien-2-yl) | |
| 4-211 | OCH₂CH₂OMe | n-Pr | 0 | CF₃ | SO₂-(Thien-2-yl) | |
| 4-212 | OCH₂CH₂OMe | i-Pr | 0 | CF₃ | SO₂-(Thien-2-yl) | |
| 4-213 | OCH₂CH₂OMe | Me | 0 | CF₃ | CO-Ph | |
| 4-214 | OCH₂CH₂OMe | Et | 0 | CF₃ | CO-Ph | |
| 4-215 | OCH₂CH₂OMe | n-Pr | 0 | CF₃ | CO-Ph | |
| 4-216 | OCH₂CH₂OMe | i-Pr | 0 | CF₃ | CO-Ph | |
| 4-217 | OCH₂CH₂OMe | Me | 0 | CF₃ | CH₂-CO-Ph | |
| 4-218 | OCH₂CH₂OMe | Et | 0 | CF₃ | CH₂-CO-Ph | |
| 4-219 | OCH₂CH₂OMe | n-Pr | 0 | CF₃ | CH₂-CO-Ph | |
| 4-220 | OCH₂CH₂OMe | i-Pr | 0 | CF₃ | CH₂-CO-Ph | |
| 4-221 | OCH₂CH₂OMe | Me | 0 | CF₃ | CH₂-CO-(4-Me-Ph) | |
| 4-222 | OCH₂CH₂OMe | Et | 0 | CF₃ | CH₂-CO-(4-Me-Ph) | |
| 4-223 | OCH₂CH₂OMe | n-Pr | 0 | CF₃ | CH₂-CO-(4-Me-Ph) | |
| 4-224 | OCH₂CH₂OMe | i-Pr | 0 | CF₃ | CH₂-CO-(4-Me-Ph) | |
| 4-225 | OCH₂CH₂OMe | Me | 1 | CF₃ | SO₂-n-Pr | |
| 4-226 | OCH₂CH₂OMe | Et | 1 | CF₃ | SO₂-n-Pr | |
| 4-227 | OCH₂CH₂OMe | n-Pr | 1 | CF₃ | SO₂-n-Pr | |
| 4-228 | OCH₂CH₂OMe | i-Pr | 1 | CF₃ | SO₂-n-Pr | |
| 4-229 | OCH₂CH₂OMe | Me | 1 | CF₃ | SO₂-(CH₂)₂OMe | |
| 4-230 | OCH₂CH₂OMe | Et | 1 | CF₃ | SO₂-(CH₂)₂OMe | |
| 4-231 | OCH₂CH₂OMe | n-Pr | 1 | CF₃ | SO₂-(CH₂)₂OMe | |
| 4-232 | OCH₂CH₂OMe | i-Pr | 1 | CF₃ | SO₂-(CH₂)₂OMe | |
| 4-233 | OCH₂CH₂OMe | Me | 1 | CF₃ | SO₂-Ph | |
| 4-234 | OCH₂CH₂OMe | Et | 1 | CF₃ | SO₂-Ph | |
| 4-235 | OCH₂CH₂OMe | n-Pr | 1 | CF₃ | SO₂-Ph | |
| 4-236 | OCH₂CH₂OMe | i-Pr | 1 | CF₃ | SO₂-Ph | |
| 4-237 | OCH₂CH₂OMe | Me | 1 | CF₃ | SO₂-(4-Me-Ph) | |
| 4-238 | OCH₂CH₂OMe | Et | 1 | CF₃ | SO₂-(4-Me-Ph) | |
| 4-239 | OCH₂CH₂OMe | n-Pr | 1 | CF₃ | SO₂-(4-Me-Ph) | |
| 4-240 | OCH₂CH₂OMe | i-Pr | 1 | CF₃ | SO₂-(4-Me-Ph) | |
| 4-241 | OCH₂CH₂OMe | Me | 1 | CF₃ | SO₂-(Thien-2-yl) | |
| 4-242 | OCH₂CH₂OMe | Et | 1 | CF₃ | SO₂-(Thien-2-yl) | |
| 4-243 | OCH₂CH₂OMe | n-Pr | 1 | CF₃ | SO₂-(Thien-2-yl) | |
| 4-244 | OCH₂CH₂OMe | i-Pr | 1 | CF₃ | SO₂-(Thien-2-yl) | |
| 4-245 | OCH₂CH₂OMe | Me | 1 | CF₃ | CO-Ph | |
| 4-246 | OCH₂CH₂OMe | Et | 1 | CF₃ | CO-Ph | |
| 4-247 | OCH₂CH₂OMe | n-Pr | 1 | CF₃ | CO-Ph | |
| 4-248 | OCH₂CH₂OMe | i-Pr | 1 | CF₃ | CO-Ph | |
| 4-249 | OCH₂CH₂OMe | Me | 1 | CF₃ | CH₂-CO-Ph | |
| 4-250 | OCH₂CH₂OMe | Et | 1 | CF₃ | CH₂-CO-Ph | |
| 4-251 | OCH₂CH₂OMe | n-Pr | 1 | CF₃ | CH₂-CO-Ph | |
| 4-252 | OCH₂CH₂OMe | i-Pr | 1 | CF₃ | CH₂-CO-Ph | |
| 4-253 | OCH₂CH₂OMe | Me | 1 | CF₃ | CH₂-CO-(4-Me-Ph) | |
| 4-254 | OCH₂CH₂OMe | Et | 1 | CF₃ | CH₂-CO-(4-Me-Ph) | |
| 4-255 | OCH₂CH₂OMe | n-Pr | 1 | CF₃ | CH₂-CO-(4-Me-Ph) | |
| 4-256 | OCH₂CH₂OMe | i-Pr | 1 | CF₃ | CH₂-CO-(4-Me-Ph) | |
| 4-257 | OCH₂CH₂OMe | Me | 2 | CF₃ | SO₂-n-Pr | |
| 4-258 | OCH₂CH₂OMe | Et | 2 | CF₃ | SO₂-n-Pr | |
| 4-259 | OCH₂CH₂OMe | n-Pr | 2 | CF₃ | SO₂-n-Pr | |
| 4-260 | OCH₂CH₂OMe | i-Pr | 2 | CF₃ | SO₂-n-Pr | |
| 4-261 | OCH₂CH₂OMe | Me | 2 | CF₃ | SO₂-(CH₂)₂OMe | |
| 4-262 | OCH₂CH₂OMe | Et | 2 | CF₃ | SO₂-(CH₂)₂OMe | |
| 4-263 | OCH₂CH₂OMe | n-Pr | 2 | CF₃ | SO₂-(CH₂)₂OMe | |
| 4-264 | OCH₂CH₂OMe | i-Pr | 2 | CF₃ | SO₂-(CH₂)₂OMe | |
| 4-265 | OCH₂CH₂OMe | Me | 2 | CF₃ | SO₂-Ph | |
| 4-266 | OCH₂CH₂OMe | Et | 2 | CF₃ | SO₂-Ph | |
| 4-267 | OCH₂CH₂OMe | n-Pr | 2 | CF₃ | SO₂-Ph | |
| 4-268 | OCH₂CH₂OMe | i-Pr | 2 | CF₃ | SO₂-Ph | |
| 4-269 | OCH₂CH₂OMe | Me | 2 | CF₃ | SO₂-(4-Me-Ph) | |
| 4-270 | OCH₂CH₂OMe | Et | 2 | CF₃ | SO₂-(4-Me-Ph) | |
| 4-271 | OCH₂CH₂OMe | n-Pr | 2 | CF₃ | SO₂-(4-Me-Ph) | |
| 4-272 | OCH₂CH₂OMe | i-Pr | 2 | CF₃ | SO₂-(4-Me-Ph) | |
| 4-273 | OCH₂CH₂OMe | Me | 2 | CF₃ | SO₂-(Thien-2-yl) | |
| 4-274 | OCH₂CH₂OMe | Et | 2 | CF₃ | SO₂-(Thien-2-yl) | |
| 4-275 | OCH₂CH₂OMe | n-Pr | 2 | CF₃ | SO₂-(Thien-2-yl) | |
| 4-276 | OCH₂CH₂OMe | i-Pr | 2 | CF₃ | SO₂-(Thien-2-yl) | |
| 4-277 | OCH₂CH₂OMe | Me | 2 | CF₃ | CO-Ph | |
| 4-278 | OCH₂CH₂OMe | Et | 2 | CF₃ | CO-Ph | |
| 4-279 | OCH₂CH₂OMe | n-Pr | 2 | CF₃ | CO-Ph | |
| 4-280 | OCH₂CH₂OMe | i-Pr | 2 | CF₃ | CO-Ph | |
| 4-281 | OCH₂CH₂OMe | Me | 2 | CF₃ | CH₂-CO-Ph | |
| 4-282 | OCH₂CH₂OMe | Et | 2 | CF₃ | CH₂-CO-Ph | |
| 4-283 | OCH₂CH₂OMe | n-Pr | 2 | CF₃ | CH₂-CO-Ph | |
| 4-284 | OCH₂CH₂OMe | i-Pr | 2 | CF₃ | CH₂-CO-Ph | |
| 4-285 | OCH₂CH₂OMe | Me | 2 | CF₃ | CH₂-CO-(4-Me-Ph) | |
| 4-286 | OCH₂CH₂OMe | Et | 2 | CF₃ | CH₂-CO-(4-Me-Ph) | |
| 4-287 | OCH₂CH₂OMe | n-Pr | 2 | CF₃ | CH₂-CO-(4-Me-Ph) | |
| 4-288 | OCH₂CH₂OMe | i-Pr | 2 | CF₃ | CH₂-CO-(4-Me-Ph) | |

**Tabelle 5: Erfindungsgemäße Verbindungen der allgemeinen Formel (I), worin R¹ für Ethyl und R² für Wasserstoff steht.**

| | | | | | | |
|---|---|---|---|---|---|---|
| 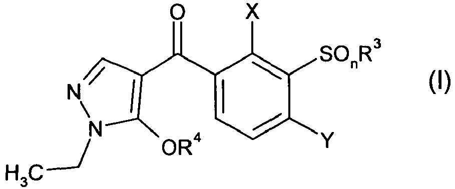 | | | | | | |

| Nr. | X | R³ | n | Y | R⁴ | Physikalische Daten: ¹H-NMR: δ [CDCl₃] |
|---|---|---|---|---|---|---|
| 5-1 | OMe | Me | 0 | CF₃ | SO₂-n-Pr | |
| 5-2 | OMe | Et | 0 | CF₃ | SO₂-n-Pr | |
| 5-3 | OMe | n-Pr | 0 | CF₃ | SO₂-n-Pr | |
| 5-4 | OMe | i-Pr | 0 | CF₃ | SO₂-n-Pr | |
| 5-5 | OMe | Me | 0 | CF₃ | SO₂-(CH₂)₂OMe | |
| 5-6 | OMe | Et | 0 | CF₃ | SO₂-(CH₂)₂OMe | |
| 5-7 | OMe | n-Pr | 0 | CF₃ | SO₂-(CH₂)₂OMe | |
| 5-8 | OMe | i-Pr | 0 | CF₃ | SO₂-(CH₂)₂OMe | |
| 5-9 | OMe | Me | 0 | CF₃ | SO₂-Ph | |
| 5-10 | OMe | Et | 0 | CF₃ | SO₂-Ph | |
| 5-11 | OMe | n-Pr | 0 | CF₃ | SO₂-Ph | |
| 5-12 | OMe | i-Pr | 0 | CF₃ | SO₂-Ph | |
| 5-13 | OMe | Me | 0 | CF₃ | SO₂-(4-Me-Ph) | |
| 5-14 | OMe | Et | 0 | CF₃ | SO₂-(4-Me-Ph) | |
| 5-15 | OMe | n-Pr | 0 | CF₃ | SO₂-(4-Me-Ph) | |
| 5-16 | OMe | i-Pr | 0 | CF₃ | SO₂-(4-Me-Ph) | |
| 5-17 | OMe | Me | 0 | CF₃ | SO₂-(Thien-2-yl) | |
| 5-18 | OMe | Et | 0 | CF₃ | SO₂-(Thien-2-yl) | |
| 5-19 | OMe | n-Pr | 0 | CF₃ | SO₂-(Thien-2-yl) | |
| 5-20 | OMe | i-Pr | 0 | CF₃ | SO₂-(Thien-2-yl) | |
| 5-21 | OMe | Me | 0 | CF₃ | CO-Ph | |
| 5-22 | OMe | Et | 0 | CF₃ | CO-Ph | |
| 5-23 | OMe | n-Pr | 0 | CF₃ | CO-Ph | |
| 5-24 | OMe | i-Pr | 0 | CF₃ | CO-Ph | |
| 5-25 | OMe | Me | 0 | CF₃ | CH₂-CO-Ph | |
| 5-26 | OMe | Et | 0 | CF₃ | CH₂-CO-Ph | |
| 5-27 | OMe | n-Pr | 0 | CF₃ | CH₂-CO-Ph | |
| 5-28 | OMe | i-Pr | 0 | CF₃ | CH₂-CO-Ph | |
| 5-29 | OMe | Me | 0 | CF₃ | CH₂-CO-(4-Me-Ph) | |
| 5-30 | OMe | Et | 0 | CF₃ | CH₂-CO-(4-Me-Ph) | |
| 5-31 | OMe | n-Pr | 0 | CF₃ | CH₂-CO-(4-Me-Ph) | |
| 5-32 | OMe | i-Pr | 0 | CF₃ | CH₂-CO-(4-Me-Ph) | |
| 5-33 | OMe | Me | 1 | CF₃ | SO₂-n-Pr | |
| 5-34 | OMe | Et | 1 | CF₃ | SO₂-n-Pr | |
| 5-35 | OMe | n-Pr | 1 | CF₃ | SO₂-n-Pr | |
| 5-36 | OMe | i-Pr | 1 | CF₃ | SO₂-n-Pr | |
| 5-37 | OMe | Me | 1 | CF₃ | SO₂-(CH₂)₂OMe | |
| 5-38 | OMe | Et | 1 | CF₃ | SO₂-(CH₂)₂OMe | |
| 5-39 | OMe | n-Pr | 1 | CF₃ | SO₂-(CH₂)₂OMe | |
| 5-40 | OMe | i-Pr | 1 | CF₃ | SO₂-(CH₂)₂OMe | |
| 5-41 | OMe | Me | 1 | CF₃ | SO₂-Ph | |
| 5-42 | OMe | Et | 1 | CF₃ | SO₂-Ph | |
| 5-43 | OMe | n-Pr | 1 | CF₃ | SO₂-Ph | |
| 5-44 | OMe | i-Pr | 1 | CF₃ | SO₂-Ph | |
| 5-45 | OMe | Me | 1 | CF₃ | SO₂-(4-Me-Ph) | |
| 5-46 | OMe | Et | 1 | CF₃ | SO₂-(4-Me-Ph) | |
| 5-47 | OMe | n-Pr | 1 | CF₃ | SO₂-(4-Me-Ph) | |
| 5-48 | OMe | i-Pr | 1 | CF₃ | SO₂-(4-Me-Ph) | |
| 5-49 | OMe | Me | 1 | CF₃ | SO₂-(Thien-2-yl) | |
| 5-50 | OMe | Et | 1 | CF₃ | SO₂-(Thien-2-yl) | |
| 5-51 | OMe | n-Pr | 1 | CF₃ | SO₂-(Thien-2-yl) | |
| 5-52 | OMe | i-Pr | 1 | CF₃ | SO₂-(Thien-2-yl) | |
| 5-53 | OMe | Me | 1 | CF₃ | CO-Ph | |
| 5-54 | OMe | Et | 1 | CF₃ | CO-Ph | |
| 5-55 | OMe | n-Pr | 1 | CF₃ | CO-Ph | |
| 5-56 | OMe | i-Pr | 1 | CF₃ | CO-Ph | |
| 5-57 | OMe | Me | 1 | CF₃ | CH₂-CO-Ph | |
| 5-58 | OMe | Et | 1 | CF₃ | CH₂-CO-Ph | |
| 5-59 | OMe | n-Pr | 1 | CF₃ | CH₂-CO-Ph | |
| 5-60 | OMe | i-Pr | 1 | CF₃ | CH₂-CO-Ph | |
| 5-61 | OMe | Me | 1 | CF₃ | CH₂-CO-(4-Me-Ph) | |
| 5-62 | OMe | Et | 1 | CF₃ | CH₂-CO-(4-Me-Ph) | |
| 5-63 | OMe | n-Pr | 1 | CF₃ | CH₂-CO-(4-Me-Ph) | |
| 5-64 | OMe | i-Pr | 1 | CF₃ | CH₂-CO-(4-Me-Ph) | |
| 5-65 | OMe | Me | 2 | CF₃ | SO₂-n-Pr | |
| 5-66 | OMe | Et | 2 | CF₃ | SO₂-n-Pr | |
| 5-67 | OMe | n-Pr | 2 | CF₃ | SO₂-n-Pr | |
| 5-68 | OMe | i-Pr | 2 | CF₃ | SO₂-n-Pr | |
| 5-69 | OMe | Me | 2 | CF₃ | SO₂-(CH₂)₂OMe | |
| 5-70 | OMe | Et | 2 | CF₃ | SO₂-(CH₂)₂OMe | |
| 5-71 | OMe | n-Pr | 2 | CF₃ | SO₂-(CH₂)₂OMe | |
| 5-72 | OMe | i-Pr | 2 | CF₃ | SO₂-(CH₂)₂OMe | |
| 5-73 | OMe | Me | 2 | CF₃ | SO₂-Ph | |
| 5-74 | OMe | Et | 2 | CF₃ | SO₂-Ph | |
| 5-75 | OMe | n-Pr | 2 | CF₃ | S0₂-Ph | |
| 5-76 | OMe | i-Pr | 2 | CF₃ | SO₂-Ph | |
| 5-77 | OMe | Me | 2 | CF₃ | S0₂-(4-Me-Ph) | |
| 5-78 | OMe | Et | 2 | CF₃ | S0₂-(4-Me-Ph) | |
| 5-79 | OMe | n-Pr | 2 | CF₃ | SO₂-(4-Me-Ph) | |
| 5-80 | OMe | i-Pr | 2 | CF₃ | SO₂-(4-Me-Ph) | |
| 5-81 | OMe | Me | 2 | CF₃ | SO₂-(Thien-2-yl) | |
| 5-82 | OMe | Et | 2 | CF₃ | SO₂-(Thien-2-yl) | |
| 5-83 | OMe | n-Pr | 2 | CF₃ | SO₂-(Thien-2-yl) | |
| 5-84 | OMe | i-Pr | 2 | CF₃ | SO₂-(Thien-2-yl) | |
| 5-85 | OMe | Me | 2 | CF₃ | CO-Ph | |
| 5-86 | OMe | Et | 2 | CF₃ | CO-Ph | |
| 5-87 | OMe | n-Pr | 2 | CF₃ | CO-Ph | |
| 5-88 | OMe | i-Pr | 2 | CF₃ | CO-Ph | |
| 5-89 | OMe | Me | 2 | CF₃ | CH₂-CO-Ph | |
| 5-90 | OMe | Et | 2 | CF₃ | CH₂-CO-Ph | |
| 5-91 | OMe | n-Pr | 2 | CF₃ | CH₂-CO-Ph | |
| 5-92 | OMe | i-Pr | 2 | CF₃ | CH₂-CO-Ph | |
| 5-93 | OMe | Me | 2 | CF₃ | CH₂-CO-(4-Me-Ph) | |
| 5-94 | OMe | Et | 2 | CF₃ | CH₂-CO-(4-Me-Ph) | |
| 5-95 | OMe | n-Pr | 2 | CF₃ | CH₂-CO-(4-Me-Ph) | |
| 5-96 | OMe | i-Pr | 2 | CF₃ | CH₂-CO-(4-Me-Ph) | |
| 5-97 | OEt | Me | 0 | CF₃ | SO₂-n-Pr | |
| 5-98 | OEt | Et | 0 | CF₃ | SO₂-n-Pr | |
| 5-99 | OEt | n-Pr | 0 | CF₃ | SO₂-n-Pr | |
| 5-100 | OEt | i-Pr | 0 | CF₃ | SO₂-n-Pr | |
| 5-101 | OEt | Me | 0 | CF₃ | SO₂-(CH₂)₂OMe | |
| 5-102 | OEt | Et | 0 | CF₃ | SO₂-(CH₂)₂OMe | |
| 5-103 | OEt | n-Pr | 0 | CF₃ | SO₂-(CH₂)₂OMe | |
| 5-104 | OEt | i-Pr | 0 | CF₃ | SO₂-(CH₂)₂OMe | |
| 5-105 | OEt | Me | 0 | CF₃ | SO₂-Ph | |
| 5-106 | OEt | Et | 0 | CF₃ | SO₂-Ph | |
| 5-107 | OEt | n-Pr | 0 | CF₃ | SO₂-Ph | |
| 5-108 | OEt | i-Pr | 0 | CF₃ | SO₂-Ph | |
| 5-109 | OEt | Me | 0 | CF₃ | SO₂-(4-Me-Ph) | |
| 5-110 | OEt | Et | 0 | CF₃ | SO₂-(4-Me-Ph) | |
| 5-111 | OEt | n-Pr | 0 | CF₃ | SO₂-(4-Me-Ph) | |
| 5-112 | OEt | i-Pr | 0 | CF₃ | SO₂-(4-Me-Ph) | |
| 5-113 | OEt | Me | 0 | CF₃ | SO₂-(Thien-2-yl) | |
| 5-114 | OEt | Et | 0 | CF₃ | SO₂-(Thien-2-yl) | |
| 5-115 | OEt | n-Pr | 0 | CF₃ | SO₂-(Thien-2-yl) | |
| 5-116 | OEt | i-Pr | 0 | CF₃ | SO₂-(Thien-2-yl) | |
| 5-117 | OEt | Me | 0 | CF₃ | CO-Ph | |
| 5-118 | OEt | Et | 0 | CF₃ | CO-Ph | |
| 5-119 | OEt | n-Pr | 0 | CF₃ | CO-Ph | |
| 5-120 | OEt | i-Pr | 0 | CF₃ | CO-Ph | |
| 5-121 | OEt | Me | 0 | CF₃ | CH₂-CO-Ph | |
| 5-122 | OEt | Et | 0 | CF₃ | CH₂-CO-Ph | |
| 5-123 | OEt | n-Pr | 0 | CF₃ | CH₂-CO-Ph | |
| 5-124 | OEt | i-Pr | 0 | CF₃ | CH₂-CO-Ph | |
| 5-125 | OEt | Me | 0 | CF₃ | CH₂-CO-(4-Me-Ph) | |
| 5-126 | OEt | Et | 0 | CF₃ | CH₂-CO-(4-Me-Ph) | |
| 5-127 | OEt | n-Pr | 0 | CF₃ | CH₂-CO-(4-Me-Ph) | |

| Nr. | X | R³ | n | Y | R⁴ | Physikalische Daten: ¹H-NMR: δ [CDCl]₃] |
|---|---|---|---|---|---|---|
| 5-128 | OEt | i-Pr | 0 | CF₃ | CH₂-CO-(4-Me-Ph) | |
| 5-129 | OEt | Me | 1 | CF₃ | SO₂-n-Pr | |
| 5-130 | OEt | Et | 1 | CF₃ | SO₂-n-Pr | |
| 5-131 | OEt | n-Pr | 1 | CF₃ | SO₂-n-Pr | |
| 5-132 | OEt | i-Pr | 1 | CF₃ | SO₂-n-Pr | |
| 5-133 | OEt | Me | 1 | CF₃ | SO₂-(CH₂)₂OMe | |
| 5-134 | OEt | Et | 1 | CF₃ | SO₂-(CH₂)₂OMe | |
| 5-135 | OEt | n-Pr | 1 | CF₃ | SO₂-(CH₂)₂OMe | |
| 5-136 | OEt | i-Pr | 1 | CF₃ | SO₂-(CH₂)₂OMe | |
| 5-137 | OEt | Me | 1 | CF₃ | SO₂-Ph | |
| 5-138 | OEt | Et | 1 | CF₃ | SO₂-Ph | |
| 5-139 | OEt | n-Pr | 1 | CF₃ | SO₂-Ph | |
| 5-140 | OEt | i-Pr | 1 | CF₃ | SO₂-Ph | |
| 5-141 | OEt | Me | 1 | CF₃ | SO₂-(4-Me-Ph) | |
| 5-142 | OEt | Et | 1 | CF₃ | SO₂-(4-Me-Ph) | |
| 5-143 | OEt | n-Pr | 1 | CF₃ | SO₂-(4-Me-Ph) | |
| 5-144 | OEt | i-Pr | 1 | CF₃ | SO₂-(4-Me-Ph) | |
| 5-145 | OEt | Me | 1 | CF₃ | SO₂-(Thien-2-yl) | |
| 5-146 | OEt | Et | 1 | CF₃ | SO₂-(Thien-2-yl) | |
| 5-147 | OEt | n-Pr | 1 | CF₃ | SO₂-(Thien-2-yl) | |
| 5-148 | OEt | i-Pr | 1 | CF₃ | SO₂-(Thien-2-yl) | |
| 5-149 | OEt | Me | 1 | CF₃ | CO-Ph | |
| 5-150 | OEt | Et | 1 | CF₃ | CO-Ph | |
| 5-151 | OEt | n-Pr | 1 | CF₃ | CO-Ph | |
| 5-152 | OEt | i-Pr | 1 | CF₃ | CO-Ph | |
| 5-153 | OEt | Me | 1 | CF₃ | CH₂-CO-Ph | |
| 5-154 | OEt | Et | 1 | CF₃ | CH₂-CO-Ph | |
| 5-155 | OEt | n-Pr | 1 | CF₃ | CH₂-CO-Ph | |
| 5-156 | OEt | i-Pr | 1 | CF₃ | CH₂-CO-Ph | |
| 5-157 | OEt | Me | 1 | CF₃ | CH₂-CO-(4-Me-Ph) | |
| 5-158 | OEt | Et | 1 | CF₃ | CH₂-CO-(4-Me-Ph) | |
| 5-159 | OEt | n-Pr | 1 | CF₃ | CH₂-CO-(4-Me-Ph) | |
| 5-160 | OEt | i-Pr | 1 | CF₃ | CH₂-CO-(4-Me-Ph) | |
| 5-161 | OEt | Me | 2 | CF₃ | SO₂-n-Pr | |

| Nr. | X | R³ | n | Y | R⁴ | Physikalische Daten: ¹H-NMR: δ [CDCl₃] |
|---|---|---|---|---|---|---|
| 5-162 | OEt | Et | 2 | CF₃ | SO₂-n-Pr | |
| 5-163 | OEt | n-Pr | 2 | CF₃ | SO₂-n-Pr | |
| 5-164 | OEt | i-Pr | 2 | CF₃ | SO₂-n-Pr | |
| 5-165 | OEt | Me | 2 | CF₃ | SO₂-(CH₂)₂OMe | |
| 5-166 | OEt | Et | 2 | CF₃ | SO₂-(CH₂)₂OMe | |
| 5-167 | OEt | n-Pr | 2 | CF₃ | SO₂-(CH₂)₂OMe | |
| 5-168 | OEt | i-Pr | 2 | CF₃ | SO₂-(CH₂)₂OMe | |
| 5-169 | OEt | Me | 2 | CF₃ | SO₂-Ph | |
| 5-170 | OEt | Et | 2 | CF₃ | SO₂-Ph | |
| 5-171 | OEt | n-Pr | 2 | CF₃ | SO₂-Ph | |
| 5-172 | OEt | i-Pr | 2 | CF₃ | SO₂-Ph | |
| 5-173 | OEt | Me | 2 | CF₃ | SO₂-(4-Me-Ph) | |
| 5-174 | OEt | Et | 2 | CF₃ | SO₂-(4-Me-Ph) | |
| 5-175 | OEt | n-Pr | 2 | CF₃ | SO₂-(4-Me-Ph) | |
| 5-176 | OEt | i-Pr | 2 | CF₃ | SO₂-(4-Me-Ph) | |
| 5-177 | OEt | Me | 2 | CF₃ | SO₂-(Thien-2-yl) | |
| 5-178 | OEt | Et | 2 | CF₃ | SO₂-(Thien-2-yl) | |
| 5-179 | OEt | n-Pr | 2 | CF₃ | SO₂-(Thien-2-yl) | |
| 5-180 | OEt | i-Pr | 2 | CF₃ | SO₂-(Thien-2-yl) | |
| 5-181 | OEt | Me | 2 | CF₃ | CO-Ph | |
| 5-182 | OEt | Et | 2 | CF₃ | CO-Ph | |
| 5-183 | OEt | n-Pr | 2 | CF₃ | CO-Ph | |
| 5-184 | OEt | i-Pr | 2 | CF₃ | CO-Ph | |
| 5-185 | OEt | Me | 2 | CF₃ | CH₂-CO-Ph | |
| 5-186 | OEt | Et | 2 | CF₃ | CH₂-CO-Ph | |
| 5-187 | OEt | n-Pr | 2 | CF₃ | CH₂-CO-Ph | |
| 5-188 | OEt | i-Pr | 2 | CF₃ | CH₂-CO-Ph | |
| 5-189 | OEt | Me | 2 | CF₃ | CH₂-CO-(4-Me-Ph) | |
| 5-190 | OEt | Et | 2 | CF₃ | CH₂-CO-(4-Me-Ph) | |
| 5-191 | OEt | n-Pr | 2 | CF₃ | CH₂-CO-(4-Me-Ph) | |
| 5-192 | OEt | i-Pr | 2 | CF₃ | CH₂-CO-(4-Me-Ph) | |
| 5-193 | OCH₂CH₂OMe | Me | 0 | CF₃ | SO₂-n-Pr | |
| 5-194 | OCH₂CH₂OMe | Et | 0 | CF₃ | SO₂-n-Pr | |
| 5-195 | OCH₂CH₂OMe | n-Pr | 0 | CF₃ | SO₂-n-Pr | |
| 5-196 | OCH₂CH₂OMe | i-Pr | 0 | CF₃ | SO₂-n-Pr | |
| 5-197 | OCH₂CH₂OMe | Me | 0 | CF₃ | SO₂-(CH₂)₂OMe | |
| 5-198 | OCH₂CH₂OMe | Et | 0 | CF₃ | SO₂-(CH₂)₂OMe | |
| 5-199 | OCH₂CH₂OMe | n-Pr | 0 | CF₃ | SO₂-(CH₂)₂OMe | |
| 5-200 | OCH₂CH₂OMe | i-Pr | 0 | CF₃ | SO₂-(CH₂)₂OMe | |
| 5-201 | OCH₂CH₂OMe | Me | 0 | CF₃ | SO₂-Ph | |
| 5-202 | OCH₂CH₂OMe | Et | 0 | CF₃ | SO₂-Ph | |
| 5-203 | OCH₂CH₂OMe | n-Pr | 0 | CF₃ | SO₂-Ph | |
| 5-204 | OCH₂CH₂OMe | i-Pr | 0 | CF₃ | SO₂-Ph | |
| 5-205 | OCH₂CH₂OMe | Me | 0 | CF₃ | SO₂-(4-Me-Ph) | |
| 5-206 | OCH₂CH₂OMe | Et | 0 | CF₃ | SO₂-(4-Me-Ph) | |
| 5-207 | OCH₂CH₂OMe | n-Pr | 0 | CF₃ | SO₂-(4-Me-Ph) | |
| 5-208 | OCH₂CH₂OMe | i-Pr | 0 | CF₃ | SO₂-(4-Me-Ph) | |
| 5-209 | OCH₂CH₂OMe | Me | 0 | CF₃ | SO₂-(Thien-2-yl) | |
| 5-210 | OCH₂CH₂OMe | Et | 0 | CF₃ | SO₂-(Thien-2-yl) | |
| 5-211 . | OCH₂CH₂OMe | n-Pr | 0 | CF₃ | SO₂-(Thien-2-yl) | |
| 5-212 | OCH₂CH₂OMe | i-Pr | 0 | CF₃ | SO₂-(Thien-2-yl) | |
| 5-213 | OCH₂CH₂OMe | Me | 0 | CF₃ | CO-Ph | |
| 5-214 | OCH₂CH₂OMe | Et | 0 | CF₃ | CO-Ph | |
| 5-215 | OCH₂CH₂OMe | n-Pr | 0 | CF₃ | CO-Ph | |
| 5-216 | OCH₂CH₂OMe | i-Pr | 0 | CF₃ | CO-Ph | |
| 5-217 | OCH₂CH₂OMe | Me | 0 | CF₃ | CH₂-CO-Ph | |
| 5-218 | OCH₂CH₂OMe | Et | 0 | CF₃ | CH₂-CO-Ph | |
| 5-219 | OCH₂CH₂OMe | n-Pr | 0 | CF₃ | CH₂-CO-Ph | |
| 5-220 | OCH₂CH₂OMe | i-Pr | 0 | CF₃ | CH₂-CO-Ph | |
| 5-221 | OCH₂CH₂OMe | Me | 0 | CF₃ | CH₂-CO-(4-Me-Ph) | |
| 5-222 | OCH₂CH₂OMe | Et | 0 | CF₃ | CH₂-CO-(4-Me-Ph) | |
| 5-223 | OCH₂CH₂OMe | n-Pr | 0 | CF₃ | CH₂-CO-(4-Me-Ph) | |
| 5-224 | OCH₂CH₂OMe | i-Pr | 0 | CF₃ | CH₂-CO-(4-Me-Ph) | |
| 5-225 | OCH₂CH₂OMe | Me | 1 | CF₃ | SO₂-n-Pr | |
| 5-226 | OCH₂CH₂OMe | Et | 1 | CH₃ | SO₂-n-Pr | |
| 5-227 | OCH₂CH₂OMe | n-Pr | 1 | CF₃ | SO₂-n-Pr | |
| 5-228 | OCH₂CH₂OMe | i-Pr | 1 | CF₃ | SO₂-n-Pr | |
| 5-229 | OCH₂CH₂OMe | Me | 1 | CF₃ | SO₂-(CH₂)₂OMe | |
| 5-230 | OCH₂CH₂OMe | Et | 1 | CF₃ | SO₂-(CH₂)₂OMe | |
| 5-231 | OCH₂CH₂OMe | n-Pr | 1 | CF₃ | SO₂-(CH₂)₂OMe | |
| 5-232 | OCH₂CH₂OMe | i-Pr | 1 | CF₃ | SO₂-(CH₂)₂OMe | |
| 5-233 | OCH₂CH₂OMe | Me | 1 | CF₃ | SO₂-Ph | |
| 5-234 | OCH₂CH₂OMe | Et | 1 | CF₃ | SO₂-Ph | |
| 5-235 | OCH₂CH₂OMe | n-Pr | 1 | CF₃ | SO₂-Ph | |
| 5-236 | OCH₂CH₂OMe | i-Pr | 1 | CF₃ | SO₂-Ph | |
| 5-237 | OCH₂CH₂OMe | Me | 1 | CF₃ | SO₂-(4-Me-Ph) | |
| 5-238 | OCH₂CH₂OMe | Et | 1 | CF₃ | SO₂-(4-Me-Ph) | |
| 5-239 | OCH₂CH₂OMe | n-Pr | 1 | CF₃ | SO₂-(4-Me-Ph) | |
| 5-240 | OCH₂CH₂OMe | i-Pr | 1 | CF₃ | SO₂-(4-Me-Ph) | |
| 5-241 | OCH₂CH₂OMe | Me | 1 | CF₃ | SO₂-(Thien-2-yl) | |
| 5-242 | OCH₂CH₂OMe | Et | 1 | CF₃ | SO₂-(Thien-2-yl) | |
| 5-243 | OCH₂CH₂OMe | n-Pr | 1 | CF₃ | SO₂-(Thien-2-yl) | |
| 5-244 | OCH₂CH₂OMe | i-Pr | 1 | CF₃ | SO₂-(Thien-2-yl) | |
| 5-245 | OCH₂CH₂OMe | Me | 1 | CF₃ | CO-Ph | |
| 5-246 | OCH₂CH₂OMe | Et | 1 | CF₃ | CO-Ph | |
| 5-247 | OCH₂CH₂OMe | n-Pr | 1 | CF₃ | CO-Ph | |
| 5-248 | OCH₂CH₂OMe | i-Pr | 1 | CF₃ | CO-Ph | |
| 5-249 | OCH₂CH₂OMe | Me | 1 | CF₃ | CH₂-CO-Ph | |
| 5-250 | OCH₂CH₂OMe | Et | 1 | CF₃ | CH₂-CO-Ph | |
| 5-251 | OCH₂CH₂OMe | n-Pr | 1 | CF₃ | CH₂-CO-Ph | |
| 5-252 | OCH₂CH₂OMe | i-Pr | 1 | CF₃ | CH₂-CO-Ph | |
| 5-253 | OCH₂CH₂OMe | Me | 1 | CF₃ | CH₂-CO-(4-Me-Ph) | |
| 5-254 | OCH₂CH₂OMe | Et | 1 | CF₃ | CH₂-CO-(4-Me-Ph) | |
| 5-255 | OCH₂CH₂OMe | n-Pr | 1 | CF₃ | CH₂-CO-(4-Me-Ph) | |
| 5-256 | OCH₂CH₂OMe | i-Pr | 1 | CF₃ | CH₂-CO-(4-Me-Ph) | |
| 5-257 | OCH₂CH₂OMe | Me | 2 | CF₃ | SO₂-n-Pr | |
| 5-258 | OCH₂CH₂OMe | Et | 2 | CF₃ | SO₂-n-Pr | |
| 5-259 | OCH₂CH₂OMe | n-Pr | 2 | CF₃ | SO₂-n-Pr | |
| 5-260 | OCH₂CH₂OMe | i-Pr | 2 | CF₃ | SO₂-n-Pr | |
| 5-261 | OCH₂CH₂OMe | Me | 2 | CF₃ | SO₂-(CH₂)₂OMe | |
| 5-262 | OCH₂CH₂OMe | Et | 2 | CF₃ | SO₂-(CH₂)₂OMe | |
| 5-263 | OCH₂CH₂OMe | n-Pr | 2 | CF₃ | SO₂-(CH₂)₂OMe | |
| 5-264 | OCH₂CH₂OMe | i-Pr | 2 | CF₃ | SO₂-(CH₂)₂OMe | |
| 5-265 | OCH₂CH₂OMe | Me | 2 | CF₃ | SO₂-Ph | |
| 5-266 | OCH₂CH₂OMe | Et | 2 | CF₃ | SO₂-Ph | |
| 5-267 | OCH₂CH₂OMe | n-Pr | 2 | CF₃ | SO₂-Ph | |
| 5-268 | OCH₂CH₂OMe | i-Pr | 2 | CF₃ | SO₂-Ph | |
| 5-269 | OCH₂CH₂OMe | Me | 2 | CF₃ | SO₂-(4-Me-Ph) | |
| 5-270 | OCH₂CH₂OMe | Et | 2 | CF₃ | SO₂-(4-Me-Ph) | |
| 5-271 | OCH₂CH₂OMe | n-Pr | 2 | CF₃ | SO₂-(4-Me-Ph) | |
| 5-272 | OCH₂CH₂OMe | i-Pr | 2 | CF₃ | SO₂-(4-Me-Ph) | |
| 5-273 | OCH₂CH₂OMe | Me | 2 | CF₃ | SO₂-(Thien-2-yl) | |
| 5-274 | OCH₂CH₂OMe | Et | 2 | CF₃ | SO₂-(Thien-2-yl) | |
| 5-275 | OCH₂CH₂OMe | n-Pr | 2 | CF₃ | SO₂-(Thien-2-yl) | |
| 5-276 | OCH₂CH₂OMe | i-Pr | 2 | CF₃ | SO₂-(Thien-2-yl) | |
| 5-277 | OCH₂CH₂OMe | Me | 2 | CF₃ | CO-Ph | |
| 5-278 | OCH₂CH₂OMe | Et | 2 | CF₃ | CO-Ph | |
| 5-279 | OCH₂CH₂OMe | n-Pr | 2 | CF₃ | CO-Ph | |
| 5-280 | OCH₂CH₂OMe | i-Pr | 2 | CF₃ | CO-Ph | |
| 5-281 | OCH₂CH₂OMe | Me | 2 | CF₃ | CH₂-CO-Ph | |
| 5-282 | OCH₂CH₂OMe | Et | 2 | CF₃ | CH₂-CO-Ph | |
| 5-283 | OCH₂CH₂OMe | n-Pr | 2 | CF₃ | CH₂-CO-Ph | |
| 5-284 | OCH₂CH₂OMe | i-Pr | 2 | CF₃ | CH₂-CO-Ph | |
| 5-285 | OCH₂CH₂OMe | Me | 2 | CF₃ | CH₂-CO-(4-Me-Ph) | |
| 5-286 | OCH₂CH₂OMe | Et | 2 | CF₃ | CH₂-CO-(4-Me-Ph) | |
| 5-287 | OCH₂CH₂OMe | n-Pr | 2 | CF₃ | CH₂-CO-(4-Me-Ph) | |
| 5-288 | OCH₂CH₂OMe | i-Pr | 2 | CF₃ | CH₂-CO-(4-Me-Ph) | |

**Tabelle 6: Erfindungsgemäße Verbindungen der allgemeinen Formel (I), worin R¹ und R² jeweils für Methyl stehen.**

| | | | | | | |
|---|---|---|---|---|---|---|
| 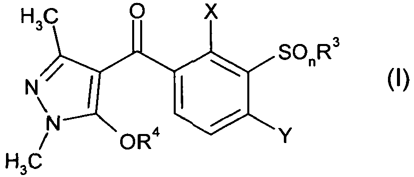 | | | | | | |

| Nr. | X | R³ | n | Y | R⁴ | Physikalische Daten: ¹H-NMR: δ [CDCl₃] |
|---|---|---|---|---|---|---|
| 6-1 | OMe | Me | 0 | CF₃ | SO₂-n-Pr | |
| 6-2 | OMe | Et | 0 | CF₃ | SO₂-n-Pr | |
| 6-3 | OMe | n-Pr | 0 | CF₃ | SO₂-n-Pr | |
| 6-4 | OMe | i-Pr | 0 | CF₃ | SO₂-n-Pr | |
| 6-5 | OMe | Me | 0 | CF₃ | SO₂-(CH₂)₂OMe | |
| 6-6 | OMe | Et | 0 | CF₃ | SO₂-(CH₂)₂OMe | |
| 6-7 | OMe | n-Pr | 0 | CF₃ | SO₂-(CH₂)₂OMe | |
| 6-8 | OMe | i-Pr | 0 | CF₃ | SO₂-(CH₂)₂OMe | |
| 6-9 | OMe | Me | 0 | CF₃ | SO₂-Ph | |
| 6-10 | OMe | Et | 0 | CF₃ | SO₂-Ph | |
| 6-11 | OMe | n-Pr | 0 | CF₃ | SO₂-Ph | |
| 6-12 | OMe | i-Pr | 0 | CF₃ | SO₂-Ph | |
| 6-13 | OMe | Me | 0 | CF₃ | SO₂-(4-Me-Ph) | |
| 6-14 | OMe | Et | 0 | CF₃ | SO₂-(4-Me-Ph) | |
| 6-15 | OMe | n-Pr | 0 | CF₃ | SO₂-(4-Me-Ph) | |
| 6-16 | OMe | i-Pr | 0 | CF₃ | SO₂-(4-Me-Ph) | |
| 6-17 | OMe | Me | 0 | CF₃ | SO₂-(Thien-2-yl) | |
| 6-18 | OMe | Et | 0 | CF₃ | SO₂-(Thien-2-yl) | |
| 6-19 | OMe | n-Pr | 0 | CF₃ | SO₂-(Thien-2-yl) | |
| 6-20 | OMe | i-Pr | 0 | CF₃ | SO₂-(Thien-2-yl) | |
| 6-21 | OMe | Me | 0 | CF₃ | CO-Ph | |
| 6-22 | OMe | Et | 0 | CF₃ | CO-Ph | |
| 6-23 | OMe | n-Pr | 0 | CF₃ | CO-Ph | |
| 6-24 | OMe | i-Pr | 0 | CF₃ | CO-Ph | |
| 6-25 | OMe | Me | 0 | CF₃ | CH₂-CO-Ph | |
| 6-26 | OMe | Et | 0 | CF₃ | CH₂-CO-Ph | |
| 6-27 | OMe | n-Pr | 0 | CF₃ | CH₂-CO-Ph | |
| 6-28 | OMe | i-Pr | 0 | CF₃ | CH₂-CO-Ph | |
| 6-29 | OMe | Me | 0 | CF₃ | CH₂-CO-(4-Me-Ph) | |
| 6-30 | OMe | Et | 0 | CF₃ | CH₂-CO-(4-Me-Ph) | |
| 6-31 | OMe | n-Pr | 0 | CF₃ | CH₂-CO-(4-Me-Ph) | |
| 6-32 | OMe | i-Pr | 0 | CF₃ | CH₂-CO-(4-Me-Ph) | |
| 6-33 | OMe | Me | 1 | CF₃ | SO₂-n-Pr | |
| 6-34 | OMe | Et | 1 | CF₃ | SO₂-n-Pr | |
| 6-35 | OMe | n-Pr | 1 | CF₃ | SO₂-n-P | |
| 6-36 | OMe | i-Pr | 1 | CF₃ | SO₂-n-Pr | |
| 6-37 | OMe | Me | 1 | CF₃ | SO₂-(CH₂)₂OMe | |
| 6-38 | OMe | Et | 1 | CF₃ | SO₂-(CH₂)₂OMe | |
| 6-39 | OMe | n-Pr | 1 | CF₃ | SO₂-(CH₂)₂OMe | |
| 6-40 | OMe | i-Pr | 1 | CF₃ | SO₂-(CH₂)₂OMe | |
| 6-41 | OMe | Me | 1 | CF₃ | SO₂-Ph | |
| 6-42 | OMe | Et | 1 | CF₃ | SO₂-Ph | |
| 6-43 | OMe | n-Pr | 1 | CF₃ | SO₂-Ph | |
| 6-44 | OMe | i-Pr | 1 | CF₃ | SO₂-Ph | |
| 6-45 | OMe | Me | 1 | CF₃ | SO₂-(4-Me-Ph) | |
| 6-46 | OMe | Et | 1 | CF₃ | SO₂-(4-Me-Ph) | |
| 6-47 | OMe | n-Pr | 1 | CF₃ | SO₂-(4-Me-Ph) | |
| 6-48 | OMe | i-Pr | 1 | CF₃ | SO₂-(4-Me-Ph) | |
| 6-49 | OMe | Me | 1 | CF₃ | SO₂-(Thien-2-yl) | |
| 6-50 | OMe | Et | 1 | CF₃ | SO₂-(Thien-2-yl) | |
| 6-51 | OMe | n-Pr | 1 | CF₃ | SO₂-(Thien-2-yl) | |
| 6-52 | OMe | i-Pr | 1 | CF₃ | SO₂-(Thien-2-yl) | |
| 6-53 | OMe | Me | 1 | CF₃ | CO-Ph | |
| 6-54 | OMe | Et | 1 | CF₃ | CO-Ph | |
| 6-55 | OMe | n-Pr | 1 | CF₃ | CO-Ph | |
| 6-56 | OMe | i-Pr | 1 | CF₃ | CO-Ph | |
| 6-57 | OMe | Me | 1 | CF₃ | CH₂-CO-Ph | |
| 6-58 | OMe | Et | 1 | CF₃ | CH₂-CO-Ph | |
| 6-59 | OMe | n-Pr | 1 | CF₃ | CH₂-CO-Ph | |
| 6-60 | OMe | i-Pr | 1 | CF₃ | CH₂-CO-Ph | |
| 6-61 | OMe | Me | 1 | CF₃ | CH₂-CO-(4-Me-Ph) | |
| 6-62 | OMe | Et | 1 | CF₃ | CH₂-CO-(4-Me-Ph) | |
| 6-63 | OMe | n-Pr | 1 | CF₃ | CH₂-CO-(4-Me-Ph) | |
| 6-64 | OMe | i-Pr | 1 | CF₃ | CH₂-CO-(4-Me-Ph) | |
| 6-65 | OMe | Me | 2 | CF₃ | SO₂-n-Pr | |
| 6-66 | OMe | Et | 2 | CF₃ | SO₂-n-Pr | |
| 6-67 | OMe | n-Pr | 2 | CF₃ | SO₂-n-Pr | |
| 6-68 | OMe | i-Pr | 2 | CF₃ | SO₂-n-Pr | |
| 6-69 | OMe | Me | 2 | CF₃ | SO₂-(CH₂)₂OMe | |
| 6-70 | OMe | Et | 2 | CF₃ | SO₂-(CH₂)₂OMe | |
| 6-71 | OMe | n-Pr | 2 | CF₃ | SO₂-(CH₂)₂OMe | |
| 6-72 | OMe | i-Pr | 2 | CF₃ | SO₂-(CH₂)₂OMe | |
| 6-73 | OMe | Me | 2 | CF₃ | SO₂-Ph | |
| 6-74 | OMe | Et | 2 | CF₃ | SO₂-Ph | |
| 6-75 | OMe | n-Pr | 2 | CF₃ | SO₂-Ph | |
| 6-76 | OMe | i-Pr | 2 | CF₃ | SO₂-Ph | |
| 6-77 | OMe | Me | 2 | CF₃ | SO₂-(4-Me-Ph) | |
| 6-78 | OMe | Et | 2 | CF₃ | SO₂-(4-Me-Ph) | |
| 6-79 | OMe | n-Pr | 2 | CF₃ | SO₂-(4-Me-Ph) | |
| 6-80 | OMe | i-Pr | 2 | CF₃ | SO₂-(4-Me-Ph) | |
| 6-81 | OMe | Me | 2 | CF₃ | SO₂-(Thien-2-yl) | |
| 6-82 | OMe | Et | 2 | CF₃ | SO₂-(Thien-2-yl) | |
| 6-83 | OMe | n-Pr | 2 | CF₃ | SO₂-(Thien-2-yl) | |
| 6-84 | OMe | i-Pr | 2 | CF₃ | SO₂-(Thien-2-yl) | |
| 6-85 | OMe | Me | 2 | CF₃ | CO-Ph | |
| 6-86 | OMe | Et | 2 | CF₃ | CO-Ph | |
| 6-87 | OMe | n-Pr | 2 | CF₃ | CO-Ph | |
| 6-88 | OMe | i-Pr | 2 | CF₃ | CO-Ph | |
| 6-89 | OMe | Me | 2 | CF₃ | CH₂-CO-Ph | |
| 6-90 | OMe | Et | 2 | CF₃ | CH₂-CO-Ph | |
| 6-91 | OMe | n-Pr | 2 | CF₃ | CH₂-CO-Ph | |
| 6-92 | OMe | i-Pr | 2 | CF₃ | CH₂-CO-Ph | |
| 6-93 | OMe | Me | 2 | CF₃ | CH₂-CO-(4-Me-Ph) | |
| 6-94 | OMe | Et | 2 | CF₃ | CH₂-CO-(4-Me-Ph) | |
| 6-95 | OMe | n-Pr | 2 | CF₃ | CH₂-CO-(4-Me-Ph) | |
| 6-96 | OMe | i-Pr | 2 | CF₃ | CH₂-CO-(4-Me-Ph) | |
| 6-97 | OEt | Me | 0 | CF₃ | SO₂-n-Pr | |
| 6-98 | OEt | Et | 0 | CF₃ | SO₂-n-Pr | |
| 6-99 | OEt | n-Pr | 0 | CF₃ | SO₂-n-Pr | |
| 6-100 | OEt | i-Pr | 0 | CF₃ | SO₂-n-Pr | |
| 6-101 | OEt | Me | 0 | CF₃ | SO₂-(CH₂)₂OMe | |
| 6-102 | OEt | Et | 0 | CF₃ | SO₂-(CH₂)₂OMe | |
| 6-103 | OEt | n-Pr | 0 | CF₃ | SO₂-(CH₂)₂OMe | |
| 6-104 | OEt | i-Pr | 0 | CF₃ | SO₂-(CH₂)₂OMe | |
| 6-105 | OEt | Me | 0 | CF₃ | SO₂-Ph | |
| 6-106 | OEt | Et | 0 | CF₃ | SO₂-Ph | |
| 6-107 | OEt | n-Pr | 0 | CF₃ | SO₂-Ph | |
| 6-108 | OEt | i-Pr | 0 | CF₃ | SO₂-Ph | |
| 6-109 | OEt | Me | 0 | CF₃ | SO₂-(4-Me-Ph) | |
| 6-110 | OEt | Et | 0 | CF₃ | SO₂-(4-Me-Ph) | |
| 6-111 | OEt | n-Pr | 0 | CF₃ | SO₂-(4-Me-Ph) | |
| 6-112 | OEt | i-Pr | 0 | CF₃ | SO₂-(4-Me-Ph) | |
| 6-113 | OEt | Me | 0 | CF₃ | SO₂-(Thien-2-yl) | |
| 6-114 | OEt | Et | 0 | CF₃ | SO₂-(Thien-2-yl) | |
| 6-115 | OEt | n-Pr | 0 | CF₃ | SO₂-(Thien-2-yl) | |
| 6-116 | OEt | i-Pr | 0 | CF₃ | SO₂-(Thien-2-yl) | |
| 6-117 | OEt | Me | 0 | CF₃ | CO-Ph | |
| 6-118 | OEt | Et | 0 | CF₃ | CO-Ph | |
| 6-119 | OEt | n-Pr | 0 | CF₃ | CO-Ph | |
| 6-120 | OEt | i-Pr | 0 | CF₃ | CO-Ph | |
| 6-121 | OEt | Me | 0 | CF₃ | CH₂-CO-Ph | |
| 6-122 | OEt | Et | 0 | CF₃ | CH₂-CO-Ph | |
| 6-123 | OEt | n-Pr | 0 | CF₃ | CH₂-CO-Ph | |
| 6-124 | OEt | i-Pr | 0 | CF₃ | CH₂-CO-Ph | |
| 6-125 | OEt | Me | 0 | CF₃ | CH₂-CO-(4-Me-Ph) | |
| 6-126 | OEt | Et | 0 | CF₃ | CH₂-CO-(4-Me-Ph) | |
| 6-127 | OEt | n-Pr | 0 | CF₃ | CH₂-CO-(4-Me-Ph) | |
| 6-128 | OEt | i-Pr | 0 | CF₃ | CH₂-CO-(4-Me-Ph) | |
| 6-129 | OEt | Me | 1 | CF₃ | SO₂-n-Pr | |
| 6-130 | OEt | Et | 1 | CF₃ | SO₂-n-Pr | |
| 6-131 | OEt | n-Pr | 1 | CF₃ | SO₂-n-Pr | |
| 6-132 | OEt | i-Pr | 1 | CF₃ | SO₂-n-Pr | |
| 6-133 | OEt | Me | 1 | CF₃ | SO₂-(CH₂)₂OMe | |
| 6-134 | OEt | Et | 1 | CF₃ | SO₂-(CH₂)₂OMe | |
| 6-135 | OEt | n-Pr | 1 | CF₃ | SO₂-(CH₂)₂OMe | |
| 6-136 | OEt | i-Pr | 1 | CF₃ | SO₂-(CH₂)₂OMe | |
| 6-137 | OEt | Me | 1 | CF₃ | SO₂-Ph | |
| 6-138 | OEt | Et | 1 | CF₃ | SO₂-Ph | |
| 6-139 | OEt | n-Pr | 1 | CF₃ | SO₂-Ph | |
| 6-140 | OEt | i-Pr | 1 | CF₃ | SO₂-Ph | |
| 6-141 | OEt | Me | 1 | CF₃ | SO₂-(4-Me-Ph) | |
| 6-142 | OEt | Et | 1 | CF₃ | SO₂-(4-Me-Ph) | |
| 6-143 | OEt | n-Pr | 1 | CF₃ | SO₂-(4-Me-Ph) | |
| 6-144 | OEt | i-Pr | 1 | CF₃ | SO₂-(4-Me-Ph) | |
| 6-145 | OEt | Me | 1 | CF₃ | SO₂-(Thien-2-yl) | |
| 6-146 | OEt | Et | 1 | CF₃ | SO₂-(Thien-2-yl) | |
| 6-147 | OEt | n-Pr | 1 | CF₃ | SO₂-(Thien-2-yl) | |
| 6-148 | OEt | i-Pr | 1 | CF₃ | SO₂-(Thien-2-yl) | |
| 6-149 | OEt | Me | 1 | CF₃ | CO-Ph | |
| 6-150 | OEt | Et | 1 | CF₃ | CO-Ph | |
| 6-151 | OEt | n-Pr | 1 | CF₃ | CO-Ph | |
| 6-152 | OEt | i-Pr | 1 | CF₃ | CO-Ph | |
| 6-153 | OEt | Me | 1 | CF₃ | CH₂-CO-Ph | |
| 6-154 | OEt | Et | 1 | CF₃ | CH₂-CO-Ph | |
| 6-155 | OEt | n-Pr | 1 | CF₃ | CH₂-CO-Ph | |
| 6-156 | OEt | i-Pr | 1 | CF₃ | CH₂-CO-Ph | |
| 6-157 | OEt | Me | 1 | CF₃ | CH₂-CO-(4-Me-Ph) | |
| 6-158 | OEt | Et | 1 | CF₃ | CH₂-CO-(4-Me-Ph) | |
| 6-159 | OEt | n-Pr | 1 | CF₃ | CH₂-CO-(4-Me-Ph) | |
| 6-160 | OEt | i-Pr | 1 | CF₃ | CH₂-CO-(4-Me-Ph) | |
| 6-161 | OEt | Me | 2 | CF₃ | SO₂-n-Pr | |
| 6-162 | OEt | Et | 2 | CF₃ | SO₂-n-Pr | |
| 6-163 | OEt | n-Pr | 2 | CF₃ | SO₂-n-Pr | |
| 6-164 | OEt | i-Pr | 2 | CF₃ | SO₂-n-Pr | |
| 6-165 | OEt | Me | 2 | CF₃ | SO₂-(CH₂)₂OMe | |
| 6-166 | OEt | Et | 2 | CF₃ | SO₂-(CH₂)₂OMe | |
| 6-167 | OEt | n-Pr | 2 | CF₃ | SO₂-(CH₂)₂OMe | |
| 6-168 | OEt | i-Pr | 2 | CF₃ | SO₂-(CH₂)₂OMe | |
| 6-169 | OEt | Me | 2 | CF₃ | SO₂-Ph | |
| 6-170 | OEt | Et | 2 | CF₃ | SO₂-Ph | |
| 6-171 | OEt | n-Pr | 2 | CF₃ | SO₂-Ph | |
| 6-172 | OEt | i-Pr | 2 | CF₃ | SO₂-Ph | |
| 6-173 | OEt | Me | 2 | CF₃ | SO₂-(4-Me-Ph) | |
| 6-174 | OEt | Et | 2 | CF₃ | SO₂-(4-Me-Ph) | |
| 6-175 | OEt | n-Pr | 2 | CF₃ | SO₂-(4-Me-Ph) | |
| 6-176 | OEt | i-Pr | 2 | CF₃ | SO₂-(4-Me-Ph) | |
| 6-177 | OEt | Me | 2 | CF₃ | SO₂-(Thien-2-yl) | |
| 6-178 | OEt | Et | 2 | CF₃ | SO₂-(Thien-2-yl) | |
| 6-179 | OEt | n-Pr | 2 | CF₃ | SO₂-(Thien-2-yl) | |
| 6-180 | OEt | i-Pr | 2 | CF₃ | SO₂-(Thien-2-yl) | |
| 6-181 | OEt | Me | 2 | CF₃ | CO-Ph | |
| 6-182 | OEt | Et | 2 | CF₃ | CO-Ph | |
| 6-183 | OEt | n-Pr | 2 | CF₃ | CO-Ph | |
| 6-184 | OEt | i-Pr | 2 | CF₃ | CO-Ph | |
| 6-185 | OEt | Me | 2 | CF₃ | CH₂-CO-Ph | |
| 6-186 | OEt | Et | 2 | CF₃ | CH₂-CO-Ph | |
| 6-187 | OEt | n-Pr | 2 | CF₃ | CH₂-CO-Ph | |
| 6-188 | OEt | i-Pr | 2 | CF₃ | CH₂-CO-Ph | |
| 6-189 | OEt | Me | 2 | CF₃ | CH₂-CO-(4-Me-Ph) | |
| 6-190 | OEt | Et | 2 | CF₃ | CH₂-CO-(4-Me-Ph) | |
| 6-191 | OEt | n-Pr | 2 | CF₃ | CH₂-CO-(4-Me-Ph) | |
| 6-192 | OEt | i-Pr | 2 | CF₃ | CH₂-CO-(4-Me-Ph) | |
| 6-193 | OCH₂CH₂OMe | Me | 0 | CF₃ | SO₂-n-Pr | |
| 6-194 | OCH₂CH₂OMe | Et | 0 | CF₃ | SO₂-n-Pr | |
| 6-195 | OCH₂CH₂OMe | n-Pr | 0 | CF₃ | SO₂-n-Pr | |
| 6-196 | OCH₂CH₂OMe | i-Pr | 0 | CF₃ | SO₂-n-Pr | |
| 6-197 | OCH₂CH₂OM | Me | 0 | CF₃ | SO₂-(CH₂)₂OMe | |
| 6-198 | OCH₂CH₂OMe | Et | 0 | CF₃ | SO₂-(CH₂)₂OMe | |
| 6-199 | OCH₂CH₂OMe | n-Pr | 0 | CF₃ | SO₂-(CH₂)₂OMe | |
| 6-200 | OCH₂CH₂OMe | i-Pr | 0 | CF₃ | SO₂-(CH₂)₂OMe | |
| 6-201 | OCH₂CH₂OMe | Me | 0 | CF₃ | SO₂-Ph | |
| 6-202 | OCH₂CH₂OMe | Et | 0 | CF₃ | SO₂-Ph | |
| 6-203 | OCH₂CH₂OMe | n-Pr | 0 | CF₃ | SO₂-Ph | |
| 6-204 | OCH₂CH₂OMe | i-Pr | 0 | CF₃ | SO₂-Ph | |
| 6-205 | OCH₂CH₂OMe | Me | 0 | CF₃ | SO₂-(4-Me-Ph) | |
| 6-206 | OCH₂CH₂OMe | Et | 0 | CF₃ | SO₂-(4-Me-Ph) | |
| 6-207 | OCH₂CH₂OMe | n-Pr | 0 | CF₃ | SO₂-(4-Me-Ph) | |
| 6-208 | OCH₂CH₂OMe | i-Pr | 0 | CF₃ | SO₂-(4-Me-Ph) | |
| 6-209 | OCH₂CH₂OMe | Me | 0 | CF₃ | SO₂-(Thien-2-yl) | |
| 6-210 | OCH₂CH₂OMe | Et | 0 | CF₃ | SO₂-(Thien-2-yl) | |
| 6-211 | OCH₂CH₂OMe | n-Pr | 0 | CF₃ | SO₂-(Thien-2-yl) | |
| 6-212 | OCH₂CH₂OMe | i-Pr | 0 | CF₃ | SO₂-(Thien-2-yl) | |
| 6-213 | OCH₂CH₂OMe | Me | 0 | CF₃ | CO-Ph | |
| 6-214 | OCH₂CH₂OMe | Et | 0 | CF₃ | CO-Ph | |
| 6-215 | OCH₂CH₂OMe | n-Pr | 0 | CF₃ | CO-Ph | |
| 6-216 | OCH₂CH₂OMe | i-Pr | 0 | CF₃ | CO-Ph | |
| 6-217 | OCH₂CH₂OMe | Me | 0 | CF₃ | CH₂-CO-Ph | |
| 6-218 | OCH₂CH₂OMe | Et | 0 | CF₃ | CH₂-CO-Ph | |
| 6-219 | OCH₂CH₂OMe | n-Pr | 0 | CF₃ | CH₂-CO-Ph | |
| 6-220 | OCH₂CH₂OMe | i-Pr | 0 | CF₃ | CH₂-CO-Ph | |
| 6-221 | OCH₂CH₂OMe | Me | 0 | CF₃ | CH₂-CO-(4-Me-Ph) | |
| 6-222 | OCH₂CH₂OMe | Et | 0 | CF₃ | CH₂-CO-(4-Me-Ph) | |
| 6-223 | OCH₂CH₂OMe | n-Pr | 0 | CF₃ | CH₂-CO-(4-Me-Ph) | |
| 6-224 | OCH₂CH₂OMe | i-Pr | 0 | CF₃ | CH₂-CO-(4-Me-Ph) | |
| 6-225 | OCH₂CH₂OMe | Me | 1 | CF₃ | SO₂-n-Pr | |
| 6-226 | OCH₂CH₂OMe | Et | 1 | CF₃ | SO₂-n-Pr | |
| 6-227 | OCH₂CH₂OMe | n-Pr | 1 | CF₃ | SO₂-n-Pr | |
| 6-228 | OCH₂CH₂OMe | i-Pr | 1 | CF₃ | SO₂-n-Pr | |
| 6-229 | OCH₂CH₂OMe | Me | 1 | CF₃ | SO₂-(CH₂)₂OMe | |
| 6-230 | OCH₂CH₂OMe | Et | 1 | CF₃ | SO₂-(CH₂)₂OMe | |
| 6-231 | OCH₂CH₂OMe | n-Pr | 1 | CF₃ | SO₂-(CH₂)₂OMe | |
| 6-232 | OCH₂CH₂OMe | i-Pr | 1 | CF₃ | SO₂-(CH₂)₂OMe | |
| 6-233 | OCH₂CH₂OMe | Me | 1 | CF₃ | SO₂-Ph | |
| 6-234 | OCH₂CH₂OMe | Et | 1 | CF₃ | SO₂-Ph | |
| 6-235 | OCH₂CH₂OMe | n-Pr | 1 | CF₃ | SO₂-Ph | |
| 6-236 | OCH₂CH₂OMe | i-Pr | 1 | CF₃ | SO₂-Ph | |
| 6-237 | OCH₂CH₂OMe | Me | 1 | CF₃ | SO₂-(4-Me-Ph) | |
| 6-238 | OCH₂CH₂OMe | Et | 1 | CF₃ | SO₂-(4-Me-Ph) | |
| 6-239 | OCH₂CH₂OMe | n-Pr | 1 | CF₃ | SO₂-(4-Me-Ph) | |
| 6-240 | OCH₂CH₂OMe | i-Pr | 1 | CF₃ | SO₂-(4-Me-Ph) | |
| 6-241 | OCH₂CH₂OMe | Me | 1 | CF₃ | SO₂-(Thien-2-yl) | |
| 6-242 | OCH₂CH₂OMe | Et | 1 | CF₃ | SO₂-(Thien-2-yl) | |
| 6-243 | OCH₂CH₂OMe | n-Pr | 1 | CF₃ | SO₂-(Thien-2-yl) | |
| 6-244 | OCH₂CH₂OMe | i-Pr | 1 | CF₃ | SO₂-(Thien-2-yl) | |
| 6-245 | OCH₂CH₂OMe | Me | 1 | CF₃ | CO-Ph | |
| 6-246 | OCH₂CH₂OMe | Et | 1 | CF₃ | CO-Ph | |
| 6-247 | OCH₂CH₂OMe | n-Pr | 1 | CF₃ | CO-Ph | |
| 6-248 | OCH₂CH₂OMe | i-Pr | 1 | CF₃ | CO-Ph | |
| 6-249 | OCH₂CH₂OMe | Me | 1 | CF₃ | CH₂-CO-Ph | |
| 6-250 | OCH₂CH₂OMe | Et | 1 | CF₃ | CH₂-CO-Ph | |
| 6-251 | OCH₂CH₂OMe | n-Pr | 1 | CF₃ | CH₂-CO-Ph | |
| 6-252 | OCH₂CH₂OMe | i-Pr | 1 | CF₃ | CH₂-CO-Ph | |
| 6-253 | OCH₂CH₂OMe | Me | 1 | CF₃ | CH₂-CO-(4-Me-Ph) | |
| 6-254 | OCH₂CH₂OMe | Et | 1 | CF₃ | CH₂-CO-(4-Me-Ph) | |
| 6-255 | OCH₂CH₂OMe | n-Pr | 1 | CF₃ | CH₂-CO-(4-Me-Ph) | |
| 6-256 | OCH₂CH₂OMe | i-Pr | 1 | CF₃ | CH₂-CO-(4-Me-Ph) | |
| 6-257 | OCH₂CH₂OMe | Me | 2 | CF₃ | SO₂-n-Pr | |
| 6-258 | OCH₂CH₂OMe | Et | 2 | CF₃ | SO₂-n-Pr | |
| 6-259 | OCH₂CH₂OMe | n-Pr | 2 | CF₃ | SO₂-n-Pr | |
| 6-260 | OCH₂CH₂OMe | i-Pr | 2 | CF₃ | SO₂-n-Pr | |
| 6-261 | OCH₂CH₂OMe | Me | 2 | CF₃ | SO₂-(CH₂)₂OMe | |
| 6-262 | OCH₂CH₂OMe | Et | 2 | CF₃ | SO₂-(CH₂)₂OMe | |
| 6-263 | OCH₂CH₂OMe | n-Pr | 2 | CF₃ | SO₂-(CH₂)₂OMe | |
| 6-264 | OCH₂CH₂OMe | i-Pr | 2 | CF₃ | SO₂-(CH₂)₂OMe | |
| 6-265 | OCH₂CH₂OMe | Me | 2 | CF₃ | SO₂-Ph | |
| 6-266 | OCH₂CH₂OMe | Et | 2 | CF₃ | SO₂-Ph | |
| 6-267 | OCH₂CH₂OMe | n-Pr | 2 | CF₃ | SO₂-Ph | |
| 6-268 | OCH₂CH₂OMe | i-Pr | 2 | CF₃ | SO₂-Ph | |
| 6-269 | OCH₂CH₂OMe | Me | 2 | CF₃ | SO₂-(4-Me-Ph) | |
| 6-270 | OCH₂CH₂OMe | Et | 2 | CF₃ | SO₂-(4-Me-Ph) | |
| 6-271 | OCH₂CH₂OMe | n-Pr | 2 | CF₃ | SO₂-(4-Me-Ph) | |
| 6-272 | OCH₂CH₂OMe | i-Pr | 2 | CF₃ | SO₂-(4-Me-Ph) | |
| 6-273 | OCH₂CH₂OMe | Me | 2 | CF₃ | SO₂-(Thien-2-yl) | |
| 6-274 | OCH₂CH₂OMe | Et | 2 | CF₃ | SO₂-(Thien-2-yl) | |
| 6-275 | OCH₂CH₂OMe | n-Pr | 2 | CF₃ | SO₂-(Thien-2-yl) | |
| 6-276 | OCH₂CH₂OMe | i-Pr | 2 | CF₃ | SO₂-(Thien-2-yl) | |
| 6-277 | OCH₂CH₂OMe | Me | 2 | CF₃ | CO-Ph | |
| 6-278 | OCH₂CH₂OMe | Et | 2 | CF₃ | CO-Ph | |
| 6-279 | OCH₂CH₂OMe | n-Pr | 2 | CF₃ | CO-Ph | |
| 6-280 | OCH₂CH₂OMe | i-Pr | 2 | CF₃ | CO-Ph | |
| 6-281 | OCH₂CH₂OMe | Me | 2 | CF₃ | CH₂-CO-Ph | |
| 6-282 | OCH₂CH₂OMe | Et | 2 | CF₃ | CH₂-CO-Ph | |
| 6-283 | OCH₂CH₂OMe | n-Pr | 2 | CF₃ | CH₂-CO-Ph | |
| 6-284 | OCH₂CH₂OMe | i-Pr | 2 | CF₃ | CH₂-CO-Ph | |
| 6-285 | OCH₂CH₂OMe | Me | 2 | CF₃ | CH₂-CO-(4-Me-Ph) | |
| 6-286 | OCH₂CH₂OMe | Et | 2 | CF₃ | CH₂-CO-(4-Me-Ph) | |
| 6-287 | OCH₂CH₂OMe | n-Pr | 2 | CF₃ | CH₂-CO-(4-Me-Ph) | |
| 6-288 | OCH₂CH₂OMe | i-Pr | 2 | CF₃ | CH₂-CO-(4-Me-Ph) | |

**Tabelle 7: Erfindungsgemäße Verbindungen der allgemeinen Formel (II)**

| | | | | | |
|---|---|---|---|---|---|
| 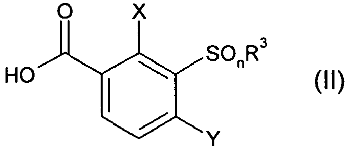 | | | | | |

| **Nr.** | **X** | **R³** | **n** | **Y** | **Physikalische Daten: ¹H-NMR: δ [DMSO-d₆]** |
|---|---|---|---|---|---|
| 7-1 | OH | Me | 0 | CF₃ | |
| 7-2 | OH | Et | 0 | CF₃ | |
| 7-3 | OH | n-Pr | 0 | CF₃ | |
| 7-4 | OH | i-Pr | 0 | CF₃ | |
| 7-5 | OH | Me | 1 | CF₃ | |
| 7-6 | OH | Et | 1 | CF₃ | |
| 7-7 | OH | n-Pr | 1 | CF₃ | |
| 7-8 | OH | i-Pr | 1 | CF₃ | |
| 7-9 | OH | Me | 2 | CF₃ | |
| 7-10 | OH | Et | 2 | CF₃ | |
| 7-11 | OH | n-Pr | 2 | CF₃ | |
| 7-12 | OH | i-Pr | 2 | CF₃ | |
| 7-13 | OMe | Me | 0 | CF₃ | 7.76 (d, 1H), 7.58 (d, 1H), 3.91 (s, 3H), 2.40 (s, 3H) |
| 7-14 | OMe | Et | 0 | CF₃ | 7.78 (d, 1H), 7.61 (d, 1H), 3.90 (s, 3H), 2.95 (q, 2H), 1.07 (t, 3H) |
| 7-15 | OMe | n-Pr | 0 | CF₃ | |
| 7-16 | OMe | i-Pr | 0 | CF₃ | |
| 7-17 | OMe | Me | 1 | CF₃ | |
| 7-18 | OMe | Et | 1 | CF₃ | |
| 7-19 | OMe | n-Pr | 1 | CF₃ | |
| 7-20 | OMe | i-Pr | 1 | CF₃ | |
| 7-21 | OMe | Me | 2 | CF₃ | |
| 7-22 | OMe | Et | 2 | CF₃ | |
| 7-23 | OMe | n-Pr | 2 | CF₃ | |
| 7-24 | OMe | i-Pr | 2 | CF₃ | |
| 7-25 | OEt | Me | 0 | CF₃ | 7.78 (d, 1H), 7.60 (d, 1H), 4.12 (q, 2H), 2.43 (s, 3H), 1.37 (t, 3H) |
| 7-26 | OEt | Et | 0 | CF₃ | 7.78 (d, 1H), 7.61 (d, 1H), 4.12 (q, 2H), 2.98 (q, 2H), 1.36 (t, 3H), 1.08 (t, 3H) |
| 7-27 | OEt | n-Pr | 0 | CF₃ | |
| 7-28 | OEt | i-Pr | 0 | CF₃ | |
| 7-29 | OEt | Me | 1 | CF₃ | |
| 7-30 | OEt | Et | 1 | CF₃ | |
| 7-31 | OEt | n-Pr | 1 | CF₃ | |
| 7-32 | OEt | i-Pr | 1 | CF₃ | |
| 7-33 | OEt | Me | 2 | CF₃ | |
| 7-34 | OEt | Et | 2 | CF₃ | |
| 7-35 | OEt | n-Pr | 2 | CF₃ | |
| 7-36 | OEt | i-Pr | 2 | CF₃ | |
| 7-37 | O-CH₂-c-Pr | Me | 0 | CF₃ | 7.77 (d, 1H), 7.60 (d, 1H), 3.92 (d, 2H), 2.46 (s, 3H), 1.27 (m, 1H), 0.57 (m, 2H), 0.33 (m, 2H) |
| 7-38 | O-CH₂-c-Pr | Et | 0 | CF₃ | |
| 7-39 | O-CH₂-c-Pr | n-Pr | 0 | CF₃ | |
| 7-40 | O-CH₂-c-Pr | i-Pr | 0 | CF₃ | |
| 7-41 | O-CH₂-c-Pr | Me | 1 | CF₃ | |
| 7-42 | O-CH₂-c-Pr | Et | 1 | CF₃ | |
| 7-43 | O-CH₂-c-Pr | n-Pr | 1 | CF₃ | |
| 7-44 | O-CH₂-c-Pr | i-Pr | 1 | CF₃ | |
| 7-45 | O-CH₂-c-Pr | Me | 2 | CF₃ | |
| 7-46 | O-CH₂-c-Pr | Et | 2 | CF₃ | |
| 7-47 | O-CH₂-c-Pr | n-Pr | 2 | CF₃ | |
| 7-48 | O-CH₂-c-Pr | i-Pr | 2 | CF₃ | |
| 7-49 | OCH₂CH₂OMe | Me | 0 | CF₃ | 7.77 (d, 1H), 7.60 (d, 1H), 4.22 (t, 2H), 3.71 (t, 2H), 2.44 (s, 3H) |
| 7-50 | OCH₂CH₂OMe | Et | 0 | CF₃ | |
| 7-51 | OCH₂CH₂OMe | n-Pr | 0 | CF₃ | |
| 7-52 | OCH₂CH₂OMe | i-Pr | 0 | CF₃ | |
| 7-53 | OCH₂CH₂OMe | Me | 1 | CF₃ | |
| 7-54 | OCH₂CH₂OMe | Et | 1 | CF₃ | |
| 7-55 | OCH₂CH₂OMe | n-Pr | 1 | CF₃ | |
| 7-56 | OCH₂CH₂OMe | i-Pr | 1 | CF₃ | |
| 7-57 | OCH₂CH₂OMe | Me | 2 | CF₃ | |
| 7-58 | OCH₂CH₂OMe | Et | 2 | CF₃ | |
| 7-59 | OCH₂CH₂OMe | n-Pr | 2 | CF₃ | |
| 7-60 | OCH₂CH₂OMe | i-Pr | 2 | CF₃ | |
| 7-61 | OCH₂CH₂SMe | Me | 0 | CF₃ | |
| 7-62 | OCH₂CH₂SMe | Et | 0 | CF₃ | |
| 7-63 | OCH₂CH₂SMe | n-Pr | 0 | CF₃ | |
| 7-64 | OCH₂CH₂SMe | i-Pr | 0 | CF₃ | |
| 7-65 | OCH₂CH₂SMe | Me | 1 | CF₃ | |
| 7-66 | OCH₂CH₂SMe | Et | 1 | CF₃ | |
| 7-67 | OCH₂CH₂SMe | n-Pr | 1 | CF₃ | |
| 7-68 | OCH₂CH₂SMe | i-Pr | 1 | CF₃ | |
| 7-69 | OCH₂CH₂SMe | Me | 2 | CF₃ | |
| 7-70 | OCH₂CH₂SMe | Et | 2 | CF₃ | |
| 7-71 | OCH₂CH₂SMe | n-Pr | 2 | CF₃ | |
| 7-72 | OCH₂CH₂SMe | i-Pr | 2 | CF₃ | |
| 7-73 | OCH₂CH₂SO₂Me | Me | 0 | CF₃ | |
| 7-74 | OCH₂CH₂SO₂Me | Et | 0 | CF₃ | |
| 7-75 | OCH₂CH₂SO₂Me | n-Pr | 0 | CF₃ | |
| 7-76 | OCH₂CH₂SO₂Me | i-Pr | 0 | CF₃ | |
| 7-77 | OCH₂CH₂SO₂Me | Me | 1 | CF₃ | |
| 7-78 | OCH₂CH₂SO₂Me | Et | 1 | CF₃ | |
| 7-79 | OCH₂CH₂SO₂Me | n-Pr | 1 | CF₃ | |
| 7-80 | OCH₂CH₂SO₂Me | i-Pr | 1 | CF₃ | |
| 7-81 | OCH₂CH₂SO₂Me | Me | 2 | CF₃ | |
| 7-82 | OCH₂CH₂SO₂Me | Et | 2 | CF₃ | |
| 7-83 | OCH₂CH₂SO₂Me | n-Pr | 2 | CF₃ | |
| 7-84 | OCH₂CH₂SO₂Me | i-Pr | 2 | CF₃ | |
| 7-85 | OCOMe | Me | 0 | CF₃ | |
| 7-86 | OCOMe | Et | 0 | CF₃ | |
| 7-87 | OCOMe | n-Pr | 0 | CF₃ | |
| 7-88 | OCOMe | i-Pr | 0 | CF₃ | |
| 7-89 | OCOMe | Me | 1 | CF₃ | |
| 7-90 | OCOMe | Et | 1 | CF₃ | |
| 7-91 | OCOMe | n-Pr | 1 | CF₃ | |
| 7-92 | OCOMe | i-Pr | 1 | CF₃ | |
| 7-93 | OCOMe | Me | 2 | CF₃ | |
| 7-94 | OCOMe | Et | 2 | CF₃ | |
| 7-95 | OCOMe | n-Pr | 2 | CF₃ | |
| 7-96 | OCOMe | i-Pr | 2 | CF₃ | |
| 7-97 | OSO₂Me | Me | 0 | CF₃ | |
| 7-98 | OSO₂Me | Et | 0 | CF₃ | |
| 7-99 | OSO₂Me | n-Pr | 0 | CF₃ | |
| 7-100 | OSO₂Me | i-Pr | 0 | CF₃ | |
| 7-101 | OSO₂Me | Me | 1 | CF₃ | |
| 7-102 | OSO₂Me | Et | 1 | CF₃ | |
| 7-103 | OSO₂Me | n-Pr | 1 | CF₃ | |
| 7-104 | OSO₂Me | i-Pr | 1 | CF₃ | |
| 7-105 | OSO₂Me | Me | 2 | CF₃ | |
| 7-106 | OSO₂Me | Et | 2 | CF₃ | |
| 7-107 | OSO₂Me | n-Pr | 2 | CF₃ | |
| 7-108 | OSO₂Me | i-Pr | 2 | CF₃ | |
| 7-109 | OMe | Me | 0 | C₂F₅ | |
| 7-110 | OMe | Et | 0 | C₂F₅ | |
| 7-111 | OMe | n-Pr | 0 | C₂F₅ | |
| 7-112 | OMe | i-Pr | 0 | C₂F₅ | |
| 7-113 | OMe | Me | 1 | C₂F₅ | |
| 7-114 | OMe | Et | 1 | C₂F₅ | |
| 7-115 | OMe | n-Pr | 1 | C₂F₅ | |
| 7-116 | OMe | i-Pr | 1 | C₂F₅ | |
| 7-117 | OMe | Me | 2 | C₂F₅ | |
| 7-118 | OMe | Et | 2 | C₂F₅ | |
| 7-119 | OMe | n-Pr | 2 | C₂F₅ | |
| 7-120 | OMe | i-Pr | 2 | C₂F₅ | |
| 7-121 | OEt | Me | 0 | C₂F₅ | |
| 7-122 | OEt | Et | 0 | C₂F₅ | |
| 7-123 | OEt | n-Pr | 0 | C₂F₅ | |
| 7-124 | OEt | i-Pr | 0 | C₂F₅ | |
| 7-125 | OEt | Me | 1 | C₂F₅ | |
| 7-126 | OEt | Et | 1 | C₂F₅ | |
| 7-127 | OEt | n-Pr | 1 | C₂F₅ | |
| 7-128 | OEt | i-Pr | 1 | C₂F₅ | |
| 7-129 | OEt | Me | 2 | C₂F₅ | |
| 7-130 | OEt | Et | 2 | C₂F₅ | |
| 7-131 | OEt | n-Pr | 2 | C₂F₅ | |
| 7-132 | OEt | i-Pr | 2 | C₂F₅ | |
| 7-133 | O-CH₂-c-Pr | Me | 0 | C₂F₅ | |
| 7-134 | O-CH₂-c-Pr | Et | 0 | C₂F₅ | |
| 7-135 | O-CH₂-c-Pr | n-Pr | 0 | C₂F₅ | |
| 7-136 | O-CH₂-c-Pr | i-Pr | 0 | C₂F₅ | |
| 7-137 | O-CH₂-c-Pr | Me | 1 | C₂F₅ | |
| 7-138 | O-CH₂-c-Pr | Et | 1 | C₂F₅ | |
| 7-139 | O-CH₂-c-Pr | n-Pr | 1 | C₂F₅ | |
| 7-140 | O-CH₂-c-Pr | i-Pr | 1 | C₂F₅ | |
| 7-141 | O-CH₂-c-Pr | Me | 2 | C₂F₅ | |
| 7-142 | O-CH₂-c-Pr | Et | 2 | C₂F₅ | |
| 7-143 | O-CH₂-c-Pr | n-Pr | 2 | C₂F₅ | |
| 7-144 | O-CH₂-c-Pr | i-Pr | 2 | C₂F₅ | |
| 7-145 | OCH₂CH₂OMe | Me | 0 | C₂F₅ | |
| 7-146 | OCH₂CH₂OMe | Et | 0 | C₂F₅ | |
| 7-147 | OCH₂CH₂OMe | n-Pr | 0 | C₂F₅ | |
| 7-148 | OCH₂CH₂OMe | i-Pr | 0 | C₂F₅ | |
| 7-149 | OCH₂CH₂OMe | Me | 1 | C₂F₅ | |
| 7-150 | OCH₂CH₂OMe | Et | 1 | C₂F₅ | |
| 7-151 | OCH₂CH₂OMe | n-Pr | 1 | C₂F₅ | |
| 7-152 | OCH₂CH₂OMe | i-Pr | 1 | C₂F₅ | |
| 7-153 | OCH₂CH₂OMe | Me | 2 | C₂F₅ | |
| 7-154 | OCH₂CH₂OMe | Et | 2 | C₂F₅ | |
| 7-155 | OCH₂CH₂OMe | n-Pr | 2 | C₂F₅ | |
| 7-156 | OCH₂CH₂OMe | i-Pr | 2 | C₂F₅ | |
| 7-157 | OCH₂CH₂SMe | Me | 0 | C₂F₅ | |
| 7-158 | OCH₂CH₂SMe | Et | 0 | C₂F₅ | |
| 7-159 | OCH₂CH₂SMe | n-Pr | 0 | C₂F₅ | |
| 7-160 | OCH₂CH₂SMe | i-Pr | 0 | C₂F₅ | |
| 7-161 | OCH₂CH₂SMe | Me | 1 | C₂F₅ | |
| 7-162 | OCH₂CH₂SMe | Et | 1 | C₂F₅ | |
| 7-163 | OCH₂CH₂SMe | n-Pr | 1 | C₂F₅ | |
| 7-164 | OCH₂CH₂SMe | i-Pr | 1 | C₂F₅ | |
| 7-165 | OCH₂CH₂SMe | Me | 2 | C₂F₅ | |
| 7-166 | OCH₂CH₂SMe | Et | 2 | C₂F₅ | |
| 7-167 | OCH₂CH₂SMe | n-Pr | 2 | C₂F₅ | |
| 7-168 | OCH₂CH₂SMe | i-Pr | 2 | C₂F₅ | |
| 7-169 | OCH₂CH₂SO₂Me | Me | 0 | C₂F₅ | |
| 7-170 | OCH₂CH₂SO₂Me | Et | 0 | C₂F₅ | |
| 7-171 | OCH₂CH₂SO₂Me | n-Pr | 0 | C₂F₅ | |
| 7-172 | OCH₂CH₂SO₂Me | i-Pr | 0 | C₂F₅ | |
| 7-173 | OCH₂CH₂SO₂Me | Me | 1 | C₂F₅ | |
| 7-174 | OCH₂CH₂SO₂Me | Et | 1 | C₂F₅ | |
| 7-175 | OCH₂CH₂SO₂Me | n-Pr | 1 | C₂F₅ | |
| 7-176 | OCH₂CH₂SO₂Me | i-Pr | 1 | C₂F₅ | |
| 7-177 | OCH₂CH₂SO₂Me | Me | 2 | C₂F₅ | |
| 7-178 | OCH₂CH₂SO₂Me | Et | 2 | C₂F₅ | |
| 7-179 | OCH₂CH₂SO₂Me | n-Pr | 2 | C₂F₅ | |
| 7-180 | OCH₂CH₂SO₂Me | i-Pr | 2 | C₂F₅ | |
| 7-181 | OMe | Me | 0 | CCl₃ | |
| 7-182 | OMe | Et | 0 | CCl₃ | |
| 7-183 | OMe | n-Pr | 0 | CCl₃ | |
| 7-184 | OMe | i-Pr | 0 | CCl₃ | |
| 7-185 | OMe | Me | 1 | CCl₃ | |
| 7-186 | OMe | Et | 1 | CCl₃ | |
| 7-187 | OMe | n-Pr | 1 | CCl₃ | |
| 7-188 | OMe | i-Pr | 1 | CCl₃ | |
| 7-189 | OMe | Me | 2 | CCl₃ | |
| 7-190 | OMe | Et | 2 | CCl₃ | |
| 7-191 | OMe | n-Pr | 2 | CCl₃ | |
| 7-192 | OMe | i-Pr | 2 | CCl₃ | |
| 7-193 | OEt | Me | 0 | CCl₃ | |
| 7-194 | OEt | Et | 0 | CCl₃ | |
| 7-195 | OEt | n-Pr | 0 | CCl₃ | |
| 7-196 | OEt | i-Pr | 0 | CCl₃ | |
| 7-197 | OEt | Me | 1 | CCl₃ | |
| 7-198 | OEt | Et | 1 | CCl₃ | |
| 7-199 | OEt | n-Pr | 1 | CCl₃ | |
| 7-200 | OEt | i-Pr | 1 | CCl₃ | |
| 7-201 | OEt | Me | 2 | CCl₃ | |
| 7-202 | OEt | Et | 2 | CCl₃ | |
| 7-203 | OEt | n-Pr | 2 | CCl₃ | |
| 7-204 | OEt | i-Pr | 2 | CCl₃ | |
| 7-205 | O-CH₂-c-Pr | Me | 0 | CCl₃ | |
| 7-206 | O-CH₂-c-Pr | Et | 0 | CCl₃ | |
| 7-207 | O-CH₂-c-Pr | n-Pr | 0 | CCl₃ | |
| 7-208 | O-CH₂-c-Pr | i-Pr | 0 | CCl₃ | |
| 7-209 | O-CH₂-c-Pr | Me | 1 | CCl₃ | |
| 7-210 | O-CH₂-c-Pr | Et | 1 | CCl₃ | |
| 7-211 | O-CH₂-c-Pr | n-Pr | 1 | CCl₃ | |
| 7-212 | O-CH₂-c-Pr | i-Pr | 1 | CCl₃ | |
| 7-213 | O-CH₂-c-Pr | Me | 2 | CCl₃ | |
| 7-214 | O-CH₂-c-Pr | Et | 2 | CCl₃ | |
| 7-215 | O-CH₂-c-Pr | n-Pr | 2 | CCl₃ | |
| 7-216 | O-CH₂-c-Pr | i-Pr | 2 | CCl₃ | |
| 7-217 | OCH₂CH₂OMe | Me | 0 | CCl₃ | |
| 7-218 | OCH₂CH₂OMe | Et | 0 | CCl₃ | |
| 7-219 | OCH₂CH₂OMe | n-Pr | 0 | CCl₃ | |
| 7-220 | OCH₂CH₂OMe | i-Pr | 0 | CCl₃ | |
| 7-221 | OCH₂CH₂OMe | Me | 1 | CCl₃ | |
| 7-222 | OCH₂CH₂OMe | Et | 1 | CCl₃ | |
| 7-223 | OCH₂CH₂OMe | n-Pr | 1 | CCl₃ | |
| 7-224 | OCH₂CH₂OMe | i-Pr | 1 | CCl₃ | |
| 7-225 | OCH₂CH₂OMe | Me | 2 | CCl₃ | |
| 7-226 | OCH₂CH₂OMe | Et | 2 | CCl₃ | |
| 7-227 | OCH₂CH₂OMe | n-Pr | 2 | CCl₃ | |
| 7-228 | OCH₂CH₂OMe | i-Pr | 2 | CCl₃ | |
| 7-229 | OCH₂CH₂SMe | Me | 0 | CCl₃ | |
| 7-230 | OCH₂CH₂SMe | Et | 0 | CCl₃ | |
| 7-231 | OCH₂CH₂SMe | n-Pr | 0 | CCl₃ | |
| 7-232 | OCH₂CH₂SMe | i-Pr | 0 | CCl₃ | |
| 7-233 | OCH₂CH₂SMe | Me | 1 | CCl₃ | |
| 7-234 | OCH₂CH₂SMe | Et | 1 | CCl₃ | |
| 7-235 | OCH₂CH₂SMe | n-Pr | 1 | CCl₃ | |
| 7-236 | OCH₂CH₂SMe | i-Pr | 1 | CCl₃ | |
| 7-237 | OCH₂CH₂SMe | Me | 2 | CCl₃ | |
| 7-238 | OCH₂CH₂SMe | Et | 2 | CCl₃ | |
| 7-239 | OCH₂CH₂SMe | n-Pr | 2 | CCl₃ | |
| 7-240 | OCH₂CH₂SMe | i-Pr | 2 | CCl₃ | |
| 7-241 | OCH₂CH₂SO₂Me | Me | 0 | CCl₃ | |
| 7-242 | OCH₂CH₂SO₂Me | Et | 0 | CCl₃ | |
| 7-243 | OCH₂CH₂SO₂Me | n-Pr | 0 | CCl₃ | |
| 7-244 | OCH₂CH₂SO₂Me | i-Pr | 0 | CCl₃ | |
| 7-245 | OCH₂CH₂SO₂Me | Me | 1 | CCl₃ | |
| 7-246 | OCH₂CH₂SO₂Me | Et | 1 | CCl₃ | |
| 7-247 | OCH₂CH₂SO₂Me | n-Pr | 1 | CCl₃ | |
| 7-248 | OCH₂CH₂SO₂Me | i-Pr | 1 | CCl₃ | |
| 7-249 | OCH₂CH₂SO₂Me | Me | 2 | CCl₃ | |
| 7-250 | OCH₂CH₂SO₂Me | Et | 2 | CCl₃ | |
| 7-251 | OCH₂CH₂SO₂Me | n-Pr | 2 | CCl₃ | |
| 7-252 | OCH₂CH₂SO₂Me | i-Pr | 2 | CCl₃ | |
| 7-253 | OMe | Me | 0 | CHF₂ | |
| 7-254 | OMe | Et | 0 | CHF₂ | |
| 7-255 | OMe | n-Pr | 0 | CHF₂ | |
| 7-256 | OMe | i-Pr | 0 | CHF₂ | |
| 7-257 | OMe | Me | 1 | CHF₂ | |
| 7-258 | OMe | Et | 1 | CHF₂ | |
| 7-259 | OMe | n-Pr | 1 | CHF₂ | |
| 7-260 | OMe | i-Pr | 1 | CHF₂ | |
| 7-261 | OMe | Me | 2 | CHF₂ | |
| 7-262 | OMe | Et | 2 | CHF₂ | |
| 7-263 | OMe | n-Pr | 2 | CHF₂ | |
| 7-264 | OMe | i-Pr | 2 | CHF₂ | |
| 7-265 | OEt | Me | 0 | CHF₂ | |
| 7-266 | OEt | Et | 0 | CHF₂ | |
| 7-267 | OEt | n-Pr | 0 | CHF₂ | |
| 7-268 | OEt | i-Pr | 0 | CHF₂ | |
| 7-269 | OEt | Me | 1 | CHF₂ | |
| 7-270 | OEt | Et | 1 | CHF₂ | |
| 7-271 | OEt | n-Pr | 1 | CHF₂ | |
| 7-272 | OEt | i-Pr | 1 | CHF₂ | |
| 7-273 | OEt | Me | 2 | CHF₂ | |
| 7-274 | OEt | Et | 2 | CHF₂ | |
| 7-275 | OEt | n-Pr | 2 | CHF₂ | |
| 7-276 | OEt | i-Pr | 2 | CHF₂ | |
| 7-277 | O-CH₂-c-Pr | Me | 0 | CHF₂ | |
| 7-278 | O-CH₂-c-Pr | Et | 0 | CHF₂ | |
| 7-279 | O-CH₂-c-Pr | n-Pr | 0 | CHF₂ | |
| 7-280 | O-CH₂-c-Pr | i-Pr | 0 | CHF₂ | |
| 7-281 | O-CH₂-c-Pr | Me | 1 | CHF₂ | |
| 7-282 | O-CH₂-c-Pr | Et | 1 | CHF₂ | |
| 7-283 | O-CH₂-c-Pr | n-Pr | 1 | CHF₂ | |
| 7-284 | O-CH₂-c-Pr | i-Pr | 1 | CHF₂ | |
| 7-285 | O-CH₂-c-Pr | Me | 2 | CHF₂ | |
| 7-286 | O-CH₂-c-Pr | Et | 2 | CHF₂ | |
| 7-287 | O-CH₂-c-Pr | n-Pr | 2 | CHF₂ | |
| 7-288 | O-CH₂-c-Pr | i-Pr | 2 | CHF₂ | |
| 7-289 | OCH₂CH₂OMe | Me | 0 | CHF₂ | |
| 7-290 | OCH₂CH₂OMe | Et | 0 | CHF₂ | |
| 7-291 | OCH₂CH₂OMe | n-Pr | 0 | CHF₂ | |
| 7-292 | OCH₂CH₂OMe | i-Pr | 0 | CHF₂ | |
| 7-293 | OCH₂CH₂OMe | Me | 1 | CHF₂ | |
| 7-294 | OCH₂CH₂OMe | Et | 1 | CHF₂ | |
| 7-295 | OCH₂CH₂OMe | n-Pr | 1 | CHF₂ | |
| 7-296 | OCH₂CH₂OMe | i-Pr | 1 | CHF₂ | |
| 7-297 | OCH₂CH₂OMe | Me | 2 | CHF₂ | |
| 7-298 | OCH₂CH₂OMe | Et | 2 | CHF₂ | |
| 7-299 | OCH₂CH₂OMe | n-Pr | 2 | CHF₂ | |
| 7-300 | OCH₂CH₂OMe | i-Pr | 2 | CHF₂ | |
| 7-301 | OCH₂CH₂SMe | Me | 0 | CHF₂ | |
| 7-302 | OCH₂CH₂SMe | Et | 0 | CHF₂ | |
| 7-303 | OCH₂CH₂SMe | n-Pr | 0 | CHF₂ | |
| 7-304 | OCH₂CH₂SMe | i-Pr | 0 | CHF₂ | |
| 7-305 | OCH₂CH₂SMe | Me | 1 | CHF₂ | |
| 7-306 | OCH₂CH₂SMe | Et | 1 | CHF₂ | |
| 7-307 | OCH₂CH₂SMe | n-Pr | 1 | CHF₂ | |
| 7-308 | OCH₂CH₂SMe | i-Pr | 1 | CHF₂ | |
| 7-309 | OCH₂CH₂SMe | Me | 2 | CHF₂ | |
| 7-310 | OCH₂CH₂SMe | Et | 2 | CHF₂ | |
| 7-311 | OCH₂CH₂SMe | n-Pr | 2 | CHF₂ | |
| 7-312 | OCH₂CH₂SMe | i-Pr | 2 | CHF₂ | |
| 7-313 | OCH₂CH₂SO₂Me | Me | 0 | CHF₂ | |
| 7-314 | OCH₂CH₂SO₂Me | Et | 0 | CHF₂ | |
| 7-315 | OCH₂CH₂SO₂Me | n-Pr | 0 | CHF₂ | |
| 7-316 | OCH₂CH₂SO₂Me | i-Pr | 0 | CHF₂ | |
| 7-317 | OCH₂CH₂SO₂Me | Me | 1 | CHF₂ | |
| 7-318 | OCH₂CH₂SO₂Me | Et | 1 | CHF₂ | |
| 7-319 | OCH₂CH₂SO₂Me | n-Pr | 1 | CHF₂ | |
| 7-320 | OCH₂CH₂SO₂Me | i-Pr | 1 | CHF₂ | |
| 7-321 | OCH₂CH₂SO₂Me | Me | 2 | CHF₂ | |
| 7-322 | OCH₂CH₂SO₂Me | Et | 2 | CHF₂ | |
| 7-323 | OCH₂CH₂SO₂Me | n-Pr | 2 | CHF₂ | |
| 7-324 | OCH₂CH₂SO₂Me | i-Pr | 2 | CHF₂ | |
| 7-325 | OMe | Me | 0 | CF(CF₃)₂ | |
| 7-326 | OMe | Et | 0 | CF(CF₃)₂ | |
| 7-327 | OMe | n-Pr | 0 | CF(CF₃)₂ | |
| 7-328 | OMe | i-Pr | 0 | CF(CF₃)₂ | |
| 7-329 | OMe | Me | 1 | CF(CF₃)₂ | |
| 7-330 | OMe | Et | 1 | CF(CF₃)₂ | |
| 7-331 | OMe | n-Pr | 1 | CF(CF₃)₂ | |
| 7-332 | OMe | i-Pr | 1 | CF(CF₃)₂ | |
| 7-333 | OMe | Me | 2 | CF(CF₃)₂ | |
| 7-334 | OMe | Et | 2 | CF(CF₃)₂ | |
| 7-335 | OMe | n-Pr | 2 | CF(CF₃)₂ | |
| 7-336 | OMe | i-Pr | 2 | CF(CF₃)₂ | |
| 7-337 | OEt | Me | 0 | CF(CF₃)₂ | |
| 7-338 | OEt | Et | 0 | CF(CF₃)₂ | |
| 7-339 | OEt | n-Pr | 0 | CF(CF₃)₂ | |
| 7-340 | OEt | i-Pr | 0 | CF(CF₃)₂ | |
| 7-341 | OEt | Me | 1 | CF(CF₃)₂ | |
| 7-342 | OEt | Et | 1 | CF(CF₃)₂ | |
| 7-343 | OEt | n-Pr | 1 | CF(CF₃)₂ | |
| 7-344 | OEt | i-Pr | 1 | CF(CF₃)₂ | |
| 7-345 | OEt | Me | 2 | CF(CF₃)₂ | |
| 7-346 | OEt | Et | 2 | CF(CF₃)₂ | |
| 7-347 | OEt | n-Pr | 2 | CF(CF₃)₂ | |
| 7-348 | OEt | i-Pr | 2 | CF(CF₃)₂ | |
| 7-349 | O-CH₂-c-Pr | Me | 0 | CF(CF₃)₂ | |
| 7-350 | O-CH₂-c-Pr | Et | 0 | CF(CF₃)₂ | |
| 7-351 | O-CH₂-c-Pr | n-Pr | 0 | CF(CF₃)₂ | |
| 7-352 | O-CH₂-c-Pr | i-Pr | 0 | CF(CF₃)₂ | |
| 7-353 | O-CH₂-c-Pr | Me | 1 | CF(CF₃)₂ | |
| 7-354 | O-CH₂-c-Pr | Et | 1 | CF(CF₃)₂ | |
| 7-355 | O-CH₂-c-Pr | n-Pr | 1 | CF(CF₃)₂ | |
| 7-356 | O-CH₂-c-Pr | i-Pr | 1 | CF(CF₃)₂ | |
| 7-357 | O-CH₂-c-Pr | Me | 2 | CF(CF₃)₂ | |
| 7-358 | O-CH₂-c-Pr | Et | 2 | CF(CF₃)₂ | |
| 7-359 | O-CH₂-c-Pr | n-Pr | 2 | CF(CF₃)₂ | |
| 7-360 | O-CH₂-c-Pr | i-Pr | 2 | CF(CF₃)₂ | |
| 7-361 | OCH₂CH₂OMe | Me | 0 | CF(CF₃)₂ | |
| 7-362 | OCH₂CH₂OMe | Et | 0 | CF(CF₃)₂ | |
| 7-363 | OCH₂CH₂OMe | n-Pr | 0 | CF(CF₃)₂ | |
| 7-364 | OCH₂CH₂OMe | i-Pr | 0 | CF(CF₃)₂ | |
| 7-365 | OCH₂CH₂OMe | Me | 1 | CF(CF₃)₂ | |
| 7-366 | OCH₂CH₂OMe | Et | 1 | CF(CF₃)₂ | |
| 7-367 | OCH₂CH₂OMe | n-Pr | 1 | CF(CF₃)₂ | |
| 7-368 | OCH₂CH₂OMe | i-Pr | 1 | CF(CF₃)₂ | |
| 7-369 | OCH₂CH₂OMe | Me | 2 | CF(CF₃)₂ | |
| 7-370 | OCH₂CH₂OMe | Et | 2 | CF(CF₃)₂ | |
| 7-371 | OCH₂CH₂OMe | n-Pr | 2 | CF(CF₃)₂ | |
| 7-372 | OCH₂CH₂OMe | i-Pr | 2 | CF(CF₃)₂ | |
| 7-373 | OCH₂CH₂SMe | Me | 0 | CF(CF₃)₂ | |
| 7-374 | OCH₂CH₂SMe | Et | 0 | CF(CF₃)₂ | |
| 7-375 | OCH₂CH₂SMe | n-Pr | 0 | CF(CF₃)₂ | |
| 7-376 | OCH₂CH₂SMe | i-Pr | 0 | CF(CF₃)₂ | |
| 7-377 | OCH₂CH₂SMe | Me | 1 | CF(CF₃)₂ | |
| 7-378 | OCH₂CH₂SMe | Et | 1 | CF(CF₃)₂ | |
| 7-379 | OCH₂CH₂SMe | n-Pr | 1 | CF(CF₃)₂ | |
| 7-380 | OCH₂CH₂SMe | i-Pr | 1 | CF(CF₃)₂ | |
| 7-381 | OCH₂CH₂SMe | Me | 2 | CF(CF₃)₂ | |
| 7-382 | OCH₂CH₂SMe | Et | 2 | CF(CF₃)₂ | |
| 7-383 | OCH₂CH₂SMe | n-Pr | 2 | CF(CF₃)₂ | |
| 7-384 | OCH₂CH₂SMe | i-Pr | 2 | CF(CF₃)₂ | |
| 7-385 | OCH₂CH₂SO₂Me | Me | 0 | CF(CF₃)₂ | |
| 7-386 | OCH₂CH₂SO₂Me | Et | 0 | CF(CF₃)₂ | |
| 7-387 | OCH₂CH₂SO₂Me | n-Pr | 0 | CF(CF₃)₂ | |
| 7-388 | OCH₂CH₂SO₂Me | i-Pr | 0 | CF(CF₃)₂ | |
| 7-389 | OCH₂CH₂SO₂Me | Me | 1 | CF(CF₃)₂ | |
| 7-390 | OCH₂CH₂SO₂Me | Et | 1 | CF(CF₃)₂ | |
| 7-391 | OCH₂CH₂SO₂Me | n-Pr | 1 | CF(CF₃)₂ | |
| 7-392 | OCH₂CH₂SO₂Me | i-Pr | 1 | CF(CF₃)₂ | |
| 7-393 | OCH₂CH₂SO₂Me | Me | 2 | CF(CF₃)₂ | |
| 7-394 | OCH₂CH₂SO₂Me | Et | 2 | CF(CF₃)₂ | |
| 7-395 | OCH₂CH₂SO₂Me | n-Pr | 2 | CF(CF₃)₂ | |
| 7-396 | OCH₂CH₂SO₂Me | i-Pr | 2 | CF(CF₃)₂ | |

### B. Formulierungsbeispiele

a) Ein Stäubemittel wird erhalten, indem man 10 Gew.-Teile einer Verbindung der Formel (I) und/oder deren Salze und 90 Gew.-Teile Talkum als Inertstoff mischt und in einer Schlagmühle zerkleinert.
b) Ein in Wasser leicht dispergierbares, benetzbares Pulver wird erhalten, indem man 25 Gewichtsteile einer Verbindung der Formel (I) und/oder deren Salze, 64 Gew.-Teile kaolinhaltigen Quarz als Inertstoff, 10 Gewichtsteile ligninsulfonsaures Kalium und 1 Gew.-Teil oleoylmethyltaurinsaures Natrium als Netz- und Dispergiermittel mischt und in einer Stiftmühle mahlt.
c) Ein in Wasser leicht dispergierbares Dispersionskonzentrat wird erhalten, indem man 20 Gew.-Teile einer Verbindung der Formel (I) und/oder deren Salze mit 6 Gew.-Teilen Alkylphenolpolyglykolether (®Triton X 207), 3 Gew.-Teilen Isotridecanolpolyglykolether (8 EO) und 71 Gew.-Teilen paraffinischem Mineralöl (Siedebereich z.B. ca. 255 bis über 277 C) mischt und in einer Reibkugelmühle auf eine Feinheit von unter 5 Mikron vermahlt.
d) Ein emulgierbares Konzentrat wird erhalten aus 15 Gew.-Teilen einer Verbindung der Formel (I) und/oder deren Salze, 75 Gew.-Teilen Cyclohexanon als Lösungsmittel und 10 Gew.-Teilen oxethyliertes Nonylphenol als Emulgator.
e) Ein in Wasser dispergierbares Granulat wird erhalten indem man
   75 Gew.-Teile einer Verbindung der Formel (I) und/oder deren Salze,
   10 Gew.-Teile ligninsulfonsaures Calcium,
   5 Gew.-Teile Natriumlaurylsulfat,
   3 Gew.-Teile Polyvinylalkohol und
   7 Gew.-Teile Kaolin
   mischt, auf einer Stiftmühle mahlt und das Pulver in einem Wirbelbett durch Aufsprühen von Wasser als Granulierflüssigkeit granuliert.
f) Ein in Wasser dispergierbares Granulat wird auch erhalten, indem man
   25 Gew.-Teile einer Verbindung der Formel (I) und/oder deren Salze,
   5 Gew.-Teile 2,2'-dinaphthylmethan-6,6'-disulfonsaures Natrium
   2 Gew.-Teile oleoylmethyltaurinsaures Natrium,
   1 Gew.-Teil Polyvinylalkohol,
   17 Gew.-Teile Calciumcarbonat und
   50 Gew.-Teile Wasser
   auf einer Kolloidmühle homogenisiert und vorzerkleinert, anschließend auf einer Perlmühle mahlt und die so erhaltene Suspension in einem Sprühturm mittels einer Einstoffdüse zerstäubt und trocknet.

### C. Biologische Beispiele

### 1. Herbizide Wirkung gegen Schadpflanzen im Vorauflauf

Samen von mono- bzw. dikotylen Unkraut- bzw. Kulturpflanzen werden in Holzfasertöpfen in sandiger Lehmerde ausgelegt und mit Erde abgedeckt. Die in Form von benetzbaren Pulvern (WP) oder als Emulsionskonzentrate (EC) formulierten erfindungsgemäßen Verbindungen werden dann als wäßrige Suspension bzw. Emulsion mit einer Wasseraufwandmenge von umgerechnet 600 bis 800 l/ha unter Zusatz von 0,2% Netzmittel auf die Oberfläche der Abdeckerde appliziert. Nach der Behandlung werden die Töpfe im Gewächshaus aufgestellt und unter guten Wachstumsbedingungen für die Testpflanzen gehalten. Die visuelle Bonitur der Schäden an den Versuchspflanzen erfolgt nach einer Versuchszeit von 3 Wochen im Vergleich zu unbehandelten Kontrollen (herbizide Wirkung in Prozent (%): 100% Wirkung = Pflanzen sind abgestorben, 0 % Wirkung = wie Kontrollpflanzen). Dabei zeigen beispielsweise die Verbindungen Nr. 2-13, 2-17 sowie 2-21 bei einer Aufwandmenge von 80 g/ha jeweils eine mindestens 90 %-ige Wirkung gegen Amaranthus retroflexus, Echinochloa crus galli und Stellaria media. Die Verbindungen Nr. 1-17 sowie 1-21 zeigen bei einer Aufwandmenge von 80 g/ha jeweils eine mindestens 90 %-ige Wirkung gegen Alopecurus myosuroides, Veronica persica und Viola tricolor. Die Verbindungen Nr. 3-21, 3-17, 3-13 sowie 1-13 zeigen bei einer Aufwandmenge von 80 g/ha jeweils eine mindestens 90 %-ige Wirkung gegen Abutilon theophrasti, Amaranthus retroflexus und Echinochloa crus galli.

### 2. Herbizide Wirkung gegen Schadpflanzen im Nachauflauf

Samen von mono- bzw. dikotylen Unkraut- bzw. Kulturpflanzen werden in Holzfasertöpfen in sandigem Lehmboden ausgelegt, mit Erde abgedeckt und im Gewächshaus unter guten Wachstumsbedingungen angezogen. 2 bis 3 Wochen nach der Aussaat werden die Versuchspflanzen im Einblattstadium behandelt. Die in Form von benetzbaren Pulvern (WP) oder als Emulsionskonzentrate (EC) formulierten erfindungsgemäßen Verbindungen werden dann als wäßrige Suspension bzw. Emulsion mit einer Wasseraufwandmenge von umgerechnet 600 bis 800 l/ha unter Zusatz von 0,2% Netzmittel auf die grünen Pflanzenteile gesprüht. Nach ca. 3 Wochen Standzeit der Versuchspflanzen im Gewächshaus unter optimalen Wachstumsbedingungen wird die Wirkung der Präparate visuell im Vergleich zu unbehandelten Kontrollen bonitiert (herbizide Wirkung in Prozent (%): 100% Wirkung = Pflanzen sind abgestorben, 0 % Wirkung = wie Kontrollpflanzen). Dabei zeigen beispielsweise die Verbindungen Nr. 2-13, 2-17 sowie 3-21 bei einer Aufwandmenge von 80 g/ha jeweils eine mindestens 90 %-ige Wirkung gegen Abutilon theophrasti, Echinochloa crus galli und Setaria viridis. Die Verbindungen Nr. 2-17 sowie 3-21 zeigen bei einer Aufwandmenge von 80 g/ha jeweils eine mindestens 90 %-ige Wirkung gegen Abutilon theophrasti, Setaria viridis und Stellaria media. Die Verbindungen Nr. 1-21, 1-17, 3-13 sowie 1-13 zeigen bei einer Aufwandmenge von 80 g/ha jeweils eine mindestens 90 %-ige Wirkung gegen Avefa fatua, Echinochloa crus galli und Veronica persica.

## Patentansprüche

1. 4-(3-Alkylthiobenzoyl)pyrazole der Formel (I) oder deren Salze 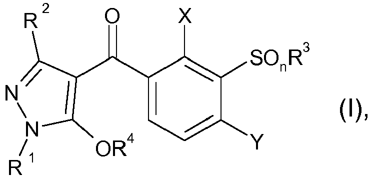
R¹ bedeutet Methyl, Ethyl, n-Propyl oder i-Propyl,
R² bedeutet Wasserstoff, Methyl, Ethyl, n-Propyl oder i-Propyl,
R³ bedeutet Methyl, Ethyl, n-Propyl oder i-Propyl,
R⁴ bedeutet Wasserstoff, (C₁-C₆)-Alkylsulfonyl, (C₁-C₄)-Alkoxy-(C₁-C₆)-alkylsulfonyl, Phenylsulfonyl, Thiophenyl-2-sulfonyl, Benzoyl, Benzoyl-(C₁-C₆)-alkyl, Benzyl, wobei die fünf letztgenannten Reste durch s Reste aus der Gruppe bestehend aus Halogen, (C₁-C₄)-Alkyl und (C₁-C₄)-Alkoxy substituiert sind;
X bedeutet OR⁵,
R⁵ bedeutet (C₁-C₆)-Alkyl,
Y bedeutet (C₁-C₆)-Halogenalkyl,
n bedeutet 0, 1 oder 2,
s bedeutet 0, 1, 2 oder 3.

2. Herbizide Mittel, **gekennzeichnet durch** einen herbizid wirksamen Gehalt an mindestens einer Verbindung der Formel (I) gemäß Anspruch 1.

3. Herbizide Mittel nach Anspruch 2 in Mischung mit Formulierungshilfsmitteln.

4. Verfahren zur Bekämpfung unerwünschter Pflanzen, **dadurch gekennzeichnet, daß** man eine wirksame Menge mindestens einer Verbindung der Formel (I) gemäß Anspruch 1 oder eines herbiziden Mittels nach Anspruch 2 oder 3 auf die Pflanzen oder auf den Ort des unerwünschten Pflanzenwachstums appliziert.

5. Verwendung von Verbindungen der Formel (I) gemäß Anspruch 1 oder von herbiziden Mitteln nach Anspruch 2 oder 3 zur Bekämpfung unerwünschter Pflanzen.

6. Verwendung nach Anspruch 5, **dadurch gekennzeichnet, daß** die Verbindungen der Formel (I) zur Bekämpfung unerwünschter Pflanzen in Kulturen von Nutzpflanzen eingesetzt werden.

7. Verwendung nach Anspruch 6, **dadurch gekennzeichnet, daß** die Nutzpflanzen transgene Nutzpflanzen sind.

8. Verbindungen der Formel (II) 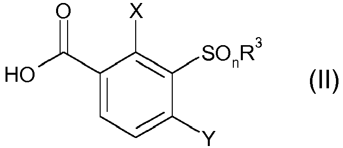 worin X, Y, R³ und n wie in Anspruch 1 definiert sind.

## Claims

1. 4-(3-Alkylthiobenzoyl)pyrazoles of the formula (I) or salts thereof 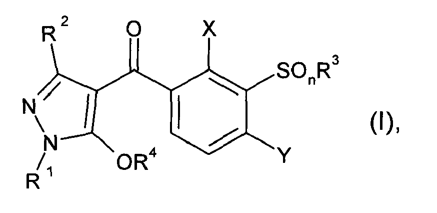
R¹ is methyl, ethyl, n-propyl or isopropyl,
R² is hydrogen, methyl, ethyl, n-propyl or isopropyl,
R³ is methyl, ethyl, n-propyl or isopropyl,
R⁴ is hydrogen, (C₁-C₆)-alkylsulfonyl, (C₁-C₄)-alkoxy-(C₁-C₆)-alkylsulfonyl, phenylsulfonyl, thiophenyl-2-sulfonyl, benzoyl, benzoyl-(C₁-C₆)-alkyl, benzyl, where the five last-mentioned radicals are substituted by s radicals from the group consisting of halogen, (C₁-C₄)-alkyl and (C₁-C₄)-alkoxy;
X is OR⁵,
R⁵ is (C₁-C₆)-alkyl,
Y is (C₁-C₆)-haloalkyl,
n is 0, 1 or 2,
s is 0, 1, 2 or 3.

2. Herbicidal compositions, **characterized by** a herbicidally effective amount of at least one compound of the formula (I) according to Claim 1.

3. Herbicidal compositions according to Claim 2 in a mixture with formulation auxiliaries.

4. Method for controlling unwanted plants, **characterized in that** an effective amount of at least one compound of the formula (I) according to Claim 1 or of a herbicidal composition according to Claim 2 or 3 is applied to the plants or the site of the unwanted vegetation.

5. Use of compounds of the formula (I) according to Claim 1 or of herbicidal compositions according to Claim 2 or 3 for controlling unwanted plants.

6. Use according to Claim 5, **characterized in that** the compounds of the formula (I) are used for controlling unwanted plants in crops of useful plants.

7. Use according to Claim 6, **characterized in that** the useful plants are transgenic useful plants.

8. Compounds of the formula (II) 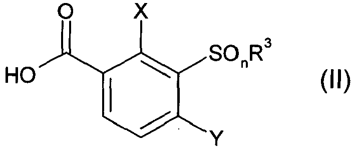 in which X, Y, R³ and n are as defined in Claim 1.

## Revendications

1. 4-(3-Alkylthiobenzoyl)pyrazoles de formule (I) ou leurs sels 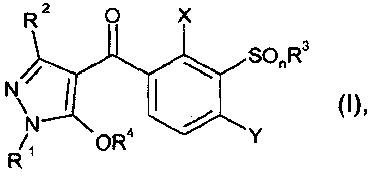 dans laquelle
R¹ signifie méthyle, éthyle, n-propyle ou i-propyle,
R² signifie hydrogène, méthyle, éthyle, n-propyle ou i-propyle,
R³ signifie méthyle, éthyle, n-propyle ou i-propyle,
R⁴ signifie hydrogène, alkylsulfonyle en (C₁-C₆), alcoxy en (C₁-C₄)-alkylsulfonyle en (C₁-C₆), phénylsulfonyle, thiophényl-2-sulfonyle, benzoyle, benzoyl-alkyle en (C₁-C₆), benzyle, les cinq derniers radicaux cités étant substitués par s radicaux du groupe constitué par halogène, alkyle en (C₁-C₄) et alcoxy en (C₁-C₄) ;
X signifie OR⁵,
R⁵ signifie alkyle en (C₁-C₆),
Y signifie halogénoalkyle en (C₁-C₆),
n signifie 0, 1 ou 2,
s signifie 0, 1, 2 ou 3.

2. Agents herbicides, **caractérisés par** une teneur herbicide efficace en au moins un composé de formule (I) selon la revendication 1.

3. Agents herbicides selon la revendication 2, en mélange avec des adjuvants de formulation.

4. Procédé de lutte contre des plantes indésirables, **caractérisé en ce qu'**une quantité efficace d'au moins un composé de formule (I) selon la revendication 1 ou d'un agent herbicide selon la revendication 2 ou 3 est appliquée sur les plantes ou à l'emplacement de la végétation indésirable.

5. Utilisation de composés de formule (I) selon la revendication 1 ou d'agents herbicides selon la revendication 2 ou 3 pour lutter contre des plantes indésirables.

6. Utilisation selon la revendication 5, **caractérisée en ce que** les composés de formule (I) sont utilisés pour lutter contre des plantes indésirables dans des cultures de plantes utiles.

7. Utilisation selon la revendication 6, **caractérisée en ce que** les plantes utiles sont des plantes utiles transgéniques.

8. Composés de formule (II) 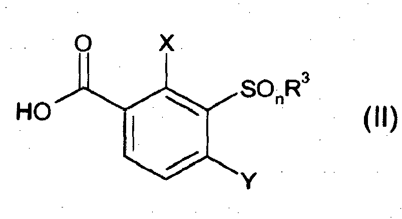 dans laquelle X, Y, R³ et n sont tels que définis dans la revendication 1.
